# EUROPÄISCHE PATENTSCHRIFT

(11) **EP 1 692 151 B1**
(45) Veröffentlichungstag und Bekanntmachung des Hinweises auf die Patenterteilung: **12.09.2007**
(21) Anmeldenummer: 04820078.6
(22) Anmeldetag: 10.12.2004
(51) Int. Cl.: C07F 17/02, C07F 9/547

(54) **FERROCENYL-1, 2-DIPHOSPHINE, DEREN HERSTELLUNG UND DEREN VERWENDUNG**
FERROCENYL-1, 2-DIPHOSPHINES, THE PRODUCTION THEREOF AND THEIR USE
FERROCENYL-1, 2-DIPHOSPHINES, LEUR PREPARATION ET LEUR UTILISATION

(30) Priorität: 12.12.2003 CH 213103
(43) Veröffentlichungstag der Anmeldung: 23.08.2006
(73) Patentinhaber: Solvias AG, 4057 Basel (CH)
(72) Erfinder: LOTZ, Matthias, CH-4054 Basel (CH); KESSELGRUBER, Martin, CH-4052 Basel (CH); THOMMEN, Marc, CH-4412 Nuglar (CH); PUGIN, Benoît, CH-4142 Münchenstein (CH)
(74) Vertreter: Maué, Paul Georg
(86) Internationale Anmeldenummer: PCT/EP2004/053389
(87) Internationale Veröffentlichungsnummer: WO 2005/056568

(56) Entgegenhaltungen:
- EP-A- 0 842 140
- IRELAND T ET AL: "FERROCENYLLIGANDEN MIT ZWEI PHOSPHANYLSUBSTITUENTEN IN A,E-POSITION FUER DIE UEBERGANGSMETALL-KATALYSIERTE ASYMMETRISCHE HYDRIERUNG FUNKTIONALISIERTER DOPPELBINDUNGEN" ANGEWANDTE CHEMIE, VCH VERLAGSGESELLSCHAFT, WEINHEIM, DE, Bd. 111, Nr. 21, 1999, Seiten 3397-3400, XP002179596 ISSN: 0044-8249

## Beschreibung

Die vorliegende Erfindung betrifft 1,2-Disekundärphosphinferrocene mit wenigstens einer zyklischen Phosphingruppe, Verfahren zu deren Herstellung; Metallkomplexe mit diesen Diphosphinferrocenen; sowie die Verwendung der Metallkomplexe als Katalysatoren für enantioselektive Anlagerungsreaktion an prochirale organische Verbindungen.

Chirale Diphosphine sind nützliche Liganden für die asymmetrische Katalyse, besonders für die enantioselektive Hydrierung von ungesättigten, prochiralen, organischen Verbindungen. In letzter Zeit sind auch Diphosphine mit zyklischen Sekundärphosphinresten (zum Beispiel Phosphetan- oder Phospholanresten) für diesen Zweck bekannt geworden. So sind in der EP-B1-0 592 552 und von M. J. Burk in Acc. of Chem. Res. Vol. 33, No. 6 (2000), Seiten 363 bis 372, 1,2-Diphospholane von zum Beispiel Benzol oder Ethan, sowie 1,1'-Ferrocenyldiphospholane, ihre Herstellung über zyklische Butandiolsulfate, und deren Verwendung in Metallkomplexen zur enantioselektiven Katalyse beschrieben. Die Phospholanreste sind in den α,α'-Stellungen mit zum Beispiel Methyl oder Ethyl substituiert, um die gewünschte Chiralität zu gewährleisten. Bei den 1,1'-Ferrocenyl-diphospholanen sind die Phospholangruppen an je einen der beiden Cyclopentadienylringe gebunden. U. Berens et al. beschreiben in Angew. Chem. 112 (11) Seiten 2057-2060 (2000) 1,1'-Diphosphetanylferrocene mit α,α'-Substituenten wie zum Beispiel Methyl. 1,1'-Diphosphetanylferrocene werden auch in der WO 98/02445 erwähnt. In der EP-B1-0 889 048 werden Benzol-1,2-diphospholane vorgeschlagen, deren Phospholanringe zusätzlich mit Alkoxygruppen substituiert sind.

Als bestes Herstellverfahren für zyklische Sekundärphosphine hat sich bislang die Umsetzung primärer Diphosphine mit gegebenenfalls substituierten Alkylensulfaten erwiesen (siehe M. J. Burk in J. Am. Chem. Soc. 1991, 113, Seiten 8518-8519). Die Herstellung primärer Diphosphine ist in der Ferrocenreihe, wie zum Beispiel für Ferrocen-1,2-diphosphine noch gar nicht beschrieben. Ferrocenyl-1,2-disekundärphosphine mit wenigstens einer zyklischen Phosphingruppe sind auf Grund der synthetischen Schwierigkeiten noch nicht bekannt.

Es wurde nun überraschend gefunden, dass man mit organischen Lithium- oder Magnesiumverbindungen in Ferrocen-Monophosphinen das nicht-aktivierte Wasserstoffatom in Orthostellung zur Phosphingruppe dann in hohen Ausbeuten regioselektiv metallieren kann, wenn die Phosphingruppe Amino- und/oder Oxysubstituenten enthält. Die Reaktion verläuft dann besonders gut, wenn am P-Atom zusätzlich Boran der Formel BH₃ gebunden ist. Diese metallierten Ferrocen-Monophosphine können in einfacher Weise, in überraschend hohen Ausbeuten und sogar im industriellen Massstab in Ferrocen-1,2-disekundärphosphine mit wenigstens einer zyklischen Sekundärphosphingruppe übergeführt werden.

In Ferrocenen wird mit der Metallierung eine planare Chiralität erzeugt. Es wurde zusätzlich überraschend gefunden, dass die Metallierung dann hoch stereoselektiv verläuft, wenn an die N- beziehungsweise O-Atome in der Phosphingruppe der Monophosphinferrocene chirale Reste gebunden sind, die insbesondere in α- oder β-Stellung zu den N- beziehungsweise O-Atomen ein chirales C-Atom enthalten. Auf diese Weise werden mit der Synthese direkt Diastereomere in hohen optischen Ausbeuten erhalten, so dass aufwendige Trennoperationen vermeidbar sind. Metallkomplexe mit den Ferrocen-1,2-disekundärphosphinen eignen sich hervorragend für eine asymmetrische Hydrierung bei hohen chemischen und optischen Ausbeuten.

Ein erster Gegenstand der Erfindung sind Verbindungen der Formel I in Form von Racematen, Gemischen von Diastereomeren oder im wesentlichen reinen Diastereomeren, 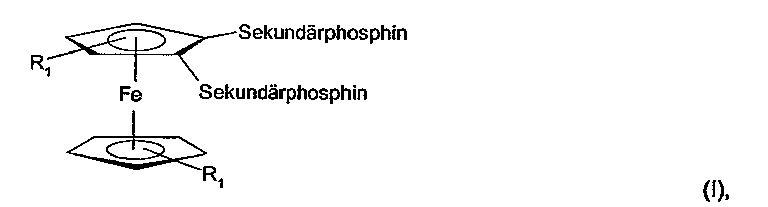 worin
R₁ Wasserstoffatom oder C₁-C₄-Alkyl bedeutet, und wenigstens ein Sekundärphosphin eine unsubstituierte oder substituierte zyklische Phosphingruppe darstellt.

Bei R₁ als Alkyl kann es sich zum Beispiel um Methyl, Ethyl, n- oder i-Propyl, n-, i- oder t-Butyl handeln. Bevorzugt ist R₁ ein Wasserstoffatom.

Die nicht-zyklische, sekundäre Phosphingruppe kann zwei gleiche oder zwei verschiedene Kohlenwasserstoffreste enthalten. Bevorzugt enthält die Phosphingruppe zwei gleiche Kohlenwasserstoffreste. Die Kohlenwasserstoffreste können unsubstituiert oder substituiert sein und sie können 1 bis 22, bevorzugt 1 bis 12, und besonders bevorzugt 1 bis 8 C-Atome enthalten. Ein bevorzugtes nicht-zyklisches Sekundärphosphino ist jenes, worin die Phosphingruppe zwei gleiche oder verschiedene Reste, ausgewählt aus der Gruppe lineares oder verzweigtes C₁-C₁₂-Alkyl; unsubstituiertes oder mit C₁-C₆-Alkyl oder C₁-C₆-Alkoxy substituiertes C₅-C₁₂-Cycloalkyl oder C₅-C₁₂-Cycloalkyl-CH₂-; Phenyl oder Benzyl; oder mit Halogen (zum Beispiel F, Cl und Br), C₁-C₆-Alkyl, C₁-C₆-Halogenalkyl (zum Beispiel Trifluormethyl), C₁-C₆-Alkoxy, C₁-C₆-Halogenalkoxy (zum Beispiel Trifluormethoxy), (C₆H₅)₃Si, (C₁-C₁₂-Alkyl)₃Si, Sekundäramino oder -CO₂-C₁-C₆-Alkyl (zum Beispiel -CO₂CH₃) substituiertes Phenyl oder Benzyl, enthält.

Beispiele für P-Substituenten als Alkyl, das bevorzugt 1 bis 6 C-Atome enthält, sind Methyl, Ethyl, n-Propyl, i-Propyl, n-Butyl, i-Butyl, t-Butyl, und die Isomeren von Pentyl und Hexyl. Beispiele für P-Substituenten als gegebenenfalls mit Alkyl substituiertes Cycloalkyl sind Cyclopentyl, Cyclohexyl, Methyl- und Ethylcyclohexyl, und Dimethylcyclohexyl. Beispiele für P-Substituenten als mit Alkyl, Alkoxy, Halogenalkyl und Halogenalkoxy substituiertes Phenyl und Benzyl sind Methylphenyl, Dimethylphenyl, Trimethylphenyl, Ethylphenyl, Methylbenzyl, Methoxyphenyl, Dimethoxyphenyl, Trifluormethylphenyl, Bis-trifluormethylphenyl, Tris-trifluormethylphenyl, Trifluormethoxyphenyl, Bis-trifluormethoxyphenyl und 3,5-Dimethyl-4-methoxyphenyl.

Bevorzugte Phosphingruppen sind solche, die gleiche oder verschiedene und bevorzugt gleiche Reste ausgewählt aus der Gruppe C₁-C₆-Alkyl, unsubstituiertes oder mit 1 bis 3 C₁-C₄-Alkyl oder C₁-C₄-Alkoxy substituiertes Cyclopentyl oder Cyclohexyl, Benzyl und besonders Phenyl, die unsubstituiert oder substituiert sind mit 1 bis 3 C₁-C₄-Alkyl, C₁-C₄-Alkoxy, F, Cl, C₁-C₄-Fluoralkyl oder C₁-C₄-Fluoralkoxy, enthalten.

Die Sekundärphosphinogruppe entspricht bevorzugt der Formel -PR₂R₃, worin R₂ und R₃ unabhängig voneinander einen Kohlenwasserstoffrest mit 1 bis 20 C-Atomen darstellen, der unsubstituiert oder substituiert ist mit Halogen, C₁-C₆-Alkyl, C₁-C₆-Halogenalkyl, C₁-C₆-Alkoxy, C₁-C₆-Halogenalkoxy, Di-C₁-C₄-Alkylamino, (C₆H₅)₃Si, (C₁-C₁₂-Alkyl)₃Si, oder -CO₂-C₁-C₆-Alkyl.

Bevorzugt sind R₂ und R₃ gleiche oder verschiedene und insbesondere gleiche Reste, ausgewählt aus der Gruppe lineares oder verzweigtes C₁-C₆-Alkyl, unsubstituiertes oder mit ein bis drei C₁-C₄-Alkyl oder C₁-C₄-Alkoxy substituiertes Cyclopentyl oder Cyclohexyl, unsubstituiertes oder mit ein bis drei C₁-C₄-Alkyl oder C₁-C₄-Alkoxy substituiertes Benzyl, und insbesondere unsubstituiertes oder mit ein bis drei C₁-C₄-Alkyl, C₁-C₄-Alkoxy, -NH₂, OH, F, Cl, C₁-C₄-Fluoralkyl oder C₁-C₄-Fluoralkoxy substituiertes Phenyl.

Besonders bevorzugt bedeuten R₂ und R₃ gleiche oder verschiedene und insbesondere gleiche Reste, ausgewählt aus der Gruppe C₁-C₆-Alkyl, Cyclopentyl, Cyclohexyl, und unsubstituiertes oder mit ein bis drei C₁-C₄-Alkyl, C₁-C₄-Alkoxy und/oder C₁-C₄-Fluoralkyl substituiertes Phenyl.

Zyklisches Sekundärphosphino kann den Formeln II, IIa, IIb oder IIc entsprechen, 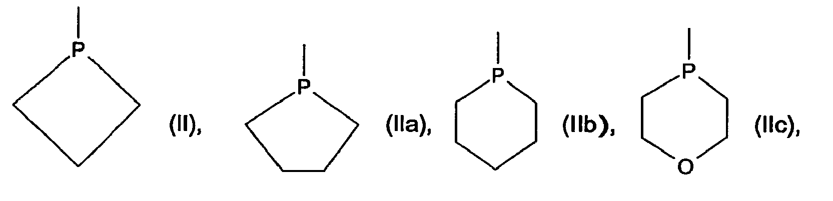 die unsubstituiert oder ein- oder mehrfach substituiert sind mit -OH, C₁-C₈-Alkyl, C₁-C₈-Hydroxyalkyl, C₄-C₈-Cycloalkyl, C₁-C₆-Alkoxy, C₁-C₄-Alkoxy-C₁-C₄-Alkyl, Phenyl, C₁-C₄-Alkyl- oder C₁-C₄-Alkoxyphenyl, Benzyl, C₁-C₄-Alkyl- oder C₁-C₄-Alkoxybenzyl, Benzyloxy, C₁-C₄-Alkyl- oder C₁-C₄-Alkoxybenzyloxy, oder C₁-C₄-Alkyliden-dioxyl.

Die Substituenten können in einer oder beiden α-Stellungen zum P-Atom gebunden sein, um chirale α-C-Atome einzuführen. Bei den Substituenten in einer oder beiden α-Stellungen handelt es sich bevorzugt um C₁-C₄-Alkyl, C₁-C₄-Hydroxyalkyl, oder Benzyl, zum Beispiel um Methyl, Ethyl, n- oder i-Propyl, n-, i- oder t-Butyl, Hydroxymethyl, 1- oder 2-Hydroxyethyl, 1-, 2- oder 3-Hydroxypropyl, Benzyl oder -CH₂-O-C₁-C₄-Alkyl beziehungsweise -CH₂-O-C₆-C₁₀-Aryl.

Bei Substituenten in den β,γ-Stellungen kann es sich zum Beispiel um C₁-C₄-Alkyl, C₁-C₄-Hydroxyalkyl, C₁-C₄-Alkoxy, Benzyloxy, oder -O-CH₂-O-, -O-CH(C₁-C₄-Alkyl)-O-, und -O-C(C₁-C₄-Alkyl)₂-O- handeln. Einige Beispiele sind Methyl, Ethyl, Methoxy, Ethoxy, -O-CH(Methyl)-O-, und -O-C(Methy))₂-O-.

Je nach Art der Substitution und Anzahl der Substituenten können die zyklischen Phosphinreste C-chiral, P-chiral oder C- und P-chiral sein.

An zwei benachbarte C-Atome in den Resten der Formeln II bis IIc kann ein aliphatischer 5- oder 6-Ring oder Benzol ankondensiert sein.

Bevorzugt entspricht das zyklische Sekundärphosphino den Formeln (nur eines der möglichen Diastereomeren angegeben) 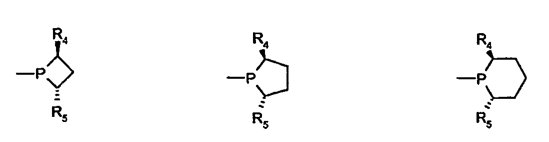 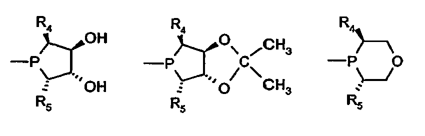 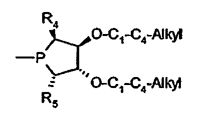 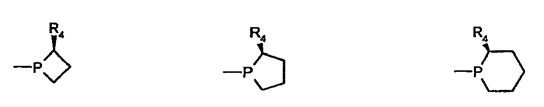 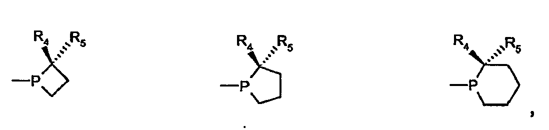 worin
die Reste R₄ und R₅ für C₁-C₄-Alkyl, C₁-C₄-Hydroxyalkyl, zum Beispiel Methyl, Ethyl, n- oder i-Propyl, Hydroxymethyl, 1- oder 2-Hydroxyethyl, Benzyl, oder -CH₂-O-C₁-C₄-Alkyl beziehungsweise -CH₂-O-C₆-C₁₀-Aryl stehen, und R₄ und R₅ gleich oder voneinander verschieden sind.

In einer bevorzugten Ausführungsform entsprechen die Verbindungen der Formel I den Formeln III oder IV, 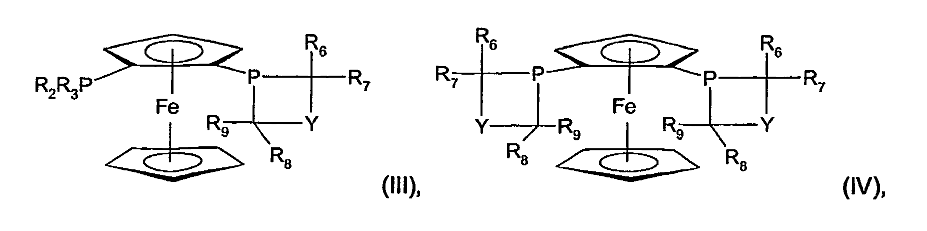 worin
R₂ und R₃ die zuvor angegebenen Bedeutungen haben, einschliesslich der Bevorzugungen, Y für -CH₂-, -CH₂CH₂-, -CH₂CH₂CH₂-, -CH(OH)CH(OH)-, -CH(OC₁-C₄-Alkyl)CH(OC₁-C₄-Alkyl)- steht, oder einen Rest der Formel 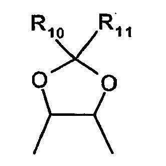 bedeutet,
R₆, R₇, R₈ und R₉ unabhängig voneinander H, C₁-C₄-Alkyl, C₁-C₄-Hydroxyalkyl, Benzyl, oder -CH₂-O-C₁-C₄-Alkyl beziehungsweise -CH₂-O-C₆-C₁₀-Aryl darstellt, und wenigstens einer der Reste R₆, R₇, R₈ und R₉ C₁-C₄-Alkyl oder Benzyl bedeutet,
R₁₀ H oder C₁-C₄-Alkyl ist, und
R₁₁ C₁-C₄-Alkyl bedeutet.

Bevorzugte Ausführungsformen der Verbindungen der Formeln III oder IV sind solche, worin R₆ H, C₁-C₄-Alkyl, C₁-C₄-Hydroxyalkyl, Benzyl, oder -CH₂-O-C₁-C₄-Alkyl beziehungsweise -CH₂-O-C₆-C₁₀-Aryl darstellt und R₇, R₈ und R₉ für H steht, oder worin R₆ und R₈ C₁-C₄-Alkyl, C₁-C₄-Hydroxyalkyl, Benzyl oder -CH₂-O-C₁-C₄-Alkyl beziehungsweise -CH₂-O-C₆-C₁₀-Aryl darstellen und R₇ und R₉ für H stehen, oder worin R₆ und R₇ C₁-C₄-Alkyl, C₁-C₄-Hydroxyalkyl, Benzyl, oder -CH₂-O-C₁-C₄-Alkyl beziehungsweise -CH₂-O-C₆-C₁₀-Aryl bedeuten und R₈ und R₉ für H stehen. Alkyl stellt bevorzugt Methyl, Ethyl, n- oder i- Propyl dar.

Die erfindungsgemässen Verbindungen der Formel I sind Liganden zur Bildung von Metallkomplexen. Es kann vorteilhaft sein bezüglich Stabilität und Handhabbarkeit auch an Luft, die Verbindungen in Phosphoniumsalze mit ein oder zwei monovalenten beziehungsweise einem divalenten Anionen überzuführen. Die Komplexbildung wird hierbei nicht beeinträchtigt. Sofern Liganden der Formel I in Lösung vorliegen, kann es zweckmässig sein, zur Lagerung und Versand in situ Salze zu bilden und Salze nicht zu isolieren. Eine Salzbildung kann aber auch unmittelbar vor der Anwendung, also der Bildung von Metallkomplexen erfolgen. Für die Salzbildung in Lösung können zum Beispiel Mineralsäuren wie Halogenwasserstoffsäuren oder Schwefelsäure verwendet werden, bevorzugt HCl oder HBr. Es können auch Halogenkarbonsäuren, Halogensulfonsäuren oder Komplexsäuren hierfür eingesetzt werden, die vorteilhafter für die Bildung kristalliner und stabiler, isolierbarer Salze eingesetzt werden. Beispiele für Anionen solcher Säuren sind RₐCOO⁻, RₐSO₃⁻, BF₄⁻, PF₆⁻, AsF₆⁻, SbF₆⁻, B(C₆F₅)₄⁻ oder B(3,5-Bistrifluormethyl-phenyl)₄⁻, wobei Rₐ C₁-C₆-Halogenalkyl, C₅-C₁₀-Halogencycloalkyl oder C₆-C₁₀-Halogenaryl bedeutet.

Das Halogenalkyl (zum Beispiel C₁-C₄-Halogenalkyl), Halogencycloalkyl (zum Beispiel C₅-C₆-Halogencycloalkyl) und Halogenaryl (zum Beispiel Halogenphenyl) kann ein oder mehrere Halogenatome enthalten und es kann sich um vollständig halogenierte Reste handeln. Beim Halogen handelt es sich bevorzugt um Cl und ganz besonders bevorzugt um F. Besonders bevorzugt sind perfluorierte Reste. Einige Beispiele sind Mono-, Di- und Trichlormethyl, 1,1,1-Trichlorethyl, 2,2-Dichlorethyl, 1,2,2-Trichlorethyl, Pentachlorethyl, Mono-, Di- und Trifluormethyl, 2,2-Difluorethyl, 1,1,1-Trifluorethyl, 1,2,2-Trifluorethyl, Pentafluorethyl und Pentafluorphenyl.

Rₐ stellt besonders bevorzugt C₁-C₄-Perfluoralkyl dar. Besonders bevorzugte Anionen sind CF₃COO⁻-, CF₃SO₃⁻, BF₄⁻, PF₆⁻, AsF₆⁻, und SbF₆⁻.

Die erfindungsgemässen Ferrocendiphosphine können über ein neues Verfahren hergestellt werden, in dem eine selektive Orthometallierung von Ferrocenylmonophosphinen mit P-O und/oder P-N gebundenen und gegebenenfalls chiralen Resten den Schlüsselschritt der Reaktionsfolge darstellt. Das Verfahren ist modular für die Schaffung unterschiedlicher Substitutionen an den beiden P-Atomen und weist hohe Ausbeuten auf. Zusätzlich kann man auf einfache Weise und hohen Ausbeuten direkt reine Diastereomere oder Paare von einfach trennbaren Paaren von Diastereomeren herstellen. Das Verfahren ist besonders für die Herstellung der erfindungsgemässen Diphosphine im industriellen Massstab geeignet.

Ein weiterer Gegenstand ist ein Verfahren zur Herstellung von Verbindungen der Formel I in Form von Racematen, Gemischen von Diastereomeren oder im wesentlichen reinen Diastereomeren, 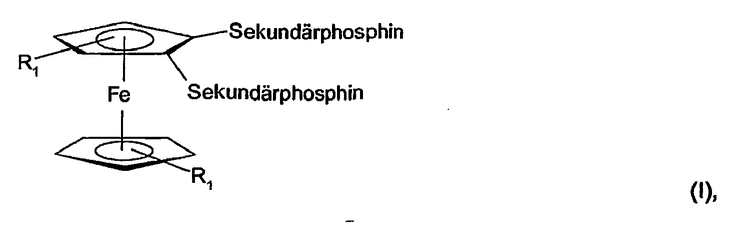 worin
R₁ Wasserstoffatom oder C₁-C₄-Alkyl bedeutet, und wenigstens ein Sekundärphosphin eine unsubstituierte oder substituierte zyklische Phosphingruppe darstellt, umfassend die Schritte
a) Umsetzung einer Verbindung der Formel V 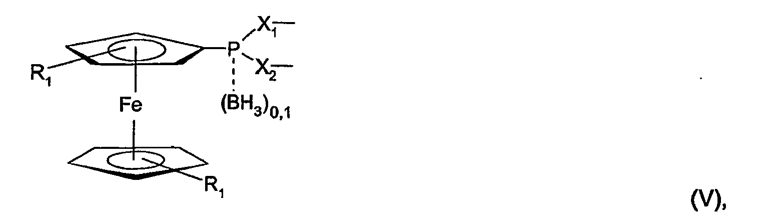 worin
   X₁ und X₂ unabhängig voneinander O oder N- bedeuten, und an die freien Bindungen der O- und N-Atome C-gebundene Kohlenwasserstoff- oder Heterokohlenwasserstoffreste gebunden sind,
   mit wenigstens äquivalenten Mengen Lithiumalkyl, einer Magnesium-Grignardverbindung, oder einem aliphatischen Li-Sekundäramid oder X₃Mg-Sekundäramid zu einer Verbindung der Formel VI, 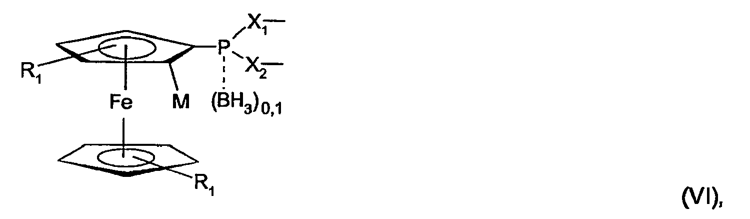 worin
   M für -Li oder -MgX₃ steht und X₃ Cl, Br oder I darstellt,
b) Umsetzung der Verbindung der Formel VI mit wenigstens äquivalenten Mengen eines Disekundäramino-phosphinhalogenids, eines Dialkoxyphosphinhalogenids, Disekundäramino-P(O)-halogenid, Dialkoxy-P(O)-halogenid oder PCl₃ oder PBr₃ zu einer Verbindung der Formel VII 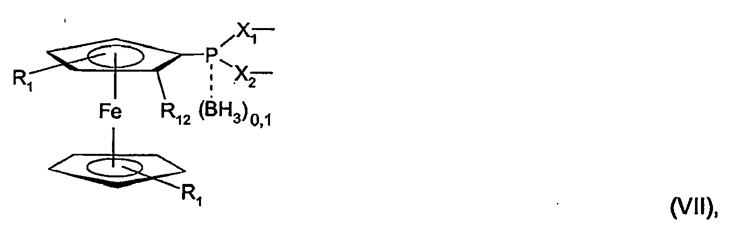 worin
   R₁₂ -PCl₂, -PBr₂, Di(sekundäramino)P-, DialkoxyP-, Disekundäramino-P(O)-, Dialkoxy-P(O)-darstellt, und
b1) aus einer Verbindung der Formel VII, wenn vorhanden, die Borangruppe entfernt, dann die Reste (Hetero)Kohlenwasserstoff-X₁, (Hetero)Kohlenwasserstoff-X₂, oder X₁-(Hetero)-Kohlenwasserstoff-X₂ sowie Disekundäramino oder Dialkoxy mit HCl oder HBr unter Bildung einer -PCl₂-Gruppe oder -PBr₂-Gruppe abspaltet, und danach die -(O)PCl₂-Gruppen, -(O)PBr₂-Gruppe -PCl₂-Gruppen oder -PBr₂-Gruppen zu einer Verbindung der Formel VIII hydriert, oder
b2) aus einer Verbindung der Formel VII die Reste (Hetero)Kohlenwasserstoff-X₁, (Hetero)-Kohlenwasserstoff-X₂, oder X₁-(Hetero)Kohlenwasserstoff-X₂ sowie Disekundäramino oder Dialkoxy mit HCl oder HBr unter Bildung einer -PCl₂-Gruppe oder -PBr₂-Gruppe abspaltet, und danach die -(O)PCl₂-Gruppen, -(O)PBr₂-Gruppen,-PCl₂-Gruppen oder -PBr₂-Gruppen hydriert, und dann die Borangruppe zur Bildung einer Verbindung der Formel VIII, 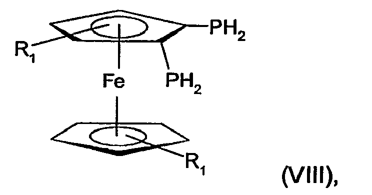 entfernt, oder
c) Umsetzung einer Verbindung der Formel VI mit einem Sekundärphosphin-halogenid zu einer Verbindung der Formel IX, 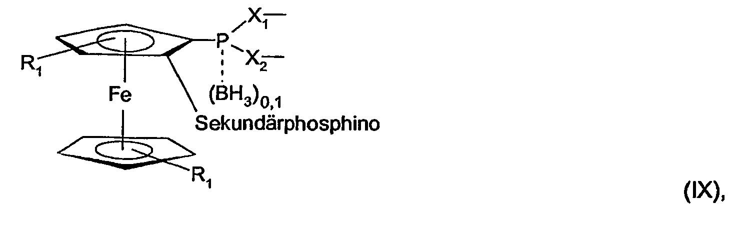
c1) aus einer Verbindung der Formel IX, wenn vorhanden, die Borangruppe entfernt, dann die Reste (Hetero)Kohlenwasserstoff-X₁, (Hetero)Kohlenwasserstoff-X₂, oder X₁-(Hetero)-Kohlenwasserstoff-X₂ mit HCl oder HBr unter Bildung einer -PCl₂-Gruppe oder -PBr₂-Gruppe abspaltet, und danach die -PCl₂-Gruppen oder -PBr₂-Gruppen zu einer Verbindung der Formel X hydriert, oder
c2) aus einer Verbindung der Formel IX die Reste (Hetero)Kohlenwasserstoff-X₁, (Hetero)-Kohlenwasserstoff-X₂, oder X₁-(Hetero) Kohlenwasserstoff-X₂ mit HCl oder HBr unter Bildung einer -PCl₂-Gruppe oder -PBr₂-Gruppe abspaltet, und danach die -PCl₂-Gruppen oder -PBr₂-Gruppen hydriert, und dann die Borangruppe zur Bildung einer Verbindung der Formel X 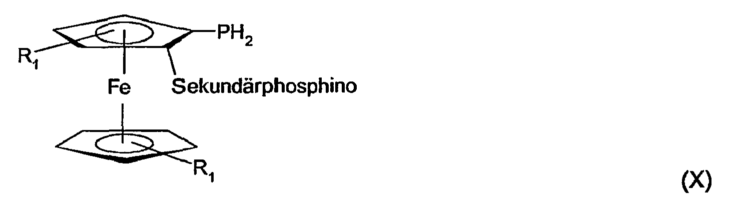 entfernt, oder
d) Umsetzung einer Verbindung der Formel VI mit einem Halogenierungsreagenz zu einer Verbindung der Formel XI 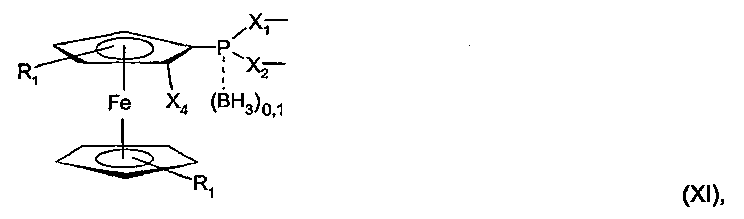 worin X₄ für Cl, Br oder I steht,
d1) aus einer Verbindung der Formel XI, wenn vorhanden, die Borangruppe entfernt, dann die Reste (Hetero)Kohlenwasserstoff-X₁, (Hetero)Kohlenwasserstoff-X₂, oder X₁-(Hetero)-Kohlenwasserstoff-X₂ mit HCl oder HBr unter Bildung einer -PCl₂-Gruppe oder -PBr₂-Gruppe abspaltet, und danach die -PCl₂-Gruppe oder -PBr₂-Gruppe zu einer Verbindung der Formel XII hydriert, oder
d2) aus einer Verbindung der Formel XI die Reste (Hetero)Kohlenwasserstoff X₁, (Hetero)-Kohlenwasserstoff-X₂, oder X₁-(Hetero) Kohlenwasserstoff-X₂ mit HCl oder HBr unter Bildung einer -PCl₂-Gruppe oder -PBr₂-Gruppe abspaltet, und danach die -PCl₂-Gruppen oder -PBr₂-Gruppen hydriert, und dann die Borangruppe zur Bildung einer Verbindung der Formel XII 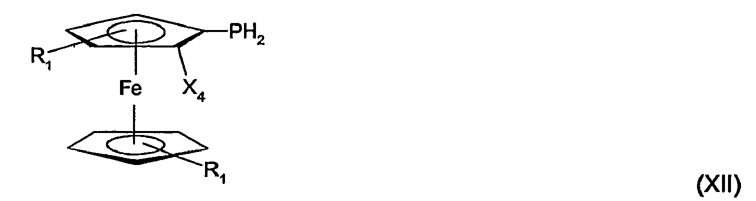 entfernt, und
d3) die Verbindung der Formel XII mit einem metallierten Sekundärphosphid zu einer Verbindung der Formel X umsetzt,
e) Umsetzung der Verbindung der Formel VII mit wenigstens 2 Äquivalenten und der Verbindung der Formel X mit wenigstens 1 Äquivalent eines zyklischen Sulfats oder eines offenkettigen Disulfonats zur Herstellung von Verbindungen der Formel I, worin eine oder beide Sekundärphosphinogruppen zyklisches Sekundärphosphino darstellen, oder
f) Umsetzung einer Verbindung der Formel XII mit wenigstens 1 Äquivalent eines zyklischen Sulfats oder eines offenkettigen Disulfonats zur Herstellung von Verbindungen der Formel XIII, 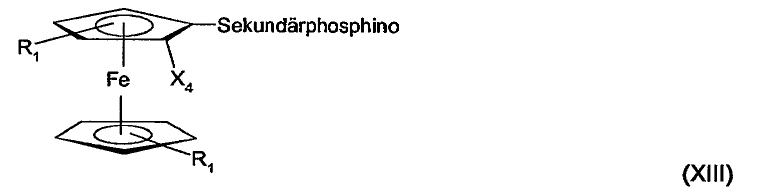 worin Sekundärphosphino zyklisches Sekundärphosphino darstellt, die gegebenenfalls mit BH₃ geschützt ist, und danach Umsetzung einer Verbindung der Formel XIII mit wenigstens 1 Äquivalent Lithiumalkyl und dann mit wenigstens 1 Äquivalent Sekundärphosphin-halogenid zu einer Verbindung der Formel I.

Aliphatisches Li-Sekundäramid oder X₃Mg-Sekundäramid in Stufe a) kann sich von Sekundäraminen ableiten, die 2 bis 18, bevorzugt 2 bis 12, und besonders bevorzugt 2 bis 10 C-Atome enthalten. Bei den an das N-Atom gebundenen aliphatischen Resten kann es sich um Alkyl, Cycloalkyl oder Cycloalkyl-alkyl handeln, oder es kann sich um N-heterocyclische Ringe mit 4 bis 12, und bevorzugt 5 bis 7 C-Atomen handeln. Beispiele für an das N-Atom gebundene Reste sind Methyl, Ethyl, n-Propyl, n-Butyl. Pentyl, Hexyl, Cyclopentyl, Cyclohexyl, und Cyclohexylmethyl. Beispiele für N-heterocyclische Ringe sind Pyrrolidin, Piperidin, Morpholin, N-Methylpiperazin, 2,2,6,6-Tetramethylpiperidin, und Azanorbornan. In einer bevorzugten Ausführungsform entsprechen die Amide den Formeln Li-N(C₁-C₄-Alkyl)₂ oder X₃Mg-N(C₁-C₄-Alkyl)₂, worin Alkyl insbesondere Methyl ist.

In den Verbindungen der Formel V bedeuten X₁ und X₂ bevorzugt N.

Im Rahmen der Erfindung können an die Gruppen X₁ und X₂ zum Beispiel folgende Kohlenwasserstoff- oder Heterokohlenwasserstoffreste gebunden sein:
ein monovalenter (Hetero)Kohlenwasserstoffrest an je X₁ und X₂ oder ein bivalenter (Hetero)Kohlenwasserstoffrest an X₁ und X₂, wenn X₁ und X₂ O bedeuten;
zwei monovalente (Hetero)Kohlenwasserstoffreste an je X₁ und X₂, wenn X₁ und X₂ N bedeuten;
zwei bivalente (Hetero)Kohlenwasserstoffreste an je X₁ und X₂, wenn X₁ und X₂ N bedeuten, wobei die bivalenten (Hetero)Kohlenwasserstoffreste mit einer Bindung, Methylen oder Ethylen überbrückt sein können;
ein bivalenter (Hetero)Kohlenwasserstoffrest an X₁ und zwei monovalente Reste an X₂,
wenn X₁ und X₂ N bedeuten, wobei ein monovalenter Rest an den bivalenten (Hetero)Kohlenwasserstoffrest gebundenes Methylen oder Ethylen ist;
ein monovalenter (Hetero)Kohlenwasserstoffreste an je X₁ und X₂, und ein bivalenter (Hetero)Kohlenwasserstoffrest an je X₁ und X₂, wenn X₁ und X₂ N bedeuten;
zwei monovalente (Hetero)Kohlenwasserstoffreste an X₁ und ein bivalenter (Hetero)Kohlenwasserstoffrest an X₂ gebunden sind, wenn X₁ und X₂ N bedeuten;
zwei bivalente (Hetero)Kohlenwasserstoffreste an je X₁ X₂ gebunden sind, wenn X₁ und X₂ N bedeuten;
X₁ O bedeutet und ein monovalenter (Hetero)Kohlenwasserstoffrest an X₁ gebunden ist, sowie X₂ N bedeutet und zwei monovalente (Hetero)Kohlenwasserstoffreste oder ein bivalenter (Hetero)Kohlenwasserstoffrest an X₂ gebunden sind;
X₁ O und X₂ N bedeuten, und ein bivalenter (Hetero)Kohlenwasserstoffrest an X₁ und X₂ sowie ein monovalenter (Hetero) Kohlenwasserstoffrest an X₂ gebunden sind;
ein bivalenter, aromatischer 1,1'-(Hetero)Kohlenwasserstoffrest, wenn X₁ und X₂ O bedeuten; oder
ein bivalenter, aromatischer 1,1'-(Hetero)Kohlenwasserstoffrest an X₁ und X₂ und ein monovalenter (Hetero)Kohlenwasserstoffrest an je X₁ und X₂, wenn X₁ und X₂ N bedeuten.

Bei den an X₁ und X₂ C-gebundenen Kohlenwasserstoff- oder Heterokohlenwasserstoffresten kann es sich
a) um gesättigte oder ungesättigte, geradkettige, verzweigte oder zyklische und monovalente Reste handeln, wobei zwei monovalente Reste an X₁ und X₂ in der Bedeutung von N gebunden sind;
b) um gesättigte, ungesättigte, geradkettige, verzweigte und/oder zyklische beziehungsweise bizyklische bivalente Reste handeln, die an X₁ und/oder X₂ gebunden sind, wenn X₁ und X₂ N bedeuten, und einen 4- bis 7-gliedrigen Ring bilden, oder
c) um gesättigte, ungesättigte, geradkettige, verzweigte und/oder zyklische beziehungsweise bizyklische bivalente Reste handeln, die ein O- und N-Atom, oder zwei N-Atome einfach oder zweifach überbrücken und mit der Gruppe -X₁-P-X₂- einen 5- bis 7-gliedrigen Ring bilden.

Heterokohlenwasserstoffreste können Heteroatome ausgewählt aus der Gruppe O, S und N(C₁-C₄-Alkyl) enthalten. Die Anzahl der Heteroatome beträgt bevorzugt 1 bis 4, bevorzugter 1 bis 3 und besonders bevorzugt 1 oder 2. Die Kohlenwasserstoff oder Heterokohlenwasserstoffreste können 1 bis 18, bevorzugt 1 bis 12, und besonders bevorzugt 1 bis 8 C-Atome und gegebenenfalls Heteroatome enthalten. Die Reste können ein- oder mehrfach, vorzugsweise ein- bis dreifach oder ein- oder zweifach substituiert sein, zum Beispiel mit Phenyl, Phenoxy, Benzyl, Benzyloxy, C₁-C₄-Alkylphenyl, C₁-C₄-Alkylphenoxy, C₁-C₄-Alkylbenzyl, C₁-C₄-Alkylbenzyloxy, C₁-C₄-Alkoxyphenyl, C₁-C₄-Alkoxyphenoxy, C₁-C₄-Alkoxybenzyl, C₁-C₄-Alkoxybenzyloxy, C₁-C₄-Alkylthiophenyl, C₁-C₄-Alkylthiophenoxy, C₁-C₄-Alkylthiobenzyl, C₁-C₄-Alkylthiobenzyloxy, Di(C₁-C₄-Alkyl)aminophenyl, Di(C₁-C₄-Alkyl)aminophenoxy, Cyclohexyl, Cyclopentyl, C₁-C₄-Alkylcyclohexyl, C₁-C₄-Alkylcyclopentyl, C₁-C₄-Alkoxycyctohexyl, C₁-C₄-Alkoxycyclopentyl, Fluor, C₁-C₄-Alkyl, C₁-C₄-Fluoralkyl, C₁-C₄-Alkoxy, C₁-C₄-Alkylthio, Di(C₁-C₄-Alkyl)amino, C₁-C₄-Alkoxy-C₁-C₄-Alkyl, C₁-C₄-Alkylthio-C₁-C₄-Alkyl, oder Di(C₁-C₄-Alkyl)amino-C₁-C₄-Alkyl. Eine Substitution in den α- oder β-Stellungen zu den Gruppen X₁ und X₂ ist insofern bevorzugt, als der Rest chirale C-Atome aufweist, die bei der Metallierung und Folgereaktionen eine optische Induktion verursachen können. Einige spezifische Substituenten sind Methyl, Ethyl, n- und i-Propyl, n-, i- und t-Butyl, Trifluormethyl, Methoxy, Ethoxy, Propoxy, i-Propoxy, Butoxy, i-Butoxy, t-Butoxy, Methylthio, Ethylthio, Dimethylamino, Diethylamino, Phenyl, Phenoxy, Methoxyphenyl und Methoxyphenoxy.

Bei den an X₁ und X₂ C-gebundenen Kohlenwasserstoff- oder Heterokohlenwasserstoffresten kann es sich bei monovalenten Resten zum Beispiel um unsubstituiertes oder substituiertes C₁-C₁₈-, bevorzugt C₁-C₁₂-, und besonders bevorzugt C₁-C₈-(Hetero)Alkyl; unsubstituiertes oder substituiertes C₂-C₁₈-, bevorzugt C₂-C₁₂-, und besonders bevorzugt C₃-C₈-(Hetero)Alkenyl; unsubstituiertes oder substituiertes C₃-C₁₂- und bevorzugt C₃-C₈-(Hetero)Cycloalkyl, unsubstituiertes oder substituiertes C₃-C₁₂- und bevorzugt C₃-C₈-(Hetero)Cycloalkenyl, unsubstituiertes oder substituiertes C₃-C₁₂- und bevorzugt C₃-C₈-(Hetero)Cycloalkyl-C₁-C₄-alkyl, unsubstituiertes oder substituiertes C₃-C₁₂- und bevorzugt C₃-C₈-(Hetero)Cycloalkenyl-C₁-C₄-alkyl, unsubstituiertes oder substituiertes C₆-C₁₄-(Hetero)aryl, und C₆-C₁₄-(Hetero)aryl-C₁-C₄-alkyl handeln. Bevorzugt sind gesättigte und aromatische Kohlenwasserstoff- oder Heterokohlenwasserstoffreste.

Bei monovalenten Kohlenwasserstoffresten kann es sich um lineares oder verzweigtes C₁-C₁₂-Alkyl, bevorzugt C₁-C₈-Alkyl und besonders bevorzugt C₁-C₄-Alkyl, C₃-C₈- und bevorzugt C₄-C₆-Cycloalkyl, C₃-C₈-Cycloalkyl- und bevorzugt C₄-C₆-Cycloalkyl-methyl oder -ethyl, C₆-C₁₄- und bevorzugt C₆-C₁₀-Aryl, C₇-C₁₅-Aralkyl und bevorzugt C₇-C₁₁-Aralkyl handeln. Einige spezifische Beispiele sind Methyl, Ethyl, n- und i-Propyl, n-Butyl, Pentyl, Hexyl, Heptyl, Octyl, Cyclopropyl, Cyclobutyl, Cyclopentyl, Cyclohexyl, Cycloheptyl, Cyclooctyl, Cyclobutylmethyl, Cyclopentyl-methyl, Cyclohexylmethyl, Cyclobutyl-ethyl, Cyclopentyl-ethyl, Cyclohexyl-ethyl, Phenyl, Naphthyl, Benzyl und Phenylethyl. Wenn eine chirale Induktion erzielt werden soll, zum Beispiel bei Ferrocenen, dann sind die Kohlenwasserstoffreste bevorzugt in α- und/oder β-Stellung zu X₁ und/oder X₂ substituiert, zum Beispiel mit C₁-C₄-Alkyl, C₁-C₄-Alkoxy, (C₁-C₄-Alkyl)₂N-, C₁-C₄-Alkoxymethyl, C₁-C₄-Alkoxyethyl, (C₁-C₄-Alkyl)₂N-methyl oder -ethyl, Phenyl, Methylphenyl, Methoxyphenyl, Phenoxy, 2-Anisyl, Benzyl oder Benzyloxy.

Einige Beispiele für monovalente Heterokohlenwasserstoffreste sind C₁-C₈-Alkoxy-C₂-C₄-alkyl, (C₁-C₄-Alkyl)₂N-C₂-C₄-alkyl, C₅-C₇-Cycloalkoxy-C₂-C₄-alkyl, C₄-C₁₀-(Hetero)Aryloxy-C₂-C₄-alkyl, C₄-C₇-Heterocycloalkyl-C₁-C₄-alkyl, C₄-C₁₀-Heteroaryl-C₁-C₄-alkyl, Einige spezifische Methoxyethyl, Ethoxyethyl, Dimethylaminoethyl, Diethylaminoethyl, Cyclohexyloxyethyl, Phenoxyethyl, N-Methylmorpholinylmethyl oder -ethyl, N-Methylpiperidinylmethyl oder -ethyl, Pyridinylmethyl oder -ethyl, und Pyrrolidinylmethyl oder -ethyl.

Bivalente Kohlenwasserstoffreste, die an je X₁ und X₂ gebunden sind und X₁ und X₂ je N bedeuten und mit dem N-Atom einen 4 bis 7-gliedrigen Ring bilden, können 2 bis 8, bevorzugt 2 bis 6, und bevorzugter 2 bis 4 C-Atome enthalten und sind bevorzugt lineares oder verzweigtes, unsubstituiertes oder substituiertes Alkylen, an das gegebenenfalls aliphatische oder aromatische Ringe kondensiert sind. Die Kohlenwasserstoffkette kann mit O-Atomen und oder -N(C₁-C₄-Alkyl) unterbrochen sein. Beispiele für bivalente Kohlenwasserstoffreste sind Trimethylen, Tetramethylen, Pentamethylen, -(CH₂)₂-O-(CH₂)₂-, -(CH₂)₂-N(CH₃)-(CH₂)₂-, Bivalente Kohlenwasserstoffreste bilden zusammen mit den Atomen, an die sie gebunden sind, einen heterocyclischen Ring. Wenn eine chirale Induktion erzielt werden soll, zum Beispiel bei Ferrocenen, dann sind die Kohlenwasserstoffreste bevorzugt in α- oder β-Stellung zu X₁ und/oder X₂ substituiert, zum Beispiel mit C₁-C₄-Alkyl, C₁-C₄-Alkoxy, C₁-C₄-Alkoxymethyl, C₁-C₄-Alkoxyethyl, -N(C₁-C₄-Alkyl), (C₁-C₄-Alkyl)₂N-methyl oder -ethyl, Phenyl, 2-Anisyl oder Benzyl substituiert. Wenn beide N-Atome mit zwei bivalenten Resten überbrückt sind, so leiten sich diese Reste von zyklischen Diaminen ab, zum Beispiel Piperazin.

Bivalente Kohlenwasserstoffreste, die an X₁ und X₂ gebunden sind und X₁ und X₂ je N bedeuten, leiten sich vorzugsweise von 1,2- oder 1,3-Diaminen ab, wobei eine Aminogruppe Teil eines Ringes sein kann. Es kann sich um lineares oder verzweigtes 1,2- oder 1,3-C₂-C₁₂-Alkylen, bevorzugt 1,2- oder 1,3-C₂-C₈-Alkylen und besonders bevorzugt 1,2- oder 1,3-C₂-C₄-Alkylen, 1,2- oder 1,3-C₃-C₈- und bevorzugt 1,2- oder 1,3-C₄-C₆-Cycloalkylen, 1-C₃-C₈-Cycloalkyl- und bevorzugt 1-C₄-C₆-Cycloalkyl-2-methylen oder -ethylen, C₆-C₁₄- und bevorzugt 1,2-C₆-C₁₀-Arylen, und C₆-C₁₀-Aralk-1-yl-2-methylen handeln. Einige spezifische Beispiele sind Ethylen, n- und i-Propylen, n- oder i-Butylen, Cyclopropyl-1,2-en, Cyclobutyl-1,2-en, Cyclopentyl-1,2-en, Cyclohexyl-1,2-en, Cycloheptyl-1,2-en, Cyclooctyl-1,2-en, Cyclobut-1-yl-2-methylen, Cyclopent-1-yl-2-methylen, Cyclohex-1-yl-2-methylen, Cyclobut-1-yl-2-ethylen, Cyclopent-1-yl-2-ethylen, Cyclohex-1-yl-2-ethylen, 1,2-Phenylen, 1,2-Naphthylen, Phen-1-yl-2-methylen und Phen-1-yl-2-ethylen. Wenn eine chirale Induktion erzielt werden soll, zum Beispiel bei Ferrocenen, dann sind die Kohlenwasserstoffreste bevorzugt in α- und/ oder β-Stellung zu X₁ und/oder X₂ substituiert, zum Beispiel mit C₁-C₄-Alkyl, C₁-C₄-Alkoxymethyl, C₁-C₄-Alkoxyethyl, -N(C₁-C₄-Alkyl), (C₁-C₄-Alkyl)₂N-methyl oder -ethyl, Phenyl, 2-Anisyl oder Benzyl.

Bei bivalenten Kohlenwasserstoffresten, die an X₁ und X₂ gebunden sind und X₁ und X₂ je N bedeuten, kann es sich auch um 1,1'-Biphenylen, 1,1'Binaphthylen und 1,1'-Bispyridin handeln.

Bevorzugte Phosphingruppen in Formel V entsprechen den Formeln: 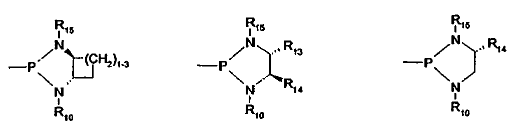 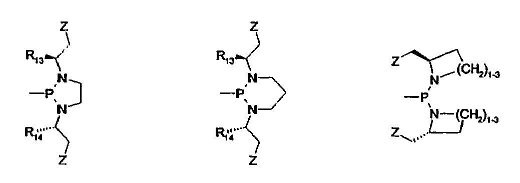 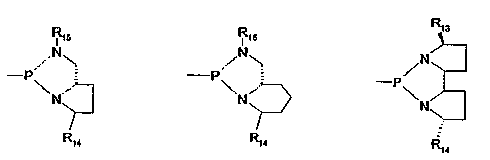 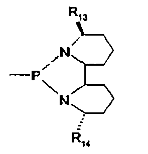 worin
R₁₅ und R₁₆ gleich oder verschieden und bevorzugt gleich sind, und C₁-C₄-Alkyl, C₁-C₄-Alkoxyethyl, (C₁-C₄-Alkyl)₂N-ethyl bedeuten,
R₁₃ und R₁₄ gleich oder verschieden und bevorzugt gleich sind, und H, C₁-C₄-Alkyl, Phenyl oder Methylphenyl darstellen, und
Z für H, C₁-C₄-Alkyl, C₁-C₄-Alkoxy, C₁-C₄-Alkylthio, -N(C₁-C₄-Alkyl)₂, Phenyl, Phenoxy, Methoxyphenyl oder Methoxyphenoxy steht.

Einige weitere Beispiele für Z sind Methyl, Ethyl, Methoxy, Ethoxy, Methylthio und Dimethylamino.

Bei der Metallierung von Aromaten handelt es sich um bekannte Reaktionen, die zum Beispiel von M. Schlosser (Editor) in Organometallics in Synthesis, Johnson Wiley & Sons (1994) oder in Jonathan Clayden Organolithiums: Selectivity for Synthesis (Tetrahedron Organic Chemistry Series), Pergamon Press (2002) beschrieben sind.

Wenigstens äquivalente Mengen bedeutet im Rahmen der Erfindung die Verwendung von 1 bis 1,2 Äquivalenten einer Magnesium-Grignardverbindung, oder einem aliphatischen Li-Sekundäramid oder X₃Mg-Sekundäramid pro reagierender =CH-Gruppe im Cyclopentadienylring.

Die Reaktion wird zweckmässig bei niedrigen Temperaturen durchgeführt, zum Beispiel 20 bis -100 °C, bevorzugt 0 bis -80 °C. Die Reaktionszeit beträgt etwa von 2 bis zu 5 Stunden. Die Reaktion wird vorteilhaft unter einem inerten Schutzgas durchgeführt, zum Beispiel Stickstoff oder Edelgasen wie Argon.

Die Reaktion wird vorteilhaft in Gegenwart von inerten Lösungsmitteln durchgeführt. Solche Lösungsmittel können alleine oder als Kombination aus wenigstens zwei Lösungsmitteln eingesetzt werden. Beispiele für Lösungsmittel sind aliphatische, cycloaliphatische und aromatische Kohlenwasserstoffe sowie offenkettige oder cyclische Ether. Spezifische Beispiele sind Petrolether, Pentan, Hexan, Heptan, Cyclohexan, Methylcyclohexan, Benzol, Toluol, Xylol, Diethylether, Dibutylether, Tertiärbutylmethylether, Ethylenglykoldimethyl- oder -diethylether, Tetrahydrofuran und Dioxan.

Verbindungen der Formel V sind bekannt oder nach bekannten beziehungsweise analogen Verfahren herstellbar. Man geht von zum Beispiel mono-lithierten Ferrocenen aus, die man mit Monohalogenphosphinen der Formel X₅P(X₁-)X₂-, worin X₅ bevorzugt Cl oder Br bedeutet, X₁ und X₂ O oder N bedeuten, und an die freien Bindungen von X₁- und X₂- ein Kohlenwasserstoffrest gebunden ist, umsetzt. Im Anschluss an die Reaktion kann in an sich bekannter Weise das Boran BH₃, wenn dessen Anwesenheit gewünscht ist, eingeführt werden, zum Beispiel durch Umsetzung der Reaktionsmischung mit einem Borankomplex wie BH₃·S(CH₃)₂. Monohalogenphosphine der Formel X₅P(X₁-)X₂- sind bekannt oder aus Phosphortrichlorid durch Umsetzung mit Alkoholen, Diolen, Aminen, Aminoalkoholen oder Diaminen in an sich bekannter Weise erhältlich.

In Verfahrensstufe b) verwendete Disekundäramino-phosphinhalogenide und Dialkoxyphosphinhalogenide sowie die Disekundäramino-phosphin(O)halogenide und Dialkoxyphosphin-(O)halogenide sind bevorzugt [(C₁-C₄-Alkyl)₂N]₂P-X₅, (C₁-C₄-AlkylO)₂P-X₅, [(C₁-C₄-Alkyl)₂N]₂P(O)-X₅ und (C₁-C₄-AlkylO)₂P(O)-X₅ worin X₅ für Br und bevorzugt für Cl steht. Einige Beispiele für Alkyl sind Methyl, Ethyl und Propyl, wobei Methyl besonders bevorzugt ist. Disekundäramino-phosphinhalogenide und Dialkoxyphosphinhalogenide können mit Boran geschützt sein. Die Reaktionsbedingungen sind analog oder ähnlich zu den für Verfahrensstufe a) zuvor beschriebenen Bedingungen.

Die Umsetzungen in den Verfahrensstufen b1, b2, c1, c2, d1 und d2 sind an sich bekannt. Die Entfernung der Borangruppe erst in der letzten Reaktionsstufe bietet den Vorteil, dass reaktionsempfindliche Gruppen geschützt bleiben.

Die Abspaltung der Borangruppe kann zum Beispiel durch Zugabe von Reagenzien wie zum Beispiel sekundären Aminen mit C₁-C₄-Alkylgruppen, Morpholin, 1,8-Diazabicyclo[5,4.0]-undec-7-en (DBU), 1,4-Diazabicyclo[2.2.2]-octan zur gelösten Verbindung der Formel III, ausreichend langes Rühren bei Temperaturen von 20 bis 100 °C, und Entfernen der flüchtigen Bestandteile vorteilhaft im Vakuum erfolgen. Methoden für das Entfernen von Boran sind zum Beispiel von M. Ohff et al. in Synthesis (1998), Seite 1391 beschrieben.

Die Bildung von -PCl₂-Gruppe oder -PBr₂-Gruppen ist ebenfalls bekannt und zum Beispiel von A. Longeau et al. in Tetrahedron: Asymmetry, 8 (1997) Seiten 987-990 beschrieben. Als Reagenz verwendet man zweckmässig organische Lösungen von HCl oder HBr in zum Beispiel Ethern, die man bei niedrigen Temperaturen (zum Beispiel -20 bis 30 °C) zu gelösten Verbindungen der Formel VII, IX oder XI mit oder ohne Borangruppe gibt.

-PCl₂-Gruppen oder -PBr₂-Gruppen können in an sich bekannter Weise hydriert werden, wie zum Beispiel mit Metallhydriden wie LiH, NaH, KH, Li(AlH₄) NaBH₄. Die Reaktion wird vorteilhaft in Gegenwart von Lösungsmitteln und bei Temperaturen von -80 °C bis 50 °C vorgenommen. Die erhaltenen primären Phosphine können isoliert oder direkt weiter umgesetzt werden.

Die Umsetzung mit einem Sekundärphosphin-halogenid gemäss Verfahrensstufe c) ist an sich bekannt und in den Beispielen beschrieben. Die Reaktionsbedingungen sind analog oder ähnlich zu den für Verfahrensstufe a) zuvor beschriebenen Bedingungen.

Die Umsetzung einer Verbindung der Formel VI mit einem Halogenierungsreagenz gemäss Verfahrensstufe d) ist ebenfalls bekannt. Als Halogenierungsreagenzien eignen sich zum Beispiel Cl₂, Br₂, I₂, Interhalogene wie ClBr, Brl, oder polyhalogenierte aliphatische Kohlenwasserstoffe wie CF₃Br, Hexachlorethan, BrCF₂-CF₂Br oder 1,1,2,2-Tetrabromethan. Die Reaktionstemperatur kann -40 °C bis 50 °C betragen. Geeignete Lösungsmittel sind zuvor für Verfahrensstufe a) erwähnt worden. Die erhaltenen halogenierten Verbindungen können isoliert oder direkt weiter umgesetzt werden.

Umsetzungen mit einem metallierten Sekundärphosphid gemäss Verfahrensstufen d3) sind ebenfalls bekannt. Bevorzugtes Metall ist Lithium.

Die Reaktion der Verfahrensstufe f) ist an sich bekannt und kann ähnlich wie Verfahrensstufe c) durchgeführt werden.

Umsetzungen primärer Phosphine mit zyklischen Sulfaten sind ebenfalls beschrieben, siehe M. J. Burk, J. Amer. Chem. Soc., 1991, 113, 8518-9, M. J. Burk, Acc. Chem. Res., 2000, 33, 363-72 und U. Behrens, M. J. Burk, A. Gerlach, W. Hems, Angew. Chemie, int. Ed., 2000, 112, 2057-2060. Zyklische Sulfate sind bekannt oder können nach analogen Verfahren hergestellt werden. Die Reaktion wird in Lösung durchgeführt, zum Beispiel Ethern. Vorteilhaft werden äquimolare Mengen starker Basen mitverwendet, zum Beispiel Li-Sekundäramide wie Lithiumdiisopropylamid. Umsetzungen mit offenkettigen Disulfonaten sind ebenfalls bekannt und zum Beispiel von T. V. RajanBabu et al., J. Amer. Chem. Soc. 2001, 123, Seiten 10207 bis 10213. Einige Beispiele für Sulfonate sind Di-phenylsulfonate, Di-tosylate, Di-mesylate, Di-trifluormethylsulfonate von offenkettigen Diolen.

Die Isolierung und Reinigung der erfindungsgemässen Ferrocendiphosphine kann nach an sich bekannten Verfahren vorgenommen werden, zum Beispiel Fällung und Filtration oder Extraktion. Eine Reinigung kann durch Destillation, Umkristallisation oder mit chromatographischen Methoden vorgenommen werden.

Die erfindungsgemässen Ferrocendiphosphine werden trotz der räumlich beanspruchenden zyklischen Sekundärphosphingruppen in überraschend hohen Ausbeuten erhalten, und häufig wird überwiegend ein Diastereomeres gebildet

Gegenstand der Erfindung sind auch die beim erfindungsgemässen Verfahren durchlaufenen Zwischenprodukte der Formeln VII, IX und XI, 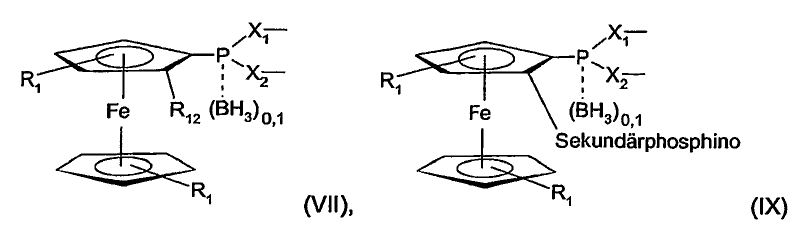 und 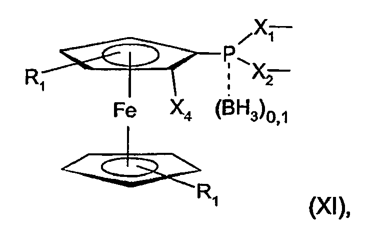 worin R₁, X₁, X₂, R₁₂ und X₂ die zuvor angegebenen Bedeutungen haben, einschliesslich der Bevorzugungen.

Ein weiter Gegenstand der Erfindung sind Verbindungen der Formeln VIII, X und XII, 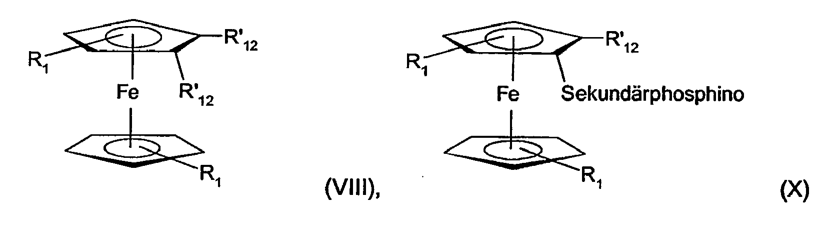 und 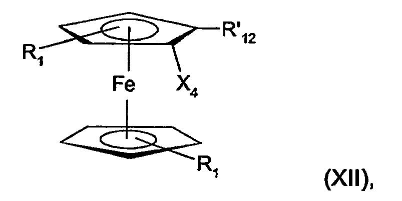 worin
R'₁₂ für -PCl₂, -PBr₂ oder -PH₂ steht, und R₁ und X₄ die zuvor angegebenen Bedeutungen haben, einschliesslich der Bevorzugungen.

Ein anderer Gegenstand der Erfindung sind Verbindungen der Formel XIII 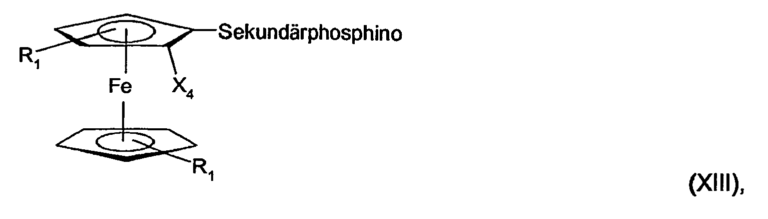 worin
R₁ und X₄ die zuvor angegebenen Bedeutungen haben, einschliesslich der Bevorzugungen, und Sekundärphosphino zyklisches Sekundärphosphino darstellt.

Die erfindungsgemässen Verbindungen der Formel I sind Liganden für Metallkomplexe ausgewählt aus der Gruppe der TM8-Metalle, besonders aus der Gruppe Ru, Rh und Ir, die hervorragende Katalysatoren oder Katalysatorvorläufer für asymmetrische Synthesen, zum Beispiel die asymmetrische Hydrierung von prochiralen, ungesättigten, organischen Verbindungen darstellen. Werden prochirale ungesättigte organische Verbindungen eingesetzt, kann ein sehr hoher Überschuss optischer Isomerer bei der Synthese organischer Verbindungen induziert und ein hoher chemischer Umsatz in kurzen Reaktionszeiten erzielt werden. Die erzielbaren Enantioselektivitäten und Katalysatoraktivitäten sind ausgezeichnet.

Ein weiterer Gegenstand der Erfindung sind Metallkomplexe von Metallen ausgewählt aus der Gruppe der TM8-Metalle mit Verbindungen der Formeln I als Liganden.

Als Metalle kommen zum Beispiel Cu, Ag, Au, Ni, Co, Rh, Pd, Ir, Ru und Pt in Frage. Bevorzugte Metalle sind Rhodium und Iridium sowie Ruthenium, Platin und Palladium.

Besonders bevorzugte Metalle sind Ruthenium, Rhodium und Iridium.

Die Metallkomplexe können je nach Oxidationszahl und Koordinationszahl des Metallatoms weitere Liganden und/oder Anionen enthalten. Es kann sich auch um kationische Metallkomplexe handeln. Solche analoge Metallkomplexe und deren Herstellung sind vielfach in der Literatur beschrieben.

Die Metallkomplexe können zum Beispiel den allgemeinen Formeln XIV und XV entsprechen,

A₁MeLₙ (XIV),

(A₁MeLₙ)^{(z+)}(E⁻)_{z} (XV),

worin
A₁ für eine Verbindung der Formel I steht,
L für gleiche oder verschiedene monodentate, anionische oder nicht-ionische Liganden steht, oder zwei L für gleiche oder verschiedene bidentate, anionische oder nicht-ionische Liganden steht;
n für 2, 3 oder 4 steht, wenn L einen monodentaten Liganden bedeutet, oder n für 1 oder 2 steht, wenn L einen bidentaten Liganden bedeutet;
z für 1, 2 oder 3 steht;
Me ein Metall ausgewählt aus der Gruppe Rh, Ir und Ru bedeutet; wobei das Metall die Oxidationsstufen 0, 1, 2, 3 oder 4 aufweist;
E- das Anion einer Sauerstoffsäure oder Komplexsäure ist; und
die anionischen Liganden die Ladung der Oxidationsstufen 1, 2, 3 oder 4 des Metalls ausgleichen.

Für die Verbindungen der Formeln I gelten die zuvor beschriebenen Bevorzugungen und Ausführungsformen.

Monodentate nicht-ionische Liganden können zum Beispiel ausgewählt sein aus der Gruppe der Olefine (zum Beispiel Ethylen, Propylen), Allyle (Allyl, 2-Methallyl), solvatisierenden Lösungsmitteln (Nitrile, lineare oder cyclische Ether, gegebenenfalls N-alkylierte Amide und Lactame, Amine, Phosphine, Alkohole, Carbonsäureester, Sulfonsäureester), Stickstoffmonoxid und Kohlenmonoxid.

Monodentate anionische Liganden können zum Beispiel ausgewählt sein aus der Gruppe Halogenid (F, Cl, Br, I), Pseudohalogenid (Cyanid, Cyanat, Isocyanat) und Anionen von Carbonsäuren, Sulfonsäuren und Phosphonsäuren (Carbonat, Formiat, Acetat, Propionat, Methylsulfonat, Trifluormethylsulfonat, Phenylsulfonat, Tosylat).

Bidentate nicht-ionische Liganden können zum Beispiel ausgewählt sein aus der Gruppe der linearen oder cyclischen Diolefine (zum Beispiel Hexadien, Cyclooctadien, Norbornadien), Dinitrile (Malondinitril), gegebenenfalls N-alkylierte Carbonsäurediamide, Diaminen, Diphosphinen, Diolen, Acetonylacetonate, Dicarbonsäurediester und Disulfonsäurediester.

Bidentate anionische Liganden können zum Beispiel ausgewählt sein aus der Gruppe der Anionen von Dicarbonsäuren, Disulfonsäuren und Diphosphonsäuren (zum Beispiel von Oxalsäure, Malonsäure, Bernsteinsäure, Maleinsäure, Methylendisulfonsäure und Methylendiphosphonsäure).

Bevorzugte Metallkomplexe sind auch solche, worin E für -Cl⁻, -Br⁻ , -I⁻, ClO₄⁻, CF₃SO₃⁻, CH₃SO₃⁻, HSO₄⁻, (CF₃SO₂)₂N⁻, (CF₃SO₂)₃C⁻, Tetraarylborate wie zum Beispiel B(Phenyl)₄⁻, B[Bis(3,5-trifluormethyl)phenyl]₄⁻, B[Bis(3,5-dimethyl)phenyl]₄⁻, B(C₆F₅)₄⁻ und B(4-Methylphenyl)₄⁻, BF₄⁻, PF₆⁻, SbCl₆⁻, AsF₆⁻ oder SbF₆⁻ steht. insbesondere bevorzugte Metallkomplexe, die besonders für Hydrierungen geeignet sind, entsprechen den Formeln XVI und XVII,

[A₁Me₂YZ] (XVI),

[A₁Me₂Y]⁺E₁⁻ (XVII),

worin
A₁ für eine Verbindung der Formel I steht;
Me₂ Rhodium oder Iridium bedeutet;
Y für zwei Olefine oder ein Dien steht;
Z Cl, Br oder I bedeutet; und
E₁- das Anion einer Sauerstoffsäure oder Komplexsäure darstellt.

Für die Verbindungen der Formel I gelten die zuvor beschriebenen Ausführungsformen und Bevorzugungen.

Bei Y in der Bedeutung als Olefin kann es sich um C₂-C₁₂-, bevorzugt C₂-C₆- und besonders bevorzugt C₂-C₄-Olefine handeln. Beispiele sind Propen, But-1-en und besonders Ethylen. Das Dien kann 5 bis 12 und bevorzugt 5 bis 8 C-Atome enthalten und es kann sich um offenkettige, cyclische oder polycyclische Diene handeln. Die beiden Olefingruppen des Diens sind bevorzugt durch ein oder zwei CH₂-Gruppen verbunden. Beispiele sind 1,3-Pentadien, Cyclopentadien, 1,5-Hexadien, 1,4-Cyclohexadien, 1,4- oder 1,5-Heptadien, 1,4- oder 1,5-Cycloheptadien, 1,4- oder 1,5-Octadien, 1,4- oder 1,5-Cyclooctadien und Norbomadien. Bevorzugt stellt Y zwei Ethylen oder 1,5- Hexadien, 1,5-Cyclooctadien oder Norbornadien dar.

In Formel XVI steht Z bevorzugt für Cl oder Br. Beispiele für E₁ sind BF₄⁻, ClO₄⁻, CF₃SO₃⁻, CH₃SO₃⁻, HSO₄⁻, B(Phenyl)₄⁻, B[Bis(3,5-trifluormethyl)phenyl]₄⁻, PF₆⁻, SbCl₆⁻, AsF₆⁻ oder SbF₆⁻.

Die erfindungsgemässen Metallkomplexe werden nach in der Literatur bekannten Methoden hergestellt (siehe auch US-A-5,371,256, US-A-5,446,844, US-A-5,583,241, und E. Jacobsen, A. Pfaltz, H. Yamamoto (Eds.), Comprehensive Asymmetric Catalysis I bis III, Springer Verlag, Berlin, 1999, und darin zitierte Literatur).

Die erfindungsgemässen Metallkomplexe stellen homogene Katalysatoren oder unter den Reaktionsbedingungen aktivierbare Katalysatorvorläufer dar, die für asymmetrische Additionsreaktionen an prochirale, ungesättigte, organische Verbindungen eingesetzt werden können.

Die Metallkomplexe können zum Beispiel zur asymmetrischen Hydrierung (Addition von Wasserstoff) von prochiralen Verbindungen mit Kohlenstoff/Kohlenstoff oder Kohlenstoff/Heteroatommehrfach-, insbesondere -doppelbindungen verwendet werden. Derartige Hydrierungen mit löslichen homogenen Metallkomplexen sind zum Beispiel in Pure and Appl. Chem., Vol. 68, No. 1, pp. 131-138 (1996) beschrieben. Bevorzugte zu hydrierende ungesättigte Verbindungen enthalten die Gruppen C=C, C=N und/oder C=O. Für die Hydrierung werden efindungsgemäss bevorzugt Metallkomplexe von Rhodium und Iridium verwendet.

Die erfindungsgemässen Metallkomplexe können auch als Katalysatoren zur asymmetrischen Hydroborierung (Addition von Borhydriden) von prochiralen organischen Verbindungen mit Kohlenstoff/Kohlen-stoffdoppelbindungen eingesetzt werden. Derartige Hydroborierungen sind zum Beispiel von Tamio Hayashi in E. Jacobsen, A. Pfaltz, H. Yamamoto (Eds.), Comprehensive Asymmetric Catalysis I bis III, Springer Verlag, Berlin, 1999, Seiten 351 bis 364 beschrieben. Geeignete Borhydride sind zum Beispiel Katecholborane. Die chiralen Borverbindungen können in Synthesen eingesetzt und/oder in an sich bekannter Weise zu anderen chiralen organischen Verbindungen umgesetzt werden, die wertvolle Bausteine für die Herstellung chiraler Zwischenprodukte oder Aktivsubstanzen darstellen. Ein Beispiel für eine solche Umsetzung ist die Herstellung von 3-Hydroxy-tetrahydrofuran (gemäss DE 198 07 330).

Die erfindungsgemässen Metallkomplexe können auch als Katalysatoren zur asymmetrischen Hydrosilylierung (Addition von Silanen) von prochiralen organischen Verbindungen mit Kohlenstoff/Kohlenstoff- oder Kohlenstoffheteroatomdoppelbindungen eingesetzt werden. Derartige Hydrosilylierungen sind zum Beispiel von G. Pioda und A. Togni in Tetrahedron: Asymmetry, 1998, 9, 3093 oder von S. Uemura, et al. in Chem. Commun. 1996, 847 beschrieben. Geeignete Silane sind zum Beispiel Trichlorsilan oder Diphenylsilan. Zur Hydrosilylierung von zum Beispiel C=O- und C=N-Gruppen verwendet man bevorzugt Metallkomplexe von Rhodium und Iridium. Zur Hydrosilylierung von zum Beispiel C=C-Gruppen verwendet man bevorzugt Metallkomplexe von Palladium. Die chiralen Silylverbindungen können in Synthesen eingesetzt und/oder in an sich bekannter Weise zu anderen chiralen organischen Verbindungen umgesetzt werden, die wertvolle Bausteine für die Herstellung chiraler Zwischenprodukte oder Aktivsubstanzen darstellen. Beispiele für solche Umsetzungen sind die Hydrolyse zu Alkoholen.

Die erfindungsgemässen Metallkomplexe können auch als Katalysatoren für asymmetrische allylische Substitutionsreaktionen (Addition von C-Nukleophilen an Allylverbindungen) eingesetzt werden. Derartige Allylierungen sind zum Beispiel von A. Pfaltz und M. Lautens in E. Jacobsen, A. Pfaltz, H. Yamamoto (Eds.), Comprehensive Asymmetric Catalysis I bis III, Springer Verlag, Berlin, 1999, Seiten 833 bis 884 beschrieben. Geeignete Vorläufer für Allylverbindungen sind zum Beispiel 1,3-Diphenyl-3-acetoxy-1-propen oder 3-Acetoxy-1-cyclohexen. Für diese Reaktion verwendet man bevorzugt Metallkomplexe von Palladium. Die chiralen Allylverbindungen können in Synthesen zur Herstellung von chiralen Zwischenprodukten oder Aktivsubstanzen eingesetzt werden.

Die erfindungsgemässen Metallkomplexe können auch als Katalysatoren zur asymmetrischen Aminierung (Addition von Aminen an Allylverbindungen oder in asymmetrischen, Heck-Reaktionen eingesetzt werden. Derartige Aminierungen sind zum Beispiel von A. Pfaltz und M. Lautens in E. Jacobsen, A. Pfaltz, H. Yamamoto (Eds.), Comprehensive Asymmetric Catalysis I bis III, Springer Verlag, Berlin, 1999, Seiten 833 bis 884, Heck-Reaktionen von O. Loiseleur et al. im Journal of Organometallic Chemistry 576 (1999), Seiten 16 bis 22, beschrieben. Geeignete Amine sind neben Ammoniak primäre und sekundäre Amine. Zur Aminierung der Allylverbindungen verwendet man bevorzugt Metallkomplexe von Palladium. Die chiralen Amine können in Synthesen zur Herstellung von chiralen Zwischenprodukten oder Aktivsubstanzen eingesetzt werden.

Ein weiterer Gegenstand der Erfindung ist die Verwendung der erfindungsgemässen Metallkomplexe als homogene Katalysatoren zur Herstellung chiraler organischer Verbindungen durch asymmetrische Anlagerung von Wasserstoff, Borhydriden oder Silanen an eine Kohlenstoff- oder Kohlenstoff-Heteroatommehrfachbindung in prochiralen organischen Verbindungen, oder die asymmetrische Addition von C-Nukleophilen oder Aminen an Allylverbindungen.

Ein weiterer Aspekt der Erfindung ist ein Verfahren zur Herstellung chiraler organischer Verbindungen durch asymmetrische Anlagerung von Wasserstoff, Borhydriden oder Silanen an eine Kohlenstoff- oder Kohlenstoff-Heteroatommehrfachbindung in prochiralen organischen Verbindungen, oder die asymmetrische Addition von C-Nukleophilen oder Amine an Allylverbindungen in Gegenwart eines Katalysators, das dadurch gekennzeichnet ist, dass man die Anlagerung in Gegenwart katalytischer Mengen wenigstens eines erfindungsgemässen Metallkomplexes durchführt.

Bevorzugte zu hydrierende prochirale, ungesättigte Verbindungen können ein oder mehrere, gleiche oder verschiedene Gruppen C=C, C=N und/oder C=O, in offenkettigen oder cyclischen organischen Verbindungen enthalten, wobei die Gruppen C=C, C=N und/oder C=O Teil eines Ringsystems sein können oder exocyclische Gruppen darstellen. Bei den prochiralen ungesättigten Verbindungen kann es sich um Alkene, Cycloalkene, Heterocycloalkene, sowie um offenkettige oder cyclische Ketone, Ketimine und Kethydrazone handeln. Sie können zum Beispiel der Formel XVIII entsprechen,

R₀₇R₀₈C=D (XVIII),

worin
R₀₇ und R₀₈ so ausgewählt sind, dass die Verbindung prochiral ist, und unabhängig voneinander einen offenkettigen oder cyclischen Kohlenwasserstoffrest oder Heterokohlenwasserstoffrest mit Heteroatomen, ausgewählt aus der Gruppe O, S und N darstellen, die 1 bis 30 und bevorzugt 1 bis 20 C-Atome enthalten;
D für O oder einen Rest der Formeln C=R₀₉R₀₁₀ oder NR₀₁₁ steht;
R₀₉ und R₀₁₀ unabhängig voneinander die gleiche Bedeutung wie R₀₇ und R₀₈ haben,
R₀₁₁ Wasserstoff, C₁-C₁₂-Alkyl, C₁-C₁₂-Alkoxy, C₃-C₁₂Cycloalkyl, C₃-C₁₂-Cycloalkyl-C₁-C₆-Alkyl, C₃-C₁₁-Heterocycloalkyl, C₃-C₁₁-Heterocycloalkyl-C₁-C₆-Alkyl, C₆-C₁₄-Aryl, C₅-C₁₃-Hetero-aryl, C₇-C₁₆-Aralkyl oder C₆-C₁₄-Heteroaralkyl bedeutet,
R₀₇ und R₀₈ zusammen mit dem C-Atom, an das sie gebunden sind, einen Kohlenwasserstoffring oder Heterokohlenwasserstoffring mit 3 bis 12 Ringgliedern bilden;
R₀₇ und R₀₈ je zusammen mit der C=C-Gruppe, an die sie gebunden sind, einen Kohlenwasserstoffring oder Heterokohlenwasserstoffring mit 3 bis 12 Ringgliedern bilden;
R₀₇ und R₀₁₁ je zusammen mit der C=N-Gruppe, an die sie gebunden sind, einen Kohlenwasserstoffring oder Heterokohlenwasserstoffring mit 3 bis 12 Ringgliedern bilden;
die Heteroatome in den heterocyclischen Ringen ausgewählt sind aus der Gruppe O, S und N; und R₀₇, P₀₈, P₀₉, R₀₁₀ und R₀₁₁ unsubstituiert oder mit C₁-C₆-Alkyl, C₁-C₆-Alkoxy, Cyclohexyl, C₆-C₁₀-Aryl, C₇-C₁₂-Aralkyl, C₁-C₄-Alkyl-C₆-C₁₀-Aryl, C₁-C₄-Alkoxy-C₆-C₁₀-Aryl, C₁-C₄-Alkyl-C₇-C₁₂-Aralkyl, C₁-C₄-Alkoxy-C₇-C₁₂-Aralkyl, -OH, =O, -CO-OR₀₁₂, -CO-NR₀₁₃R₀₁₄ oder -NR₀₁₃R₀₁₄ substituiert sind, worin R₀₁₂ für H, ein Alkalimetall, C₁-C₆-Alkyl, Cyclohexyl, Phenyl oder Benzyl steht, und R₀₁₃ und R₀₁₄ unabhängig voneinander Wasserstoff, C₁-C₆-Alkyl, Cyclohexyl, Phenyl oder Benzyl darstellen, oder R₀₁₃ und R₀₁₄ zusammen Tetramethylen, Pentamethylen oder 3-Oxapentylen bedeuten.

Beispiele und Bevorzugungen für Substituenten sind zuvor genannt worden.

Bei R₀₇ und R₀₈ kann es sich zum Beispiel um C₁-C₂₀-Alkyl und bevorzugt C₁-C₁₂-Alkyl, C₁-C₂₀-Heteroalkyl und bevorzugt C₁-C₁₂-Heteroalkyl mit Heteroatomen ausgewählt aus der Gruppe O, S und N, C₃-C₁₂-Cycloalkyl und bevorzugt C₄-C₈-Cycloalkyl, C-gebundenes C₃-C₁₁-Hetrocycloalkyl und bevorzugt C₄-C₈-Heterocycloalkyl mit Heteroatomen ausgewählt aus der Gruppe O, S und N, C₃-C₁₂-Cycloalkyl-C₁-C₆-Alkyl und bevorzugt C₄-C₈-Cycloalkyl-C₁-C₆-Alkyl, C₃-C₁₁-Hetrocycloalkyl-C₁-C₆-Alkyl und bevorzugt C₄-C₈-Heterocycloalkyl-C₁-C₆-Alkyl mit Heteroatomen ausgewählt aus der Gruppe O, S und N, C₆-C₁₄-Aryl und bevorzugt C₆-C₁₀-Aryl, C₅-C₁₃-Heteroaryl und bevorzugt C₅-C₉-Heteroaryl mit Heteroatomen ausgewählt aus der Gruppe O, S und N, C₇-C₁₅-Aralkyl und bevorzugt C₇-C₁₁-Aralkyl, C₆-C₁₂-Heteroaralkyl und bevorzugt C₆-C₁₀-Heteroaralkyl mit Heteroatomen ausgewählt aus der Gruppe O, S und N.

Wenn R₀₇ und R₀₈, R₀₇ und R₀₉, oder R₀₇ und R₀₁₁ je zusammen mit der Gruppe, an die sie gebunden sind, einen Kohlenwasserstoffring oder Heterokohlenwasserstoffring bilden, so enthält der Ring bevorzugt 4 bis 8 Ringglieder. Der Heterokohlenwasserstoffring kann zum Beispiel 1 bis 3, und vorzugsweise ein oder zwei Heteroatome enthalten.

R₀₁₁ bedeutet bevorzugt Wasserstoff, C₁-C₆-Alkyl, C₁-C₆-Alkoxy, C₄-C₈-Cycloalkyl, C₄-C₈-Cycloalkyl-C₁-C₄-Alkyl, C₄-C₁₀-Heterocycloalkyl, C₄-C₁₀-Heterocycloalkyl-C₁-C₄-Alkyl, C₆-C₁₀-Aryl, C₅-C₉-Heteroaryl, C₇-C₁₂-Aralkyl und C₅-C₁₃-Heteroaralkyl.

Einige Beispiele für ungesättigte organische Verbindungen sind Acetophenon, 4-Methoxyacetophenon, 4-Tri-fluormethylacetophenon, 4-Nitroacetophenon, 2-Chloracetophenon, entsprechende gegebenenfalls N-substituierte Acetophenonbenzylimine, unsubstituiertes oder substituiertes Benzocyclohexanon oder Benzocyclopentanon und entsprechende Imine, Imine aus der Gruppe unsubstituiertes oder substituiertes Tetrahydrochinolin, Tetrahyropyridin und Dihydropyrrol, und ungesättigte Carbonsäuren, -ester, -amide und -salze wie zum Beispiel α- und gegebenenfalls β-substituierte Acrylsäuren oder Crotonsäuren. Bevorzugte Carbonsäuren sind solche der Formel

R₀₁₂-CH=C(R₀₁₃)-C(O)OH

sowie ihre Salze, Ester und Amide, worin R₀₁₂ C₁-C₆-Alkyl, unsubstituiertes oder mit 1 bis 4 C₁-C₆-Alkyl, C₁-C₆-Alkoxy, C₁-C₆-Alkoxy-C₁-C₄-alkoxy substituiertes C₃-C₈-Cycloalkyl, oder unsubstituiertes oder mit 1 bis 4 C₁-C₆-Alkyl, C₁-C₆-Alkoxy, C₁-C₆-Alkoxy-C₁-C₄-alkoxy substituiertes C₆-C₁₀-Aryl und bevorzugt Phenyl darstellt, und R₀₁₃ lineares oder verzweigtes C₁-C₆-Alkyl (zum Beispiel Isopropyl), unsubstituiertes oder wie zuvor definiert substituiertes Cyclopentyl, Cyclohexyl, Phenyl oder geschütztes Amino (zum Beispiel Acetylamino) bedeutet.

Das erfindungsgemässe Verfahren kann bei tiefen oder erhöhten Temperaturen, zum Beispiel Temperaturen von -20 bis 150 °C, bevorzugt von -10 bis 100 °C, und besonders bevorzugt von 10 bis 80 °C durchgeführt werden. Die optischen Ausbeuten sind im allgemeinen bei tieferer Temperatur besser als bei höheren Temperaturen.

Das erfindungsgemässe Verfahren kann bei Normaldruck oder Überdruck durchgeführt werden. Der Druck kann zum Beispiel von 10⁵ bis 2x10⁷ Pa (Pascal) betragen. Hydrierungen können bei Normaldruck oder bei Überdruck durchgeführt werden. Bei Normaldruck werden häufig bessere Selektivitäten beobachtet.

Katalysatoren werden bevorzugt in Mengen von 0,0001 bis 10 Mol-%, besonders bevorzugt 0,001 bis 10 Mol-%, und insbesondere bevorzugt 0,01 bis 5 Mol-% verwendet, bezogen auf die zu hydrierende Verbindung.

Die Herstellung der Liganden und Katalysatoren sowie die Anlagerung können ohne oder in Gegenwart eines inerten Lösungsmittels durchgeführt werden, wobei ein Lösungsmittel oder Gemische von Lösungsmitteln eingesetzt werden können. Geeignete Lösungsmittel sind zum Beispiel aliphatische, cycloaliphatiche und aromatische Kohlenwasserstoffe (Pentan, Hexan, Petrolether, Cyclohexan, Methylcyclohexan, Benzol, Toluol, Xylol), aliphatische Halogenkohlenwasserstoffe (Methylenchlorid, Chloroform, Di- und Tetrachlorethan), Nitrile (Acetonitril, Propionitril, Benzonitril), Ether (Diethylether, Dibutylether, t-Butylmethylether, Ethylenglykoldimethylether, Ethylenglykoldiethylether, Diethylenglykoldimethylether, Tetrahydrofuran, Dioxan, Diethylenglykolmonomethyl- oder monoethyether), Ketone (Aceton, Methylisobutylketon), Carbonsäureester und Lactone (Essigsäureethyl- oder -methylester, Valerolacton), N-substituierte Lactame (N-Methylpyrrolidon), Carbonsäureamide (Dimethylamid, Dimethylformamid), acyclische Harnstoffe (Dimethylimidazolin), und Sulfoxide und Sulfone (Dimethylsulfoxid, Dimethylsulfon, Tetramethylensulfoxid, Tetramethylensulfon) und Alkohole (Methanol, Ethanol, Propanol, Butanol, Ethylenglykolmonomethylether, Ethylenglykolmonoethylether, Diethylenglykolmonomethylether) und Wasser. Die Lösungsmittel können alleine oder in Mischung von wenigstens zwei Lösungsmitteln verwendet werden.

Die Reaktion kann in Gegenwart von Cokatalysatoren durchgeführt werden, zum Beispiel quaternären Ammoniumhalogeniden (Tetrabutylammoniumiodid) und/oder in Gegenwart von Protonensäuren, zum Beispiel Mineralsäuren, durchgeführt werden (siehe zum Beispiel US-A-5,371,256, US-A-5,446,844 und US-A-5,583,241 und EP-A-0 691 949). Die Cokatalysatoren sind besonders für Hydrierungen geeignet.

Die als Katalysatoren verwendeten Metallkomplexe können als getrennt hergestellte isolierte Verbindungen zugegeben werden, oder auch in situ vor der Reaktion gebildet und dann mit dem zu hydrierenden Substrat vermischt werden. Es kann vorteilhaft sein, bei der Reaktion unter Verwendung von isolierten Metallkomplexen zusätzlich Liganden zuzugeben, oder bei der in situ Herstellung einen Überschuss der Liganden einzusetzen. Der Überschuss kann zum Beispiel 1 bis 10 und vorzugsweise 1 bis 5 Mol betragen, bezogen auf die zur Herstellung verwendete Metallverbindung.

Das erfindungsgemässe Verfahren wird im allgemeinen so durchgeführt, dass man den Katalysator vorlegt und dann das Substrat, gegebenenfalls Reaktionshilfsmittel und die anzulagernde Verbindung zugibt, und danach die Reaktion startet. Gasförmige anzulagernde Verbindungen, wie zum Beispiel Wasserstoff oder Ammoniak, werden vorzugsweise aufpresst. Das Verfahren kann in verschiedenen Reaktortypen kontinuierlich oder satzweise durchgeführt werden.

Die erfindungsgemäss herstellbaren chiralen organischen Verbindungen sind aktive Substanzen oder Zwischenprodukte zur Herstellung solcher Substanzen, insbesondere im Bereich der Herstellung von Pharmazeutika und Agrochemikalien.

Die nachfolgenden Beispiele erläutern die Erfindung.

### A) Herstellung von Halogenphosphinen

Alle Reaktionen werden in getrockneten Lösungsmitteln und unter Inertgas durchgeführt.

### Beispiel A1 : Herstellung von

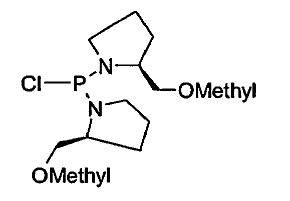

In einem 500 mL-Rundkolben mit Argoneinlass wird PCl₃ (7,38 g, 53,75 mmol) in trockenem Tetrahydrofuran (THF, 150 ml) unter Argon gelöst und die Lösung in einem Eisbad auf 0 °C gekühlt. Es wird tropfenweise Triethylamin (11,97 g, 118,25 mmol, 2,20 Äquivalente) zugegeben und im Anschluss langsam (S)-Methoxymethylpyrrolidin (12,69 g, 110,19 mmol, 2,05 Äquivalente) zugetropft. Während der Zugabe wird die Bildung eines weissen Niederschlags beobachtet. Das Eisbad wird entfernt und die erhaltene Suspension über Nacht (14 h) bei Raumtemperatur (RT) gerührt. Der gebildete weisse Niederschlag wird unter Argon mittels einer Umkehrfritte abfiltriert und mit trockenem THF (2 mal 25 ml) gewaschen. Vom erhaltenen gelblichen Filtrat wird ein ³¹P-NMR (C₆D₆) aufgenommen. Die so gewonnene Lösung wird ohne weitere Reinigung umgesetzt. ³¹P-NMR (C₆D₆, 121 MHz): 154.3 (s).

### Beispiel A2: Herstellung von

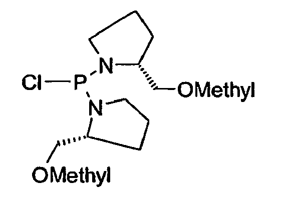

Es wird gemäss Beispiel A1 verfahren, aber (R)-Methoxymethylpyrrolidin verwendet.

### B) Herstellung von aromatischen Monophosphinen

### Beispiel B1: Herstellung von

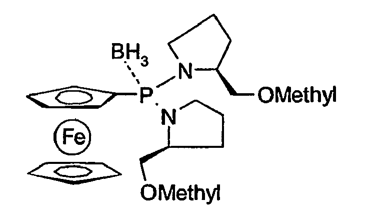

In einem 1 I-Rundkolben mit Argoneinlass werden Ferrocen (10,00 g, 53,75 mmol) und Kaliumtertiärbutylat (754 mg, 6,72 mmol, 0,125 Äquivalente) unter Argon in trockenem THF (100 ml) gelöst. Die Lösung wird auf -78 °C gekühlt und *t*-Butyllithium (1,5 M in Hexan; 71,67 ml, 107,50 mmol, 2,00 Äquivalente) wird innerhalb von 45 Minuten zugetropft. Die Lösung wird 1,5 h bei -78 °C gerührt und mit n-Heptan (75 ml) versetzt. Nach dem Absitzen des erhaltenen Niederschlages wird die überstehende Lösung bei -78 °C unter Argon mit einer Umdrücknadel entfernt. Der Niederschlag wird mit n-Heptan (60 ml) bei -78 °C gewaschen und die Waschlösung erneut mit einer Umdrücknadel entfernt. Dieser Vorgang wird dreimal wiederholt. Der erhaltene Niederschlag wird in trockenem THF (50 ml) gelöst und eine Lösung von A1 (53,75 mmol, 1,00 Äquivalente) in THF (200 ml) bei -78 °C innerhalb von 1,5 h zugetropft. Die Lösung wird über Nacht (14 h) unter Erwärmung auf RT gerührt. Im Anschluss wird tropfenweise Boran-Dimethylsulfid-Komplex (5,10 ml, 53,75 mmol, 1,00 Äquivalente) zugegeben und über Nacht bei RT gerührt. Das Reaktionsgemisch wird mit gesättigter NH₄Cl-Lösung (50 ml) hydrolysiert und mit Tertiärbutylmethylether (TBME) (3 x 100 ml) extrahiert. Die vereinigten organischen Phasen werden über Na₂SO₄ getrocknet und das Lösungsmittel am Rotationsverdampfer abdestilliert. Das Rohprodukt (24,18 g) wird säulenchromatographisch gereinigt (200 g Kieselgel, n-Heptan/TBME 5:1). Die Titelverbindung (17,23 g, 70 % der Theorie) wird als orange-farbener Feststoff erhalten. ³¹P-NMR (C₆D₆, 121 MHz): 80,8 (m, breit).

### Beispiel B2: Herstellung von

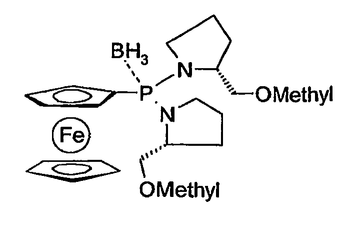

Es wird gemäss Beispiel B1 verfahren, aber die Verbindung A2 anstelle von A1 verwendet.

### Beispiel B3: Herstellung von

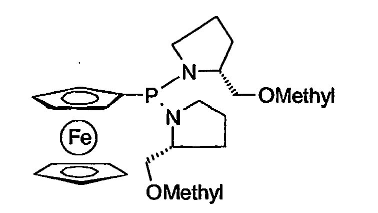

Zu einer Lösung von 4,53 g (17,1 mMol) Bromoferrocen in 15 ml THF werden bei -78 °C 17,9 mmol einer 1,6 molaren n-Butyl-Li-Lösung in Hexan langsam zugetropft und das Gemisch 10 Minuten bei dieser Temperatur gerührt. Dann lässt man die Temperatur auf 0 - 5 °C ansteigen (Eiskühlung), tropft eine Lösung von 18,8 mmol der Verbindung gemäss Beispiel A2 in 78 ml THF zu und rührt das Gemisch über Nacht bei RT. Das Lösungsmittel wird anschliessend abgezogen und das Rohprodukt über eine kurze Säule (Silikagel 60 Fluka, Laufmittel TBME) gereinigt. Nach Destillation der gefärbten Fraktionen am Rotationsverdampfer wird ein oranges, beinahe festes, Öl erhalten. ³¹P-NMR (C₆D₆, 121 MHz): 70,7 (s).

### C) Herstellung von primären Ferrocenphosphinen

### Beispiel C1: Herstellung von Ferrocen-1,2-diphosphin

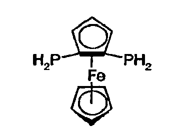

### a) Herstellung von

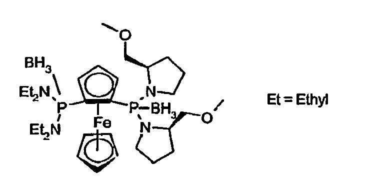

In einem 50 ml Rundkolben mit Argoneinlass wird die Verbindung gemäss Beispiel B2 (1,00 g, 2,18 mmol) in trockenem Tertiärbutylmethylether (TBME) (5,00 ml) und n-Hexan (5,00 ml) gelöst und die erhaltene Lösung auf -30 °C gekühlt. Dabei fällt das Edukt als gelber Feststoff aus. Es wird tropfenweise s-Butyl-Li (1,3 M in Cyclohexan; 1,76 ml, 2,29 mmol, 1,05 Äquivalente) zugegeben. Dabei geht der gelbe Feststoff allmählich in Lösung, die Lösung färbt sich orange-rot und nach etwa 30 Minuten fällt ein orange-farbener Feststoff aus. Nach 2 Stunden Rühren bei -30 °C wird CIP(NEthyl₂)₂ (551 mg, 2,62 mmol, 1,2 Äquivalente) zugegeben, das Kühlbad entfernt und die Suspension 2 h unter Erwärmung auf Raumtemperatur (RT) gerührt. Anschliessend wird BH₃·SMe₂ (0,25 ml, 2,62 mmol, 1,2 Äquivalente) zugetropft und die Suspension über Nacht (14 h) bei RT gerührt. Das Reaktionsgemisch wird mit gesättigter NaCl-Lösung (50 ml) hydrolysiert, TBME (50 ml) zugegeben, die organische Phase abgetrennt und über Na₂SO₄ getrocknet. Das Lösungsmittel wird am Rotationsverdampfer abdestilliert und das Rohprodukt säulenchromatographisch gereinigt (100 g Kieselgel, n-Heptan/TBME 5:1). Die Ferrocenylverbindung (1,10 g, 1,71 mmol, 78 %) wird in Form eines orange-farbenen Feststoffes erhalten. ³¹P-NMR (C₆D₆, 121 MHz): 99,7-99,0 (m, br), 79,9-79,5 (m, br).

### b) Herstellung von

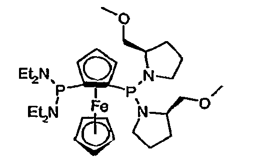

Für das Entfernen der Borangruppe werden 1,00 g (1,87 mmol) der gemäss a) hergestellten Verbindung in 5 ml Diethylamin aufgenommen und über Nacht bei 50°C gerührt. Im Anschluss werden alle flüchtigen Bestandteile im Ölpumpenvakuum bei 50 °C entfernt. Der erhaltene Rückstand wird dreimal in Diethylamin (je 2,00 ml) aufgenommen, für 30 Minuten bei 50 °C gerührt und alle flüchtigen Bestandteile bei 50 °C im Ölpumpenvakuum entfernt (30 Minuten). Der Rückstand wird zweimal in trockenem TBME (2 ml) aufgenommen und alle flüchtigen Bestandteile bei 50 °C im Ölpumpenvakuum entfernt. Zurück bleibt das entschützte Produkt, welches ohne Reinigung in Stufe c) weiterverwendet wird. ³¹P-NMR (C₆D₆, 121 MHz): -151 ppm.

### c) Herstellung von

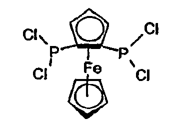

Das gemäss b) hergestellte Reaktionsprodukt wird in 5 ml TBME gelöst und bei 0°C tropfenweise unter Rühren mit 8 mmol einer HCl-Lösung (2n in Diethylether) versetzt. Die Reaktionslösung wird anschliessend durch Dekantieren von den ausgefallenen Ammoniumverbindungen abgetrennt und unter Argon am Rotationsverdampfer flüchtige Bestandteile entfernt. Der Rückstand wird direkt in Stufe d) eingesetzt. ³¹P-NMR (C₆D₆, 121 MHz): 156.5 (s)

### d) Herstellung der Titelverbindung

0,43 g (11,20 mmol) Lithiumaluminiumhydrid werden unter Argon in 10 ml absoslutem Tetrahydrofuran (THF) suspendiert und auf -78°C gekühlt. Das gemäss c) erhaltene Rohprodukt wird in 5 ml absolutem THF gelöst und zur gekühlten Suspension des Lithiumaluminiumhydrids getropft. Das Reaktionsgemisch wird 30 Minuten bei dieser Temperatur und dann 30 Minuten bei 20°C gerührt. Zur Suspension werden 3,8 ml 2N NaOH tropfenweise zugegeben und die überstehende Lösung zur Isolierung der Titelverbindung abfiltriert und eingedampft. ³¹P-NMR (C₆D₆, 121 MHz): -151 ppm.

### Beispiel C2: Herstellung von

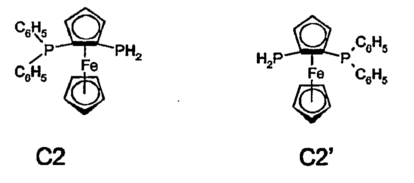

### a) Herstellung von

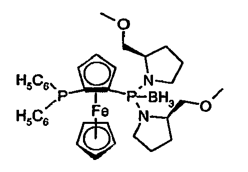

Zu einer Lösung von 4,05 g (8,80 mmol) der Verbindung gemäss Beispiel B2 in 60 ml Hexan / TBME 1:1 werden bei -40 °C 9,5 mmol sekundär-Butyllithium (1,3 molar in Hexan) zugetropft. Das Reaktionsgemisch wird während 2 Stunden bei dieser Temperatur weitergerührt. Zur resultierenden orangen Suspension werden anschließend 9,43 mmol Chlordiphenylphosphin gegeben. Dann läßt man das Reaktionsgemisch langsam unter Rühren auf Raumtemperatur erwärmen. Nach 2h Rühren über Nacht wird mit Wasser / Methylenchlorid extrahiert, die organischen Phasen mit Natriumsulfat getrocknet und das Lösungsmittel am Rotationsverdampfer unter Vakuum abdestilliert. Reinigung durch Säulenchromatographie (Kieselgel 60, Laufmittel Hexan / TBME 6:1) liefert das gewünschte Produkt als oranges kristallines Material in einer Ausbeute von 85%. ³¹P-NMR (C₆D₆, 121 MHz): -25,2 (s), +79 (s, breit). 1H-NMR (C₆D₆, 300 MHz), charakteristische Signale: 7,80 (m), 7,37 (m), 6,97-7,16 (m), 4,08 (s, 5H, Cyclopentadienring), 3,28 (s, 3H, O-CH₃), 3,10 (s, 3H, O-CH₃).

### b) Herstellung von

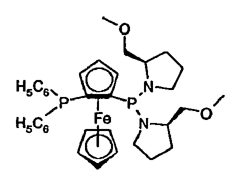

Für das Entfernen der Borangruppe werden 400m g (0,62 mmol) der gemäss a) hergestellten Verbindung in 5 ml Diethylamin aufgenommen und über Nacht bei 50 °C gerührt. Im Anschluss werden alle flüchtigen Bestandteile im Ölpumpenvakuum bei 50 °C entfernt. Der erhaltene Rückstand wird dreimal in Diethylamin (je 2,00 ml) aufgenommen, für 30 Minuten bei 50 °C gerührt und alle flüchtigen Bestandteile bei 50 °C im Ölpumpenvakuum entfernt (30 Minuten). Der Rückstand wird zweimal in trockenem TBME (2 ml) aufgenommen und alle flüchtigen Bestandteile bei 50 °C im Ölpumpenvakuum entfernt. Zurück bleibt das BH₃-freie Produkt, welches ohne Reinigung in Stufe c) weiterverwendet wird. ³¹P-NMR (C₆D₆, 121 MHz): -23,5 (d, J_{PP} ∼73Hz), +69,4 (d, J_{PP} ∼73Hz).

### c) Herstellung von

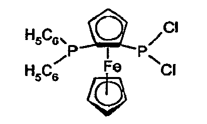

Das gemäss b) hergestellte Reaktionsprodukt wird in 5 ml TBME gelöst und bei 0 °C tropfenweise unter Rühren mit 2,6 mmol einer HCl-Lösung (2n in Diethylether) versetzt. Die Reaktionslösung wird anschliessend durch Dekantieren von den ausgefallenen Ammoniumverbindungen abgetrennt und unter Argon am Rotationsverdampfer flüchtige Bestandteile entfernt. Der Rückstand wird direkt in Stufe d) eingesetzt. ³¹P-NMR (C₆D₆, 121 MHz): -23,6 (d, **J**_{PP} ∼ 170Hz), +161,6 (d, **J**_{PP} ∼ 170Hz).

### d1) Herstellung der Titelverbindung C2

2 mmol Lithiumaluminiumhydrid werden unter Argon in 3 ml absolutem THF suspendiert und auf -78 °C gekühlt. Das gemäß c) hergestellte Rohprodukt wird in 3 ml absolutem THF gelöst und zur gekühlten Suspension von Lithiumaluminiumhydrid getropft. Das Reaktionsgemisch wird 30 Minuten bei dieser Temperatur und dann 30 Minuten bei 20°C gerührt. Zur Suspension werden 0,8 ml 2N NaOH tropfenweise zugegeben und die überstehende Lösung zur Isolierung der Titelverbindung abfiltriert und eingedampft. ³¹P-NMR (C₆D₆, 121 MHz): -20 (m,), -152 (m).

### d2) Herstellung der Titelverbindung C2'

Es wird gemäss Beispiel C2 verfahren, aber im Schritt a) die Verbindung B1 anstelle von B2 verwendet.

### Beispiel C3: Herstellung von

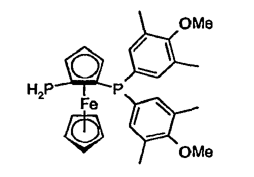

### a) Herstellung von

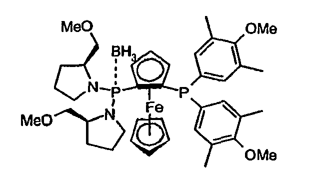

Zu einer Lösung von 4 g (8,70 mmol) der Verbindung gemäß Beispiel B1 in 60 ml Hexan / TBME 1:1 werden bei -40 °C 9.43 mmol sekundär-Butyllithium (1,3 molar in Hexan) zugetropft. Das Reaktionsgemisch wird während 2 Stunden bei dieser Temperatur weitergerührt. Zur resultierenden orangen Suspension werden anschließend 9,43 mmol Bis(3,5-Dimethyl-4-methoxy-phenyl)phosphinchlorid gegeben. Dann läßt man das Reaktionsgemisch langsam unter Rühren auf Raumtemperatur erwärmen. Nach 2 Stunden Rühren wird mit Wasser / TBME extrahiert, die organischen Phasen mit Natriumsulfat getrocknet und das Lösungsmittel am Rotationsverdampfer unter Vakuum abdestilliert. Reinigung durch Säulenchromatographie liefert das gewünschte Produkt als gelbes kristallines Material in einer Ausbeute von 74%. ³¹P-NMR (C₆D₆, 121 MHz): -26,7 (s), +79 (s, breit). 1H-NMR (C₆D₆, 300 MHz), einige charakteristische Signale: 7,71 (s, 1 H), 7,68 (s, 1 H), 7,25 (s, 1 H), 7,23 (s, 1 H), 4,17 (s, 5H, Cyclopentadienring), 3,31 (s, 3H, O-CH₃), 3,30 (s, 3H, O-CH₃), 3,27 (s, 3H, O-CH₃), 3,11 (s, 3H, O-CH₃), 2,14 (s, 3H, CH₃), 2,11 (s, 3H, CH₃).

### b) Herstellung von

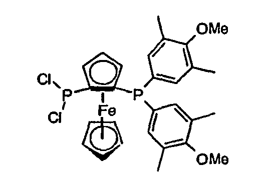

Zu einer Lösung von 2,34 g (3,08 mmol) der Verbindung a) in 20 ml THF werden bei 0 °C langsam 18,5 mmol HCl (Lösung in Diethyläther) zugetropft. Danach wird die Kühlung entfernt und weitere 2 Stunden gerührt. Eine Probe ergibt im ³¹P-NMR (C₆D₆, 121 MHz) folgende Signale: 165 (PCl₂), 16 (breit, P(3,5-Dimethyl-4-methoxyphenyl)₂- Boranaddukt. Lässt man weniger lang reagieren, ist auch etwas freies P(3,5-Dimethyl-4-methoxyphenyl)₂ im Bereich -28 ppm sichtbar.

### c) Herstellung der Titelverbindung C3:

Das gemäss Stufe b) erhaltene Dichlorphosphin wird ohne Reinigung weiter umgesetzt. Nach Abkühlen auf 0 °C werden portionenweise langsam mit 30 mmol Lithiumaluminiumhydrid zugegeben. Nach 2 Stunden Rühren bei Raumtemperatur wird wieder auf 0 °C gekühlt, und es werden langsam 10 ml Wasser zugetropft. Zur resultierenden grauen Suspension wird Natriumsulfat zugegeben. Es scheidet sich eine organische Phase ab. Diese wird abgetrennt und das Gemisch mehrmals mit Heptan gewaschen. Die organischen Phasen werden vereinigt und mit Natriumsulfat getrocknet. Nach Abdestillieren des Lösungsmittels unter Vakuum wird das Rohprodukt in TBME gelöst und zur Entfernung von Boran in Gegenwart von Diethanolamin während mehreren Stunden bei 50 °C gerührt. Das TBME wird anschliessend mit Wasser, 1 n HCl und nochmals mit Wasser gewaschen, über Natriumsulfat getrocknet und schliesslich am Vakuum abdestilliert. Das gewünschte Produkt wird in guter Ausbeute als rotes Oel erhalten und ohne Reinigung weiterverwendet. ³¹P-NMR (C₆D₆, 121 MHz): -152 (d, PH₂), -22 (d, P(3,5-Dimethyl-4-methoxyphenyl)₂). 1 H-NMR (C₆D₆, 300 MHz), einige charakteristische Signale: 7,52 (s, 1 H), 7,49 (s, 1 H), 7,19 (s, 1 H), 7,16 (s, 1 H), 4,04 (s, 5H, Cyclopentadienring), 3,33 (s, 3H, O-CH₃), 3,27 (s, 3H, O-CH₃), 3,15 (s, 3H, O-CH₃), 2,88 (s, 3H, O-CH₃), 2,14 (s, 3H, CH₃), 2,08 (s, 3H, CH₃).

### Beispiel C4: Herstellung von

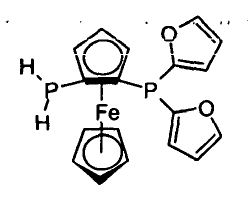

### a) Herstellung von

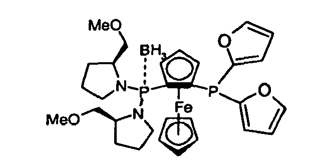

Zu einer Lösung von 4 g (8,70 mmol) der Verbindung gemäss Beispiel B1 in 60 ml Hexan / TBME 1:1 werden bei -40 °C 9,43 mmol sekundär-Butyllithium (1,3 molar in Hexan) zugetropft. Das Reaktionsgemisch wird während 2 Stunden bei dieser Temperatur weitergerührt. Zur resultierenden orangen Suspension werden anschließend 9,43 mmol Difuryl-phosphinchlorid gegeben. Dann läßt man das Reaktionsgemisch langsam unter Rühren auf Raumtemperatur erwärmen. Nach 2 Stunden Rühren wird mit Wasser / Dichlormethan extrahiert, die organischen Phasen mit Natriumsulfat getrocknet und das Lösungsmittel am Rotationsverdampfer unter Vakuum abdestilliert. Das feste Rohprodukt wird durch Umkristallisation in Methanol gereinigt. Das Produkt wird als gelbes kristallines Material in einer Ausbeute von 72% erhalten. ³¹P-NMR (C₆D₆, 121 MHz): -71,6 (s), +76.5 (s, breit). 1H-NMR (C₆D₆, 300 MHz), einige charakteristische Signale: 7,20 (m, 1H), 6,81 (m, 1H), 6,52 (m, 1H), 5,97 (m, 2H), 5,21 (m, 1 H), 4,27 (s, 5H, Cyclopentadienring), 3,28 (s, 3H, O-CH₃), 3,13 (s, 3H, O-CH₃).

### b) Herstellung von

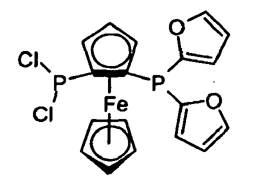

Zu einer Lösung von 2,0 g (3,22 mmol) der Verbindung a) in 20 ml THF werden bei 0 °C innerhalb von 30 Minuten 18,5 mmol HCl (Lösung in Diethyläther) zugetropft. Danach wird die Kühlung entfernt und weitere 2 Stunden gerührt. In der roten Lösung bildet sich ein heller Niederschlag und ein rotes Oel. Nach Abfiltrieren des Niederschlags wird das Reaktionsgemisch mittels NMR gemessen. ³¹P-NMR (C₆D₆, 121 MHz): 162,7 (d), -72,5 (d).

### c) Herstellung der Titelverbindung

Das gemäss Stufe b) erhaltene Dichlorphosphin wird ohne Reinigung weiter umgesetzt. Nach Abkühlen auf 0 °C werden portionsweise langsam mit 32 mmol Lithiumaluminiumhydrid zugegeben. Nach 3 Stunden Ausrühren bei Raumtemperatur wird wieder auf 0 °C gekühlt, und es werden langsam 10 ml Wasser zugetropft. Zur resultierenden grauen Suspension wird Natriumsulfat zugegeben. Es scheidet sich eine organische Phase ab. Diese wird abgetrennt und das Gemisch mehrmals mit Heptan gewaschen. Die organischen Phasen werden vereinigt, mit wenig 0,5 molarer wässriger Methansulfonsäure und anschliessend mit Wasser gewaschen, über Natriumsulfat getrocknet und das Lösungsmittel unter Vakuum abdestilliert. Das gewünschte Produkt wird in guter Ausbeute als oranges Oel erhalten und ohne Reinigung weiterverwendet. ³¹P-NMR (C₆D₆, 121 MHz): -67,5 (d, P(Furyl)₂, -152,0 (d, PH₂). 1H-NMR (C₆D₆, 300 MHz), einige charakteristische Signale: 7,27 (m), 6,73 (m), 6,51 (m), 6,07 (m), 5,97 (m), 3,94 (s, 5H, Cyclopentadienring).

### Beispiel C5: Herstellung von

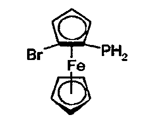

### a) Herstellung von

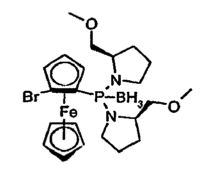

In einem 50 ml Rundkolben mit Argoneinlass wird Verbindung B2 (1,00 g, 2,18 mmol) in trockenem TBME (5,00 ml) und n-Hexan (5,00 ml) gelöst und die erhaltene Lösung auf -30 °C gekühlt. Dabei fällt das Edukt als gelber Feststoff aus. Es wird tropfenweise s-Butyl-Li (1,3 M in Cyclohexan; 1,76 ml, 2,29 mmol, 1,05 Äquivalente) zugegeben. Dabei geht der gelbe Feststoff allmählich in Lösung, die Lösung färbt sich orange-rot und nach etwa 30 Minuten fällt ein orange-farbener Feststoff aus.

Nach 2 Stunden Rühren bei -30 °C wird BrF₂C-CF₂Br (680 mg, 2,62 mmol, 1,2 Äquivalente) zugetropft, das Kühlbad entfernt und die Suspension 2 h unter Erwärmung auf RT gerührt. Das Reaktionsgemisch wird im Hochvakuum am Rotationsverdampfer bis zur Trockene eingeengt und ohne Reinigung in Stufe b) weiterverwendet. ³¹P-NMR (C₆D₆, 121 MHz): 76,5 (m).

### b) Herstellung von

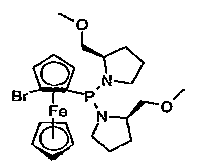

Für das Entfernen der Borangruppe wird der gemäss a) erhaltene Rückstand in 5 ml Diethylamin aufgenommen und über Nacht bei 50°C gerührt. Im Anschluss werden alle flüchtigen Bestandteile im Ölpumpenvakuum bei 50 °C entfernt. Der erhaltene Rückstand wird dreimal in Diethylamin (je 2,00 ml) aufgenommen, für 30 Minuten bei 50 °C gerührt und alle flüchtigen Bestandteile bei 50 °C im Ölpumpenvakuum entfernt (30 Minuten). Der Rückstand wird zweimal in trockenem TBME (2 ml) aufgenommen und alle flüchtigen Bestandteile bei 50 °C im Ölpumpenvakuum entfernt. Zurück bleibt das entschützte Produkt, welches ohne Reinigung in Stufe c) weiterverwendet wird.

### c) Herstellung von

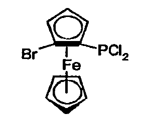

Das gemäss b) hergestellte Reaktionsprodukt wird in 5 ml TBME gelöst und bei 0°C tropfenweise unter Rühren mit 2,6 mmol einer HCI-Lösung (2n in Diethylether) versetzt. Die Reaktionslösung wird anschliessend durch Dekantieren von den ausgefallenen Ammoniumverbindungen abgetrennt und unter Argon am Rotationsverdampfer flüchtige Bestandteile entfernt. Der Rückstand wird direkt in Stufe d) eingesetzt. ³¹P-NMR (C₆D₆, 121 MHz): 160,6 (s)

### d) Herstellung der Titelverbindung

2 mmol Lithiumaluminiumhydrid werden unter Argon in 8 ml absoslutem THF suspendiert und auf -78°C gekühlt. Das gemäss c) hergestellte Rohprodukt wird in 8 ml absolutem THF gelöst und zur gekühlten Suspension von Lithiumaluminiumhydrid getropft. Das Reaktionsgemisch wird 30 Minuten bei dieser Temperatur und dann 30 Minuten bei 20°C gerührt. Zur Suspension werden 1,7 ml 2N NaOH tropfenweise zugegeben und die überstehende Lösung zur Isolierung der orangen Titelverbindung abfiltriert und eingedampft. Das Rohprodukt wird direkt in Beispiel D1 weiterverwendet.

### D) Herstellung von 1-Halogen-2-phospholan-ferrocenen

### Beispiel D1: Herstellung von

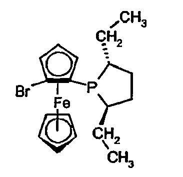

0,30 g (1 mmol) des Rohprodukts gemäss Beispiel C5d werden in 5 ml absolutem THF gelöst, entgast und mit 1 ml (1 mmol) einer 1 N Lösung von Lithiumdiisopropylamid in THF versetzt. Die resultierende rote Lösung wird zu 0,22 g (1,20 mmol) (3S,6S)-Oktan-3,6-diol-sulfat getropft. Bei Raumtemperatur werden weitere 1,2 ml 1 N Lithiumdiisopropylamid in THF zugesetzt und 60 Minuten nachgerührt. Es werden 8 ml Wasser zugefügt und dann mit Diethylether extrahiert. Die organischen Phasen werden über Natriumsulfat getrocknet und eingedampft. Man erhält die Titelverbindung als oranges, festes Produkt.

### E) Herstellung von erfindungsgemässen Ferrocendiphosphinen

### Beispiel E1: Herstellung von

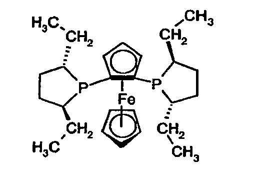

0,55 g (2,20 mmol) der Titelverbindung C1 werden in 10 ml absolutem THF gelöst, entgast und mit 2,2 ml (2,20 mmol) einer 1N Lösung von Lithiumdüsopropylamid in THF versetzt. Die rote Lösung wird zu 0,96 g (5,30 mmol) von (3R,6R)-Oktan-3,6-diol-sulfat getropft. Bei RT werden weitere 7 ml 1N Lithiumdiisopropylamid in THF zugesetzt und 60 Minuten nachgerührt. Dann werden 20 ml Wasser zugefügt und mit Diethylether extrahiert. Die organischen Phasen werden über Natriumsulfat getrocknet und eingedampft. Man erhält 0,848 g (82%) der Titelverbindung. ¹H NMR: 0,8-2,65 ppm (m, 32H); 4,20-4,25 (m, 6H); 4,37 (s, 1 H); 4,40 (s, 1H) und ³¹P NMR: -11,0 (d), -3,3 (d).

### Beispiel E2: Herstellung von

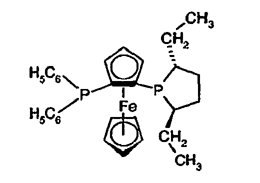

0,4 g (1 mmol) der Titelverbindung von Beispiel C2 werden in 5 ml absolutem THF gelöst, entgast und mit 1 ml (1 mmol) einer 1 N Lösung von Lithiumdiisopropylamid in THF versetzt. Die rote Lösung wird zu 0,22 g (1,20 mmol) (3S,6S)-Oktan-3,6-diol-sulfat getropft. Bei RT werden weitere 1,2 ml 1N Lithiumdiisopropylamid in THF zugesetzt und 60 Minuten nachgerührt. Es werden 8 ml Wasser zugefügt und dann mit Diethylether extrahiert. Die organischen Phasen werden über Natriumsulfat getrocknet und eingedampft. Man erhält die Titelverbindung als orangen Feststoff.

### Beispiel E3: Herstellung von

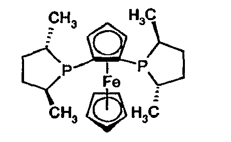

0,7 g (3,80 mmol) der Titelverbindung C1 werden in 10 ml absolutem THF gelöst, entgast und mit 1,05 molaren Äquivalenten einer 1 N Lösung von Lithiumdiisopropylamid in THF versetzt. Die rote Lösung wird zu 1,21 g (6,72 mmol) von (2R,5R)-Hexan-2,5-diol-sulfat getropft. Bei Raumtemperatur werden weitere 3,4 molare Äquivalente 1 N Lithiumdiisopropylamid in THF zugesetzt und 60 Minuten nachgerührt. Dann werden 20 ml Wasser zugefügt und mit Diethylether extrahiert. Die organischen Phasen werden über Natriumsulfat getrocknet und eingedampft. Man erhält 0,60 g (52%) der Titelverbindung. 1 H NMR: 0,8-0,9 (dd, 3H), 1,1-1,2 (m, 2H), 1,2-1,3 (dd, 3H), 1,4-1,5 (dd, 3H), 1,5-1,6 (dd, 3H), 1,8-2,2 (m, 5H), 2,3-2,4 (m, 1 H), 2,6-2,7 (m, 1 H), 3,1-3,2 (m, 1 H), 4,0 (m, 1 H), 4,2 (m, 6H), 4,3 (m, 1 H) und ³¹P NMR: -8,3 (d), 5,9 (d).

### Beispiel E4: Herstellung von

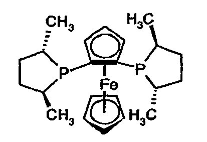

6,72 g (26,88 mmol) der Titelverbindung C1 werden in 100 ml absolutem THF gelöst, entgast und mit 1,05 molaren Äquivalenten einer 1N Lösung von Lithiumdüsopropylamid in THF versetzt. Die rote Lösung wird zu 10,17 g (56,45 mmol) von (2R,5R)-Hexan-2,5-diol-ditosylat getropft. Bei Raumtemperatur werden weitere 3,4 molare Äquivalente 1 N Lithiumdiisopropylamid in THF zugesetzt und 60 Minuten nachgerührt. Dann werden 20 ml Wasser zugefügt und mit Diethylether extrahiert. Die organischen Phasen werden über Natriumsulfat getrocknet, eingedampft und mittels Säulenchromatographie gereinigt (Pentan:Diethylether, 4:1). Man erhält 4,47 g (40%) der Titelverbindung. Die NMR-Spektren entsprechen denen der Verbindung gemäss Beispiel E3.

### Beispiel E5: Herstellung von

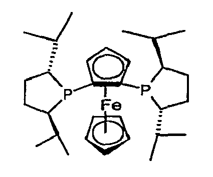

0,6 g (2,40 mmol) der Titelverbindung C1 werden in 10 ml absolutem THF gelöst, entgast und mit 1,05 molaren Äquivalenten einer 1 N Lösung von Lithiumdiisopropylamid in THF versetzt. Die rote Lösung wird zu 1,36 g (5,76 mmol) von (3S,6S)-2,7-Dimethyl-oktan-2,5-diolsulfat getropft. Bei Raumtemperatur werden weitere 3,4 molare Äquivalente 1 N Lithiumdiisopropylamid in THF zugesetzt und 60 Minuten nachgerührt. Dann werden 20 ml Wasser zugefügt und mit Diethylether extrahiert. Die organischen Phasen werden über Natriumsulfat getrocknet, eingedampft und mittels Säulenchromatographie gereinigt (Pentan:Diethylether, 4:1). Man erhält 0,85 g (67%) der Titelverbindung. 31P NMR: -21,8 (d), -0,1 (d).

### Beispiel E6: Herstellung von

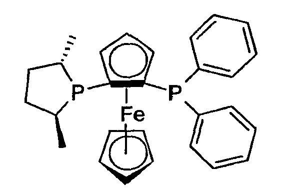

Zu einer Lösung von 410 mg (1,02 mmol) der Titelverbindung C2' in 12 ml THF werden 1,05 molare Äquivalente einer 1 N Lösung von Lithiumdiisopropylamid in THF gegeben. Die Lösung wird zu 221 mg (1,22 mmol) (2R,5R)-Hexan-2,5-diol-sulfat getropft. Bei RT werden wietere 1,2 molare Äquivalente einer 1N Lösung von Lithiumdiisopropylamid in THF zugetropft und eine Stunde nachgerührt. Nach Abdestillieren des Lösungsmittels wird das Rohprodukt mit Säulenchromatographie (Kieselgel 60, Laufmittel: Ethylacetat) gereinigt. Das Produkt wird in einer Ausbeute von 45% isoliert. ³¹P-NMR (C₆D₆, 121 MHz): -6,0 (d, J_{PP} ~90 Hz), -24,2 (d, J_{PP} ~90 Hz).

### Beispiel E7: Herstellung von

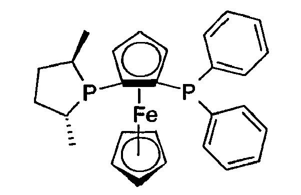

Zu einer Lösung von 436 mg (1.09 mmol) der Verbindung C2' in 12 ml THF werden 1,05 molare Äquivalente einer 1N Lösung von Lithiumdiisopropylamid in THF gegeben. Die Lösung wird zu 235 mg (1,3 mmol) (2S,5S)-Hexan-2,5-diol-sulfat getropft. Bei Raumtemperatur werden weitere 1,2 molare Äquivalente einer 1 N Lösung von Lithiumdiisopropylamid in THF zugetropft und 1 Stunde nachgerührt. Nach Abdestillieren des Lösungsmittels wird das Rohprodukt mittels Säulenchromatographie (Kieselgel 60, Laufmittel: Ethylacetat) gereinigt. Das Produkt wird in einer Ausbeute von 40% isoliert. ³¹P-NMR (C₆D₆, 121 MHz): -3,2 (d, J_{PP} -50 Hz), -23,1 (d, J_{PP} ∼50 Hz).

### Beispiel E8: Herstellung von

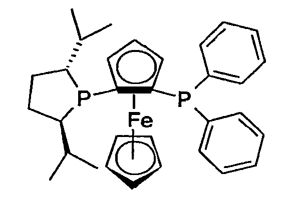

Zu einer Lösung von 450 mg (1,12 mmol) der Verbindung C2' in 10 ml THF werden 1,05 molare Äquivalente einer 1N Lösung von Lithiumdiisopropylamid in THF zugegeben. Die Lösung wird zu 295 mg (1,34 mmol) (3S,6S)-2,7-Dimethyl-oktan-2,5-diol-sulfat getropft. Bei RT werden weitere 1,2 molare Äquivalente einer 1N Lösung von Lithiumdiisopropylamid in THF zugetropft und 1 Stunde nachgerührt. Nach Abdestillieren des Lösungsmittels wird das Rohprodukt durch Säulenchromatographie (Kieselgel 60, Laufmittel: Ethylacetat) gereinigt. Das Produkt wird in einer Ausbeute von 50% isoliert. ³¹P-NMR (C₆D₆, 121 MHz): -13,0 (d, J_{PP} -80 Hz), -24,7 (d, J_{PP} -80 Hz).

### Beispiel E9: Herstellung von

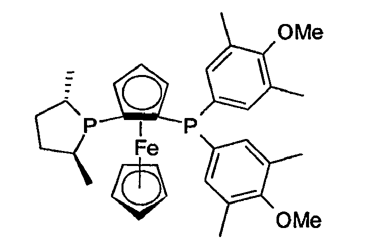

Zu einer Lösung von 1,13 g (3,08 mmol) der Verbindung C3 in 20 ml THF werden 1,05 molare Äquivalente einer 1N Lösung von Lithiumdiisopropylamid in THF gegeben. Die Lösung wird zu 3,7 mMol (2R,5R)-Hexan-2,5-diol-sulfat getropft. Bei Raumtemperatur (RT) werden weitere 1,2 molare Äquivalente einer 1 N Lösung von Lithiumdiisopropylamid in THF zugetropft und 1 Stunde nachgerührt. Nach Abdestillieren des Lösungsmittels wird das Rohprodukt in Ethylacetat / 10% wässerige NaBF₄ extrahiert. Die organischen Phasen werden mit Natriumsulfat getrocknet und unter Vakuum eingedampft. Das orange Produkt wird in einer Ausbeute von 80% erhalten. Bei Bedarf kann es durch Säulenchromatographie (Kieselgel 60, Laufmittel: Heptan/TBME 3:1) gereinigt werden. ³¹P-NMR (C₆D₆, 121 MHz): -6,6 (d, J_{PP} ∼ 85 Hz), -25,5 (d, J_{PP} ∼ 85 Hz).

### Beispiel E10: Herstellung von

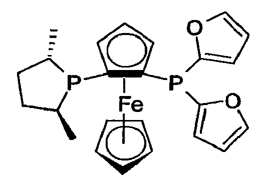

Zu einer Lösung von 400 mg (1,05 mmol) der Verbindung C4 in 10 ml THF werden 1,05 molare Äquivalente einer 1N Lösung von Lithiumdiisopropylamid in THF gegeben. Die Lösung wird zu 1,26 mMol (2R,5R)-Hexan-2,5-diol-sulfat getropft. Bei RT werden weitere 1,2 molare Äquivalente einer 1N Lösung von Lithiumdiisopropylamid in THF zugetropft und dann eine Stunde nachgerührt. Nach Abdestillieren des Lösungsmittels wird das Rohprodukt in Ethylacetat / 10% wässerige NaBF₄ extrahiert. Die organischen Phasen werden mit Natriumsulfat getrocknet und unter Vakuum eingedampft. Das orange Produkt wird in einer Ausbeute von 76% erhalten. Bei Bedarf kann es durch Säulenchromatographie (Kieselgel 60, Laufmittel: Heptan / TBME 3:1) gereinigt werden. ³¹P-NMR (C₆D₆, 121 MHz): -6,8 (d, J_{PP} ∼ 100 Hz), -71,1 (d, J_{PP} ∼ 100 Hz).

### Beispiel E11: Herstellung eines Phosphoniumsalzes 1,2-Bis(2,5-dimethylphospholano)ferrocen bis(hexafluorphosphat)

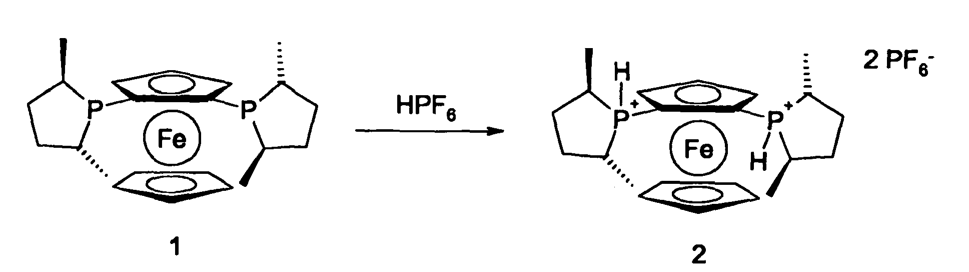

192 mg (0,46 mmol) 1 werden in 2 ml Dichlormethan gelöst und bei 0°C mit 135 µl (0,92 mmol) HPF₆ (65% in Wasser) versetzt. Die Mischung wird eingedampft. Durch Behandlung mit 5 ml Diethylether wird ein gelber Feststoff erhalten (327 mg, 86%). ³¹P NMR (ppm): -143 (Septett, PF₆), -8 (breites s, P⁺)

### F) Herstellung von Metallkomplexen

### Beispiel F1: Herstellung eines Rhodiumkomplexes

1 Äquivalent einer Suspension von [Rh(COD)₂]BF₄ in der 5-fachen Menge (w/w) THF wird unter Rühren auf 65°C erwärmt. Anschliessend wird eine Lösung von 1 Äquivalent des Diphosphins gemäß Beispiel E1 in der 5-fachen Menge (w/w) THF in einem Zeitraum von 20 Minuten zugetropft. Zur Reaktionslösung werden dann 4 ml TBME zugetropft. Beim langsamen Abkühlen bilden sich über Nacht Kristalle, welche durch Filtration und Nachwaschen mit THF isoliert werden. Die Mutterlauge wird unter reduziertem Druck vollständig eingedampft, der Rückstand in TBME aufgenommen und im Ultraschallbad mit einem Glasstab verrieben. Dabei entstehen feine gelbe Kristalle. Die Suspension wird auf 0°C gekühlt, 3 Stunden stehen gelassen und abfiltriert. Nach Waschen mit TBME werden diese Kristalle gesammelt und am Hochvakuum getrocknet. Beide Kristallfraktionen weisen identische NMR-Spektren auf. ³¹P NMR (CDCl₃, 121 MHz): 58,6 (d), 46,1 (d).

### Beispiel F2: Herstellung von

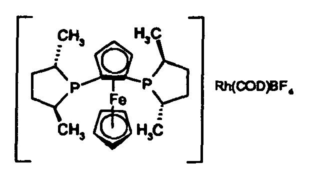

Gemäss der allgemeinen Vorschrift in Beispiel F1 wird die Verbindung aus Beispiel E4 mit [Rh(COD)₂]BF₄ umgesetzt und in einer Ausbeute von 90% die Titelverbindung erhalten. 31P NMR: 48,7 (dd), 60,0 (d).

### Beispiel F3: Herstellung von

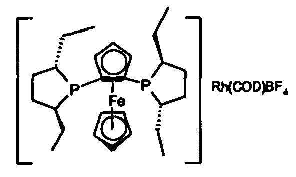

Gemäss der allgemeinen Vorschrift in Beispiel F1 wird die Verbindung aus Beispiel E1 mit [Rh(COD)₂)BF₄ umgesetzt und in einer Ausbeute von 87% die Titelverbindung erhalten. 31P NMR: 46,5 (dd), 58,4 (dd).

### Beispiel F4: Herstellung von

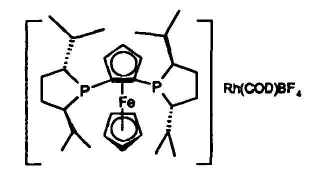

Gemäss der allgemeinen Vorschrift in Beispiel F1 wird die Verbindung aus Beispiel E5 mit [Rh(COD)₂]BF₄ umgesetzt und in einer Ausbeute von 67% die Titelverbindung erhalten. 31P NMR: 46,5 (dd), 58,3 (d).

### Beispiel F5: Herstellung von

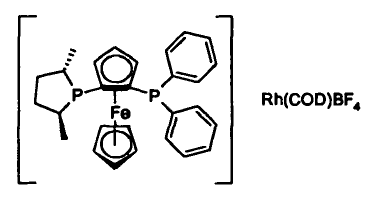

Gemäss der allgemeinen Vorschrift in Beispiel F1 wird die Verbindung aus Beispiel E6 mit [Rh(COD)₂]BF₄ umgesetzt und in einer Ausbeute von 70% die Titelverbindung erhalten. ³¹P-NMR (C₆D₆, 121 MHz): 53,5 (m), 40,0 (m).

### Beispiel F6: Herstellung von

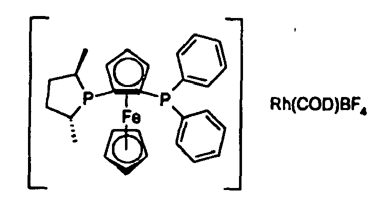

Gemäss der allgemeinen Vorschrift in Beispiel F1 wird die Verbindung aus Beispiel E7 mit [Rh(COD)₂]BF₄ umgesetzt und in einer Ausbeute von 85% die Titelverbindung erhalten. ³¹P-NMR (C₆D₆, 121 MHz): 59,8 (m), 37,1 (m).

### Beispiel F7: Herstellung von

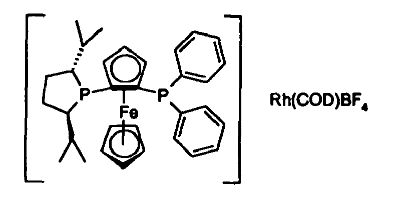

Gemäss der allgemeinen Vorschrift in Beispiel F1 wird die Verbindung aus Beispiel E8 mit [Rh(COD)₂]BF₄ umgesetzt und in einer Ausbeute von 55% die Titelverbindung erhalten. ³¹P-NMR (C₆D₆, 121 MHz): 42,0 (m), 39,4 (m).

### Beispiel F8: Herstellung von

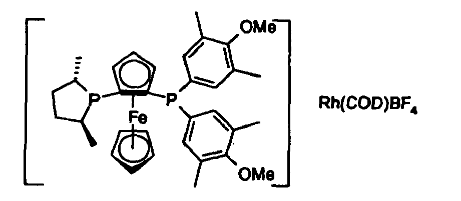

Gemäss der allgemeinen Vorschrift in Beispiel F1 wird die Verbindung aus Beispiel E9 mit [Rh(COD)₂]BF₄ umgesetzt und in einer Ausbeute von 35% die Titelverbindung erhalten. ³¹P-NMR (C₆D₈, 121 MHz): 52,2 (m), 37,0 (m).

### Beispiel F9: Herstellung von

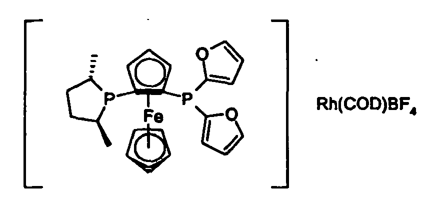

Gemäss der allgemeinen Vorschrift in Beispiel F1 wird die Verbindung aus Beispiel E10 mit [Rh(COD)₂]BF₄ umgesetzt und in einer Ausbeute von 62% die Titelverbindung erhalten. ³¹P-NMR (C₆D₆, 121 MHz): 53,4 (m), 1,0 (m).

### G) ANWENDUNGSBESIPIELE

Bei den Anwendungsbeispielen sind sowohl Umsatz als auch die optischen Ausbeuten (Enantiomerenüberschuss ee) nicht optimiert.

### Beispiel G1: Hydrierung von Dimethylitaconat (Hydrierung mit isoliertem Rh-Komplex)

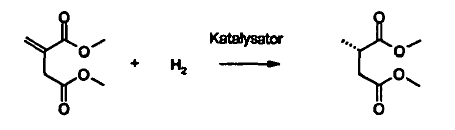

In einem 25 ml Kolben mit Magnetrührer und Gas- sowie Vakuumanschluss werden 3,85 mg (0,005 mmol) des Rhodium Komplexes gemäss Beispiel F1 vorgelegt. Der Kolben wird mit einem Septum verschlossen und durch mehrmaliges Evakuieren und Auffüllen mit Argon unter Argon gesetzt. Mit einer Spritze mit Nadel wird durch das Septum eine entgaste Lösung von 158 mg (1 mmol) Dimethylitaconat in 4 ml Methanol zugegeben und 10 Minuten gerührt. Der Rührer wird abgestellt, der Kolben durch Ansetzen von Vakuum und Auffüllen mit Wasserstoff (Normaldruck) unter Wasserstoff gesetzt und die Wasserstoffzufuhr offen gelassen. Durch Einschalten des Rührers wird die Hydrierung gestartet. Nach einer Stunde wird der Rührer abgestellt und die Reaktionslösung im Gaschromatograph mit chiraler Säule (Chirasil-L-val) untersucht. Der Umsatz ist vollständig, die optische Ausbeute ee des Hydrierproduktes (Dimethyl-methylsuccinat) ist grösser als 99%.

### Beispiel G2: Hydrierung von Dimethylitaconat

Analog zu Beispiel G2 wird die Hydrierung mit demRhodium-Komplex gemäß Beispiel F5 durchgeführt. Der Umsatz ist vollständig, die optische Ausbeute ee des Hydrierproduktes (Dimethyl-methylsuccinat) ist grösser als 98%.

Beispiel G3: Hydrierung von Dimethylitaconat (Hydrierung mit in situ hergestelltem Katalysator) In einem 25 ml Kolben mit Magnetrührer und Gas- sowie Vakuumanschluss werden 0,0055 mmol des Liganden gemäss Beispiel E9 vorgelegt. Der Kolben wird mit einem Septum verschlossen und durch mehrmaliges Evakuieren und Auffüllen mit Argon unter Argon gesetzt. Mit einer Spritze mit Nadel wird durch das Septum eine entgaste Lösung von 0,005 mmol [Rh(COD)₂]BF₄ in 1 ml Methanol 5 Minuten gerührt und anschließend eine Lösung von 158 mg (1 mmol) Dimethylitaconat in 4 ml Methanol zugegeben und 10 Minuten gerührt. Der Rührer wird abgestellt, der Kolben durch Ansetzen von Vakuum und Auffüllen mit Wasserstoff (Normaldruck) unter Wasserstoff gesetzt und die Wasserstoffzufuhr offen gelassen. Durch Einschalten des Rührers wird die Hydrierung gestartet. Nach einer Stunde wird der Rührer abgestellt und die Reaktionslösung im Gaschromatograph mit chiraler Säule (Chirasil-L-val) untersucht. Der Umsatz ist vollständig, die optische Ausbeute ee des Hydrierproduktes (Dimethyl-methylsuccinat) ist grösser als 97%.

### Beispiel G4: Hydrierung von

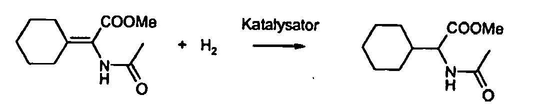

In einem 25 ml Kolben mit Magnetrührer und Gas- sowie Vakuumanschluss werden 0,011 mmol des Liganden gemäss Beispiel E4 vorgelegt. Der Kolben wird mit einem Septum verschlossen und durch mehrmaliges Evakuieren und Auffüllen mit Argon unter Argon gesetzt. Mit einer Spritze mit Nadel wird durch das Septum eine entgaste Lösung von 0,01 mmol [Rh(Norbornadien)₂]BF₄ in 1 ml Methanol 5 Minuten gerührt und anschließend eine Lösung von 1 mmol N-Acetyl-cyclohexylidenglycin-methylester in 4 ml Methanol zugegeben und 10 Minuten gerührt. Der Rührer wird abgestellt, der Kolben durch Ansetzen von Vakuum und Auffüllen mit Wasserstoff (Normaldruck) unter Wasserstoff gesetzt und die Wasserstoffzufuhr offen gelassen. Durch Einschalten des Rührers wird die Hydrierung gestartet und es wird über Nacht gerührt. Dann wird der Rührer abgestellt und die Reaktionslösung wird gaschromatographisch mit einer chiralen Säule (Chirasil-L-val) untersucht. Der Umsatz liegt bei über 80% und die optische Ausbeute ee an N-Acetyl-cyclohexylglycin-methylester ist 91%.

### Beispiel G5: Hydrierung von

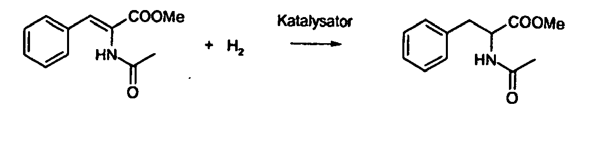

Die Hydrierung wird analog zu Beispiel G4 mit Acetamidozimtsäure-methylester und Ligand E5 durchgeführt: Der Umsatz ist laut Gaschromatographie (Chirasi)-L-val) vollständig und die optische Ausbeute ee ist 97% .

### Beispiel G6: Hydrierung von

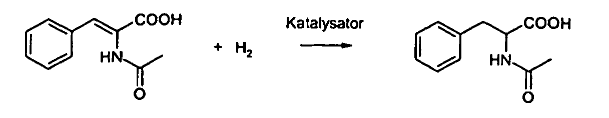

Die Hydrierung wird analog zu Beispiel G4 mit Acetamidozimtsäure durchgeführt. Das Produkt wird mit Diazomethan zum entsprechenden Methylester überführt und gaschromatographisch (Chirasil-L-val) untersucht. Der Umsatz ist vollständig und die optische Ausbeute ee ist 97%.

## Patentansprüche

1. Verbindungen der Formel I in Form von Racematen, Gemischen von Diastereomeren oder im wesentlichen reinen Diastereomeren, 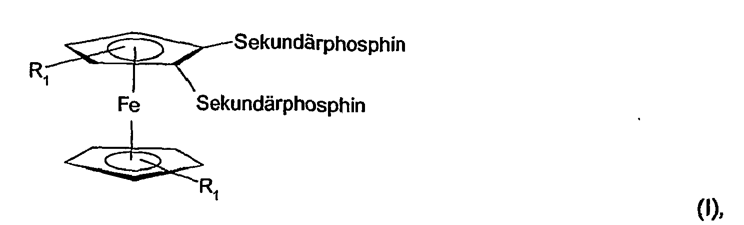 worin
R₁ Wasserstoffatom oder C₁-C₄-Alkyl bedeutet, und wenigstens ein Sekundärphosphin eine unsubstituierte oder substituierte zyklische Phosphingruppe darstellt, oder deren Phosphoniumsalze mit ein oder zwei monovalenten Anionen oder einem divalenten Anion.

2. Verbindungen gemäss Anspruch 1, worin das zyklische Sekundärphosphin den Formeln II, IIa, IIb oder IIc entspricht, 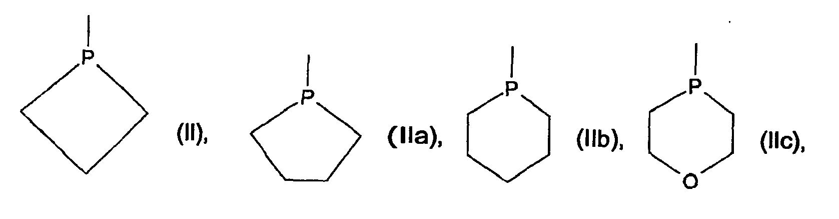 die unsubstituiert oder ein- oder mehrfach substituiert sind mit -OH, C₁-C₈-Alkyl, C₄-C₈-Cycloalkyl, C₁-C₆-Alkoxy, C₁-C₄-Alkoxy-C₁-C₄-Alkyl, Phenyl, C₁-C₄-Alkyl- oder C₁-C₄-Alkoxyphenyl, Benzyl, C₁-C₄-Alkyl- oder C₁-C₄-Alkoxybenzyl, Benzyloxy, C₁-C₄-Alkyl- oder C₁-C₄-Alkoxybenzyloxy, oder C₁-C₄-Alkyliden-dioxyl.

3. Verbindungen gemäss Anspruch 2, worin Substituenten in einer oder beiden α-Stellungen zum P-Atom gebunden sind.

4. Verbindungen gemäss Anspruch 1, worin die Verbindungen der Formel I den Formeln III oder IV entsprechen, 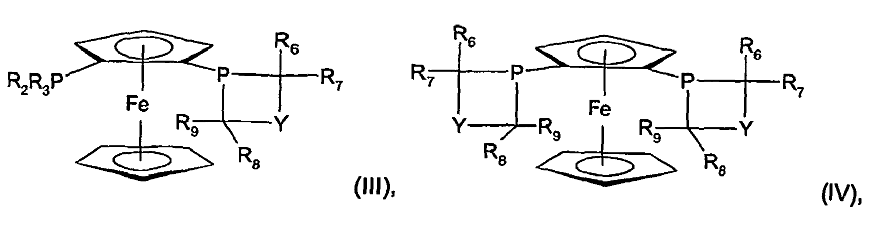 worin
R₂ und R₃ unabhängig voneinander einen Kohlenwasserstoffrest mit 1 bis 20 C-Atomen darstellen, der unsubstituiert oder substituiert ist mit Halogen, C₁-C₆-Alkyl, C₁-C₆-Halogenalkyl, C₁-C₆-Alkoxy, C₁-C₆-Halogenalkoxy, Di-C₁-C₄-Alkylamino, (C₆H₅)₃Si, (C₁-C₁₂-Alkyl)₃Si, oder - CO₂-C₁-C₆-Alkyl,
Y für -CH₂-, -CH₂CH₂-, -CH₂CH₂CH₂-, -CH(OH)CH(OH)-, -CH(OC₁-C₄-Alkyl)CH(OC₁-C₄-Alkyl)- steht, oder einen Rest der Formel 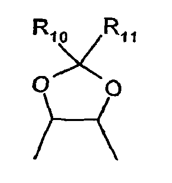 bedeutet,
R₆, R₇, R₈ und R₉ unabhängig voneinander H, C₁-C₄-Alkyl oder Benzyl darstellen, und wenigstens einer der Reste R₆, R₇, R₈ und R₉ C₁-C₄-Alkyl, Benzyl oder -CH₂-O-C₁-C₄-Alkyl beziehungsweise -CH₂-O-C₆-C₁₀-Aryl bedeutet,
R₁₀ H oder C₁-C₄-Alkyl ist, und
R₁₁ C₁-C₄-Alkyl bedeutet.

5. Verfahren zur Herstellung von Verbindungen der Formel I in Form von Racematen, Gemischen von Diastereomeren oder im wesentlichen reinen Diastereomeren, 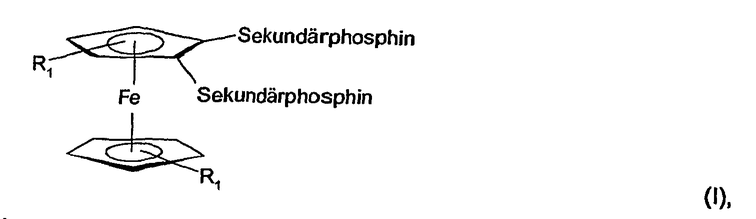 worin
R₁ Wasserstoffatom oder C₁-C₄-Alkyl bedeutet, und wenigstens ein Sekundärphosphin eine unsubstituierte oder substituierte zyklische Phosphingruppe darstellt, umfassend die Schritte
a) Umsetzung einer Verbindung der Formel V 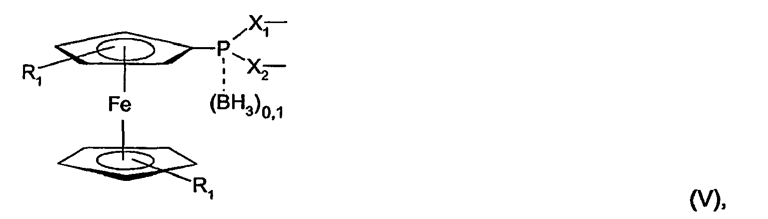 worin
X₁ und X₂ unabhängig voneinander O oder N- bedeuten, und an die freien Bindungen der O- und N-Atome C-gebundene Kohlenwasserstoff- oder Heterokohlenwasserstoffreste gebunden sind,
mit wenigstens äquivalenten Mengen Lithiumalkyl, einer Magnesium-Grignardverbindung, oder einem aliphatischen Li-Sekundäramid oder X₃Mg-Sekundäramid zu einer Verbindung der Formel VI, 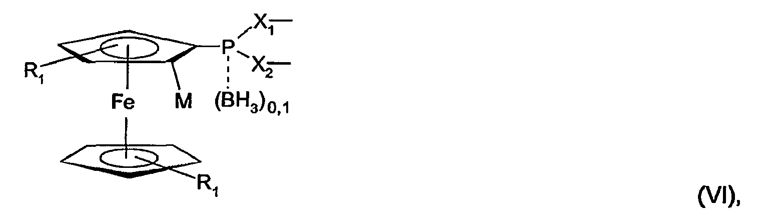 worin
M für -Li oder -MgX₃ steht und X₃ Cl, Br oder I darstellt,
b) Umsetzung der Verbindung der Formel VI mit wenigstens äquivalenten Mengen eines Disekundäramino-phosphinhalogenids, eines Dialkoxyphosphinhalogenids, Disekundäramino-P(O)-halogenid, Dialkoxy-P(O)-halogenid oder PCl₃ oder PBr₃ zu einer Verbindung der Formel VII 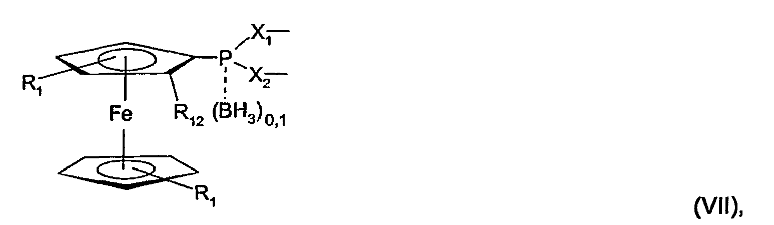 worin R₁₂ -PCl₂, -PBr₂, Di(sekundäramino)P-, DialkoxyP-, Disekundäramino-P(O)-, Dialkoxy-P(O)-darstellt, und
b1) aus einer Verbindung der Formel VII, wenn vorhanden, die Borangruppe entfernt, dann die Reste (Hetero)Kohlenwasserstoff-X₁, (Hetero)Kohlenwasserstoff-X₂, oder X₁-(Hetero)-Kohlenwasserstoff-X₂ sowie Disekundäramino oder Dialkoxy mit HCl oder HBr unter Bildung einer -PCl₂-Gruppe oder -PBr₂-Gruppe abspaltet, und danach die -(O)PCl₂-Gruppen, -(O)PBr₂-Gruppen, -PCl₂-Gruppen oder -PBr₂-Gruppen zu einer Verbindung der Formel VIII hydriert, oder
b2) aus einer Verbindung der Formel VII die Reste (Hetero)Kohlenwasserstoff-X₁, (Hetero)-Kohlenwasserstoff-X₂, oder X₁-(Hetero)Kohlenwasserstoff-X₂ sowie Disekundäramino oder Dialkoxy mit HCl oder HBr unter Bildung einer -PCl₂-Gruppe oder -PBr₂-Gruppe abspaltet, und danach die -(O)PCl₂-Gruppen, -(O)PBr₂-Gruppen,-PCl₂-Gruppen oder -PBr₂-Gruppen hydriert, und dann die Borangruppe zur Bildung einer Verbindung der Formel VIII, 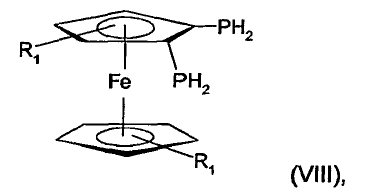 entfernt, oder
c) Umsetzung einer Verbindung der Formel VI mit einem Sekundärphosphin-halogenid zu einer Verbindung der Formel IX, 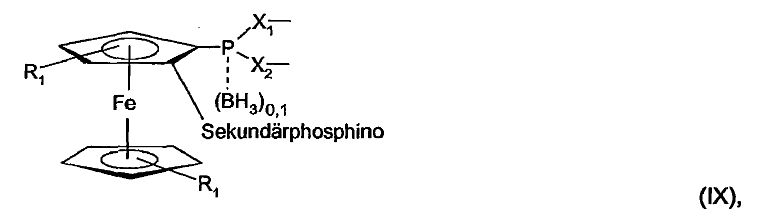
c1) aus einer Verbindung der Formel IX, wenn vorhanden, die Borangruppe entfernt, dann die Reste (Hetero)Kohtenwasserstoff-X₁, (Hetero)Kohlenwasserstoff-X₂, oder X₁-(Hetero)-Kohlenwasserstoff-X₂ mit HCl oder HBr unter Bildung einer -PCl₂-Gruppe oder -PBr₂-Gruppe abspaltet, und danach die -PCl₂-Gruppen oder -PBr₂-Gruppen zu einer Verbindung der Formel X hydriert, oder
c2) aus einer Verbindung der Formel IX die Reste (Hetero)Kohlenwasserstoff-X₁, (Hetero)-Kohlenwasserstoff-X₂, oder X₁-(Hetero) Kohlenwasserstoff-X₂ mit HCl oder HBr unter Bildung einer -PCl₂-Gruppe oder -PBr₂-Gruppe abspaltet, und danach die -PCl₂-Gruppen oder -PBr₂-Gruppen hydriert, und dann die Borangruppe zur Bildung einer Verbindung der Formel X 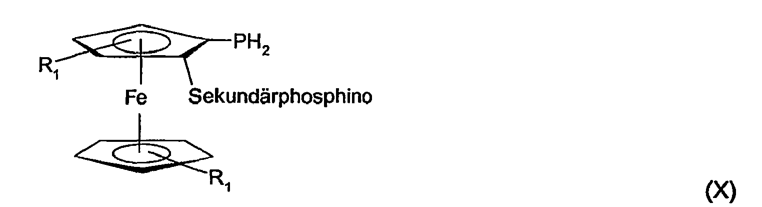 entfernt, oder
d) Umsetzung einer Verbindung der Formel VI mit einem Halogenierungsreagenz zu einer Verbindung der Formel XI 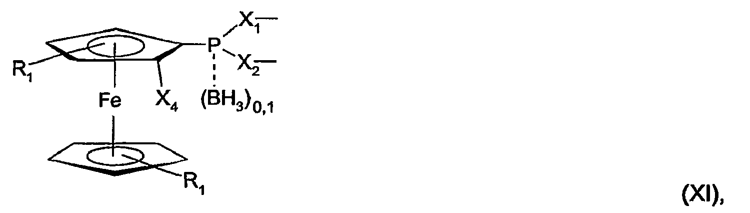 worin X₄ für Cl, Br oder I steht,
d1) aus einer Verbindung der Formel XI, wenn vorhanden, die Borangruppe entfernt, dann die Reste (Hetero)Kohlenwasserstoff-X₁, (Hetero)Kohlenwasserstoff-X₂, oder X₁-(Hetero)-Kohlenwasserstoff-X₂ mit HCl oder HBr unter Bildung einer -PCl₂-Gruppe oder -PBr₂-Gruppe abspaltet, und danach die -PCl₂-Gruppe oder -PBr₂-Gruppe zu einer Verbindung der Formel XII hydriert, oder
d2) aus einer Verbindung der Formel XI die Reste (Hetero)Kohlenwasserstoff-X₁, (Hetero)-Kohlenwasserstoff-X₂, oder X₁-(Hetero) Kohlenwasserstoff-X₂ mit HCl oder HBr unter Bildung einer -PCl₂-Gruppe oder -PBr₂-Gruppe abspaltet, und danach die -PCl₂-Gruppen oder -PBr₂-Gruppen hydriert, und dann die Borangruppe zur Bildung einer Verbindung der Formel XII 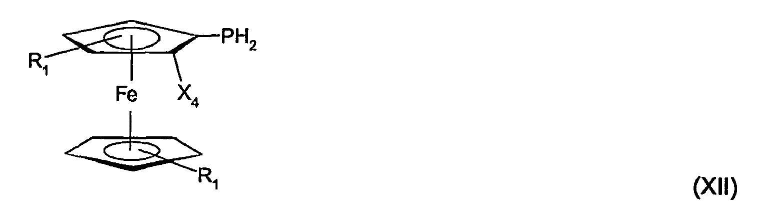 entfernt, und
d3) die Verbindung der Formel XII mit einem metallierten Sekundärphosphid zu einer Verbindung der Formel X umsetzt,
e) Umsetzung der Verbindung der Formel VII mit wenigstens 2 Äquivalenten und der Verbindung der Formel X mit wenigstens 1 Äquivalent eines zyklischen Sulfats oder eines offenkettigen Disulfonats zur Herstellung von Verbindungen der Formel I, worin eine oder beide Sekundärphosphinogruppen zyklisches Sekundärphosphino darstellen, oder
f) Umsetzung einer Verbindung der Formel XII mit wenigstens 1 Äquivalent eines zyklischen Sulfats oder eines offenkettigen Disulfonats zur Herstellung von Verbindungen der Formel XIII, 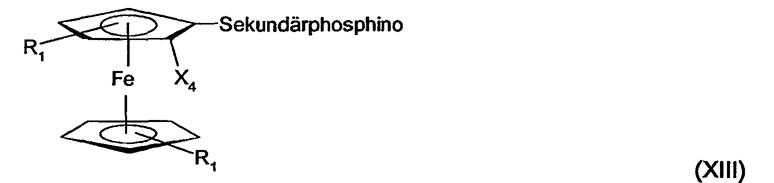 worin Sekundärphosphino zyklisches Sekundärphosphino darstellt, die gegebenenfalls mit BH₃ geschützt ist, und danach Umsetzung einer Verbindung der Formel XIII mit wenigstens 1 Äquivalent Lithiumalkyl und dann mit wenigstens 1 Äquivalent Sekundärphosphin-halogenid zu einer Verbindung der Formel I.

6. Verbindungen der Formeln VII, IX und XI, 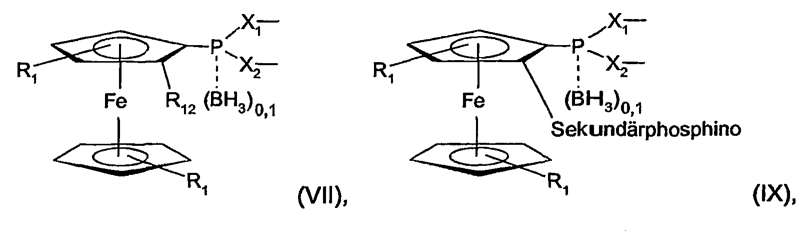 und 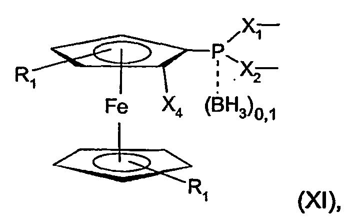 worin
X₁ und X₂ unabhängig voneinander O oder N- bedeuten, und an die freien Bindungen der O- und N-Atome C-gebundene Kohlenwasserstoff- oder Heterokohlenwasserstoffreste gebunden sind, und
R_{1'} R₁₂ und X₄ die in Anspruch 5 angegebenen Bedeutungen haben.

7. Verbindungen der Formeln VIII, X und XII, 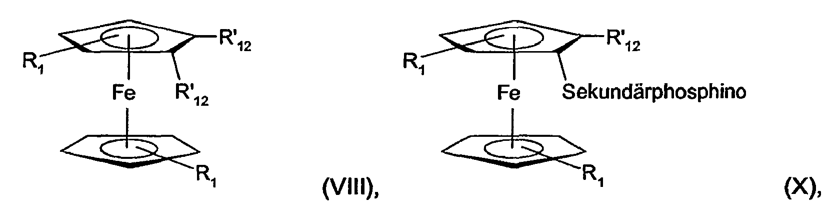 und 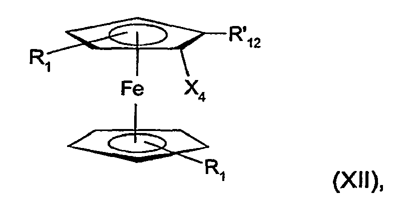 worin R'₁₂ für -PCl₂, -PBr₂ oder -PH₂ steht, und R₁ und X₄ die in Anspruch 5 angegebenen Bedeutungen haben.

8. Verbindungen der Formel XIII 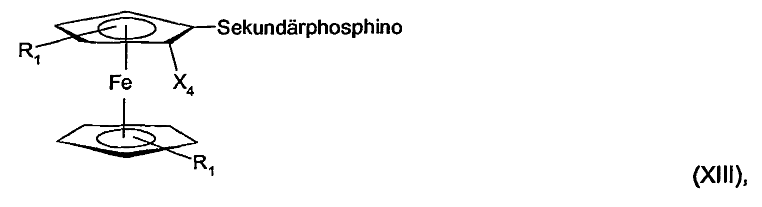 worin R₁ und X₄ die die in Anspruch 5 angegebenen Bedeutungen haben, und Sekundärphosphino zyklisches Sekundärphosphino darstellt.

9. Metallkomplexe von Metallen ausgewählt aus der Gruppe der TM8-Metalle mit Verbindungen der Formeln I als Liganden.

10. Metallkomplexe gemäss Anspruch 9, worin das TM-8 Metall Ruthenium, Rhodium oder Iridium ist.

11. Metallkomplexe gemäss Anspruch 9, **dadurch gekennzeichnet, dass** sie den Formeln XIV und XV entsprechen,
A₁MeLₙ (XIV),
(A₁MeLₙ)^{(z+)}(E⁻)_{z} (XV),
worin
A₁ für eine Verbindung der Formel I steht,
L für gleiche oder verschiedene monodentate, anionische oder nicht-ionische Liganden steht, oder zwei L für gleiche oder verschiedene bidentate, anionische oder nicht-ionische Liganden steht;
n für 2, 3 oder 4 steht, wenn L einen monodentaten Liganden bedeutet, oder n für 1 oder 2 steht, wenn L einen bidentaten Liganden bedeutet;
z für 1, 2 oder 3 steht;
Me ein Metall ausgewählt aus der Gruppe Rh, Ir und Ru bedeutet; wobei das Metall die Oxidationsstufen 0, 1, 2, 3 oder 4 aufweist;
E- das Anion einer Sauerstoffsäure oder Komplexsäure ist; und
die anionischen Liganden die Ladung der Oxidationsstufen 1, 2, 3 oder 4 des Metalls ausgleichen.

12. Metallkomplexe gemäss Anspruch 9, **dadurch gekennzeichnet, dass** sie den Formeln XVI und XVII entsprechen,
[A₁Me₂YZ] (XVI),
[A₁Me₂Y]⁺E₁⁻ (XVII),
worin
A₁ für eine Verbindung der Formel I steht;
Me₂ Rhodium oder Iridium bedeutet;
Y für zwei Olefine oder ein Dien steht;
Z Cl, Br oder 1 bedeutet; und
E₁⁻ das Anion einer Sauerstoffsäure oder Komplexsäure darstellt.

13. Verwendung von Metallkomplexen gemäss Anspruch 9 als homogene Katalysatoren zur Herstellung chiraler organischer Verbindungen durch asymmetrische Anlagerung von Wasserstoff, Borhydriden oder Silanen an eine Kohlenstoff- oder Kohlenstoff-Heteroatommehrfachbindung in prochiralen organischen Verbindungen, oder die asymmetrische Addition von C-Nukleophilen oder Aminen an Allylverbindungen.

14. Verfahren zur Herstellung chiraler organischer Verbindungen durch asymmetrische Anlagerung von Wasserstoff, Borhydriden oder Silanen an eine Kohlenstoff- oder Kohlenstoff-Heteroatommehrfachbindung in prochiralen organischen Verbindungen, oder die asymmetrische Addition von C-Nukleophilen oder Amine an Allylverbindungen in Gegenwart eines Katalysators, **dadurch gekennzeichnet, dass** man die Anlagerung in Gegenwart katalytischer Mengen wenigstens eines Metallkomplexes gemäss Anspruch 9 durchführt.

## Claims

1. A compound of the formula I in the form of a racemate, a mixture of diastereomers or an essentially pure diastereomer, 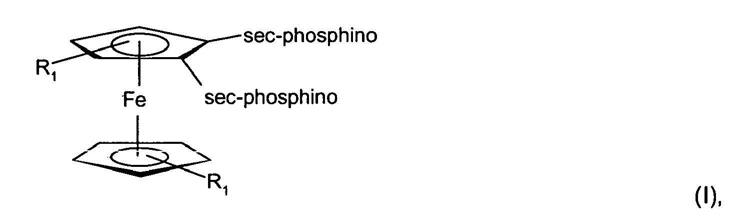 where
R₁ is a hydrogen atom or C₁-C₄-alkyl and at least one sec-phosphine group is an unsubstituted or substituted cyclic phosphino group, or a phosphonium salt thereof having one or two monovalent anions or one divalent anion.

2. The compound as claimed in claim 1, wherein the cyclic sec-phosphino corresponds to the formula II, IIa, IIb or IIc, 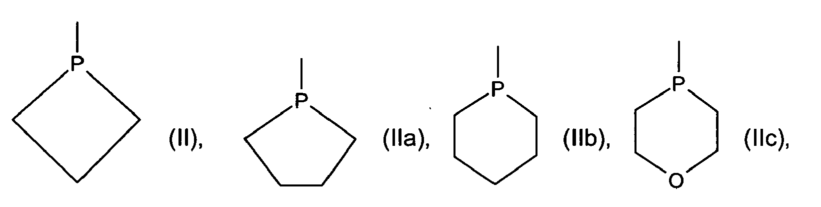 which are unsubstituted or substituted by one or more -OH, C₁-C₈-alkyl, C₄-C₈-cycloalkyl, C₁-C₆-alkoxy, C₁-C₄-alkoxy-C₁-C₄-alkyl, phenyl, C₁-C₄-alkyl- or C₁-C₄-alkoxyphenyl, benzyl, C₁-C₄-alkyl- or C₁-C₄-alkoxybenzyl, benzyloxy, C₁-C₄-alkyl- or C₁-C₄-alkoxybenzyloxy or C₁-C₄-alkylidenedioxyl groups.

3. The compound as claimed in claim 2, wherein substituents are present in one or both α positions relative to the P atom.

4. The compound as claimed in claim 1, wherein the compound of the formula I corresponds to the formula III or IV, 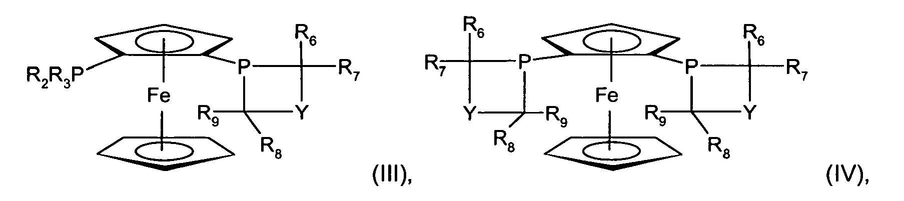 where
R₂ and R₃ are each, independently of one another, a hydrocarbon radical which has from 1 to 20 carbon atoms and is unsubstituted or substituted by halogen, C₁-C₆-alkyl, C₁-C₆-haloalkyl, C₁-C₆-alkoxy, C₁-C₆-haloalkoxy, di-C₁-C₄-alkylamino, (C₆H₅)₃Si, (C₁-C₁₂-alkyl)₃Si, or -CO₂-C₁-C₆-alkyl,
Y is -CH₂-, -CH₂CH₂-, -CH₂CH₂CH₂-, -CH(OH)CH(OH)-, -CH(OC₁-C₄-alkyl)CH(OC₁-C₄-alkyl)- or a radical of the formula 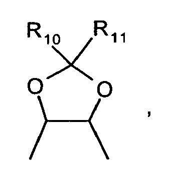
R₆, R₇, R₈ and R₉ are each, independently of one another, H, C₁-C₄-alkyl or benzyl, and at least one of the radicals R₆, R₇, R₈ and R₉ is C₁-C₄-alkyl, benzyl or -CH₂-O-C₁-C₄-alkyl or -CH₂-O-C₆-C₁₀-aryl,
R₁₀ is H or C₁-C₄-alkyl and
R₁₁ is C₁-C₄-alkyl.

5. A process for preparing compounds of the formula I in the form of racemates, mixtures of diastereomers or essentially pure diastereomers, 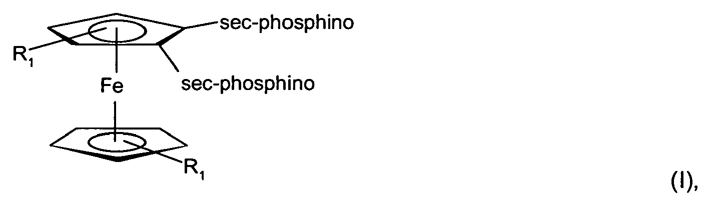 where
R₁ is a hydrogen atom or C₁-C₄-alkyl and at least one sec-phosphino is an unsubstituted or substituted cyclic phosphino group, which comprises the steps
a) reaction of a compound of the formula V 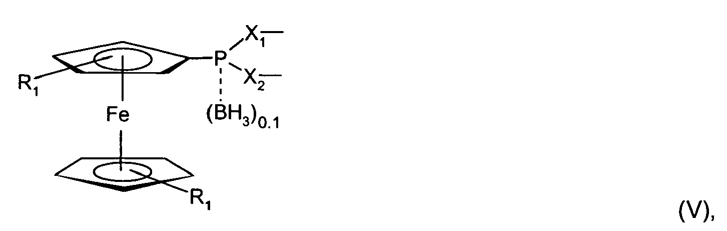 where
X₁ and X₂ are each, independently of one another, O or N and C-bonded hydrocarbon or heterohydrocarbon radicals are bound to the free bonds of the O and N atoms, with at least equivalent amounts of a lithium alkyl, a magnesium Grignard compound or an aliphatic Li sec-amide or X₃Mg sec-amide to form a compound of the formula VI, 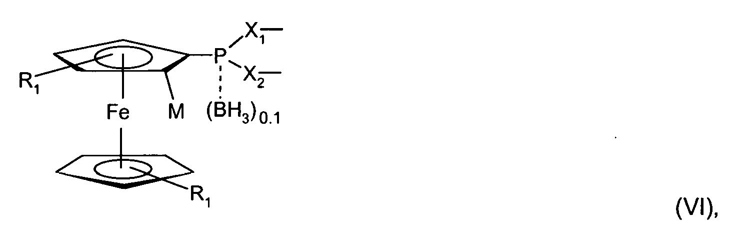 where
M is -Li or -MgX₃ and X₃ is Cl, Br or I,
b) reaction of the compound of the formula VI with at least equivalent amounts of a di-sec-aminophosphine halide, a dialkoxyphosphine halide, di-sec-amino-P(O) halide, dialkoxyP(O) halide or PCl₃ or PBr₃ to form a compound of the formula VII 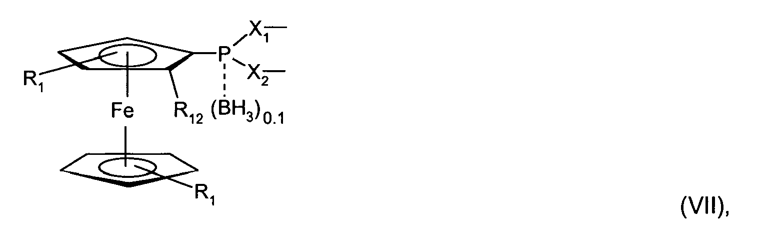 where
R₁₂ is -PCl₂, -PBr₂, di(sec-amino)P-, dialkoxyP-, di-sec-amino-P(O)-, dialkoxy-P(O)-, and
b1) removing any borane group present from a compound of the formula VII, then splitting off the radicals (hetero)hydrocarbon-X₁, (hetero)hydrocarbon-X₂ or X₁-(hetero)hydrocarbon-X₂ or di-sec-amino or dialkoxy by means of HCl or HBr to form a -PCl₂ group or -PBr₂ group and then hydrogenating the -(O)PCl₂ groups, -(O)PBr₂ groups, -PCl₂ groups or -PBr₂ groups to form a compound of the formula VIII or
b2) splitting off the radicals (hetero)hydrocarbon-X₁, (hetero)hydrocarbon-X₂ or X₁-(hetero)hydrocarbon-X₂ or di-sec-amino or dialkoxy from a compound of the formula VII by means of HCl or HBr to form a -PCl₂ group or -PBr₂ group and then hydrogenating the -(O)PCl₂ groups, -(O)PBr₂ groups, -PCl₂ groups or -PBr₂ groups and then removing the borane group to form a compound of the formula VIII, 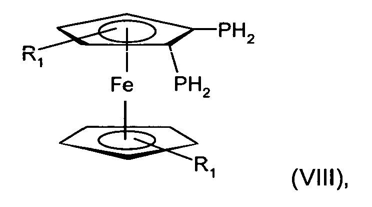 or
c) reaction of a compound of the formula VI with a sec-phosphine halide to form a compound of the formula IX, 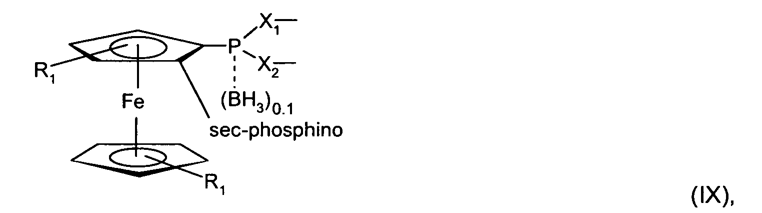
c1) removing any borane group present from a compound of the formula IX, then splitting off the radicals (hetero)hydrocarbon-X₁, (hetero)hydrocarbon-X₂ or X₁-(hetero)hydrocarbon-X₂ by means of HCl or HBr to form a -PCl₂ group or -PBr₂ group and then hydrogenating the -PCl₂ groups or -PBr₂ groups to form a compound of the formula X or
c2) splitting off the radicals (hetero)hydrocarbon-X₁, (hetero)hydrocarbon-X₂ or X₁-(hetero)-hydrocarbon-X₂ from a compound of the formula IX by means of HCl or HBr to form a -PCl₂ group or -PBr₂ group and then hydrogenating the -PCl₂ groups or -PBr₂ groups and then removing the borane group to form a compound of the formula X 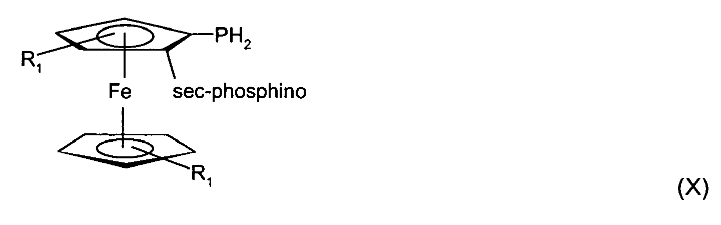 or
d) reaction of a compound of the formula VI with a halogenating reagent to form a compound of the formula XI 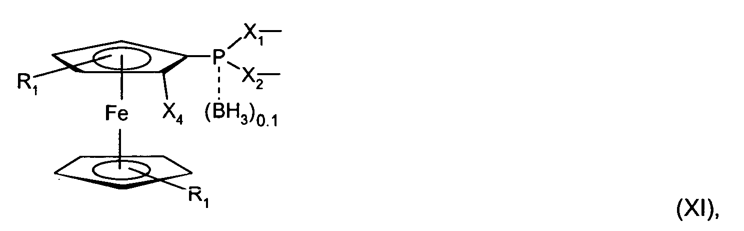 where X₄ is Cl, Br or I,
d1) removing any borane group present from a compound of the formula XI, then splitting off the radicals (hetero)hydrocarbon-X₁, (hetero)hydrocarbon-X₂ or X₁-(hetero)hydrocarbon-X₂ by means of HCl or HBr to form a -PCl₂ group or -PBr₂ group and then hydrogenating the -PCl₂ group or -PBr₂ group to form a compound of the formula XII or
d2) splitting off the radicals (hetero)hydrocarbon-X₁, (hetero)hydrocarbon-X₂ or X₁-(hetero)-hydrocarbon-X₂ from a compound of the formula XI by means of HCl or HBr to form a -PCl₂ group or -PBr₂ group and then hydrogenating the -PCl₂ groups or -PBr₂ groups and then removing the borane group to form a compound of the formula XII 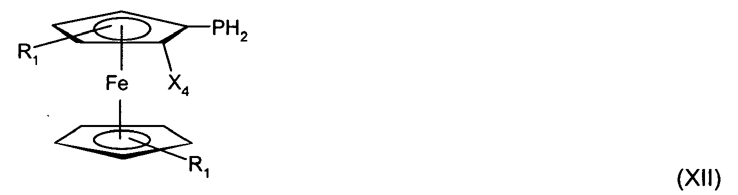 and
d3) reacting the compound of the formula XII with a metalated sec-phosphide to form a compound of the formula X,
e) reaction of the compound of the formula VII with at least 2 equivalents and of the compound of the formula X with at least 1 equivalent of a cyclic sulfate or an open-chain disulfonate to produce compounds of the formula I in which one or both sec-phosphino groups are cyclic sec-phosphino or
f) reaction of a compound of the formula XII with at least 1 equivalent of a cyclic sulfate or an open-chain disulfonate to produce compounds of the formula XIII, 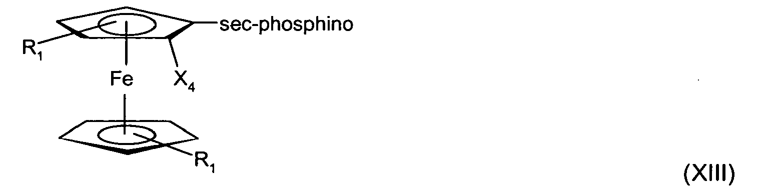 where sec-phosphino is cyclic sec-phosphino which may, if appropriate, be protected by BH₃, and then reaction of a compound of the formula XIII with at least 1 equivalent of a lithium alkyl and then with at least 1 equivalent of a sec-phosphine halide to form a compound of the formula I.

6. A compound of the formula VII, IX and XI, 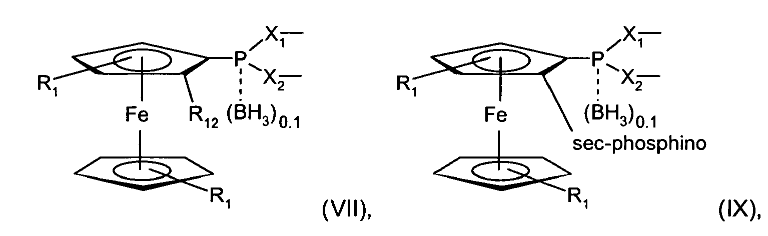 and 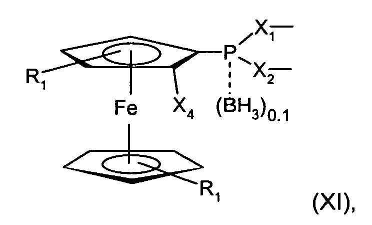 where
X₁ and X₂ are each, independently of one another, O or N and C-bonded hydrocarbon or heterohydrocarbon radicals are bound to the free bonds of the O and N atoms and
R₁, R₁₂ and X₄ are as defined in claim 5.

7. A compound of the formula VIII, X or XII, 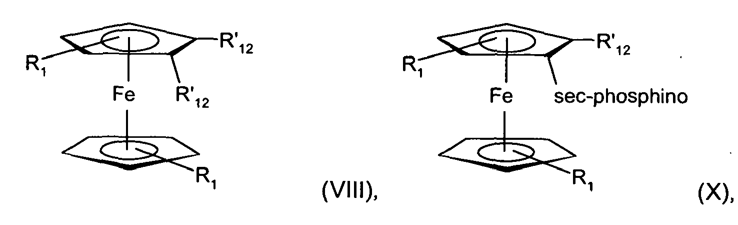 and 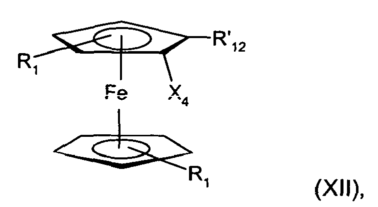 where R'₁₂ is -PCl₂, -PBr₂ or -PH₂ and R₁ and X₄ are as defined in claim 5.

8. A compound of the formula XIII 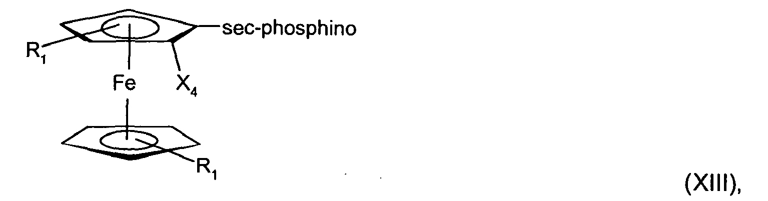 where R₁ and X₄ are as defined in claim 5 and sec-phosphino is cyclic sec-phosphino.

9. A complex of a metal selected from the group consisting of the group 8 transition metals with compounds of the formula I as ligands.

10. The metal complex as claimed in claim 9, wherein the group 8 transition metal is ruthenium, rhodium or iridium.

11. The metal complex as claimed in claim 9, **characterized in that** it corresponds to the formula XIV or XV,
A₁MeLₙ (XIV),
(A₁MeLₙ)^{(z+)}(E⁻)_{z} (XV),
where
A₁ is a compound of the formula I,
L represents identical or different monodentate, anionic or nonionic ligands, or L₂ represents identical or different bidentate, anionic or nonionic ligands;
n is 2, 3 or 4 when L is a monodentate ligand or n is 1 or 2 when L is a bidentate ligand;
z is 1, 2 or 3;
Me is a metal selected from the group consisting of Rh, Ir and Ru; with the metal having the oxidation state 0, 1, 2, 3 or 4;
E⁻ is the anion of an oxo acid or complex acid; and
the anionic ligands balance the charge of the oxidation state 1, 2, 3 or 4 of the metal.

12. The metal complex as claimed in claim 9, **characterized in that** it corresponds to the formula XVI or XVII, I,
[A₁Me₂YZ] (XVI),
[A₁Me₂Y]⁺E₁⁻ (XVII),
where
A₁ is a compound of the formula I;
Me₂ is rhodium or iridium;
Y represents two olefins or diene;
Z is Cl, Br or I; and
E₁⁻ is the anion of an oxo acid or complex acid.

13. The use of metal complexes as claimed in claim 9 as homogeneous catalysts for preparing chiral organic compounds by asymmetric addition of hydrogen, boron hydrides or silanes onto a carbon-carbon or carbon-heteroatom multiple bond in prochiral organic compounds or asymmetric addition of carbon nucleophiles or amines onto allyl compounds.

14. A process for preparing chiral organic compounds by asymmetric addition of hydrogen, boron hydrides or silanes onto a carbon-carbon or carbon-heteroatom multiple bond in prochiral organic compounds or asymmetric addition of carbon nucleophiles or amines onto allyl compounds in the presence of a catalyst, **characterized in that** the addition reaction is carried out in the presence of catalytic amounts of at least one metal complex as claimed in claim 9.

## Revendications

1. Composés de formule I sous forme de racémates, de mélanges de diastéréoisomères ou de distéréoisomères essentiellement purs, 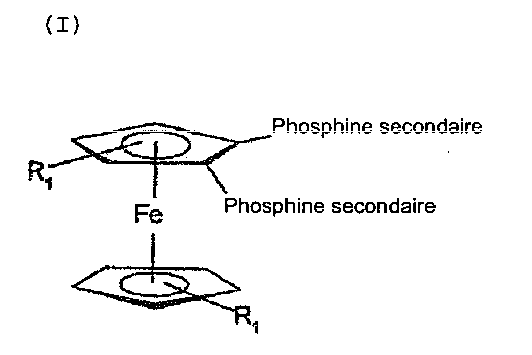 où
R₁ représente un atome d'hydrogène ou un groupe alkyle en C₁-C₄, et au moins un groupe phosphine secondaire représente un groupe phosphine cyclique substitué ou non substitué, ou son sel de phosphonium avec un ou deux anions monovalents ou un anion divalent.

2. Composés selon la revendication 1, dans lesquels le groupe phosphine secondaire cyclique correspond aux formules II, IIa, IIb ou IIc, 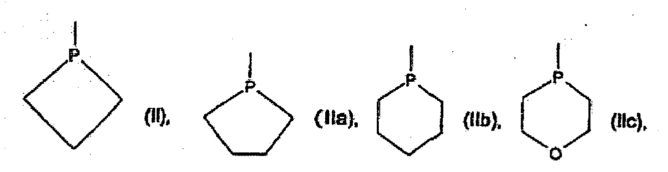 qui sont non substituées ou substituées une ou plusieurs fois par -OH, alkyle en C₁-C₈, cycloalkyle en C₄-C₈, alcoxy en C₁-C₆, alcoxy-(en C₁-C₄)-alkyle-(en C₁-C₄), phényle, alkyl- (en C₁-C₄)phényle ou alcoxy-(en C₁-C₄) phényle, benzyle, alkyl-(en C₁-C₄)benzyle ou alcoxy-(en C₁-C₄) benzyle, benzyloxy, alkyl-(en C₁-C₄)benzyloxy ou alcoxy (en C₁-C₄)benzyloxy, ou alkylidène- (en C₁-C₄) dioxyle.

3. Composés selon la revendication 2, dans lesquels les substituants sont liés en une ou en deux positions à l'atome de P.

4. Composés selon la revendication 1, dans lesquels les composés de formule I correspondent aux formules III ou IV, 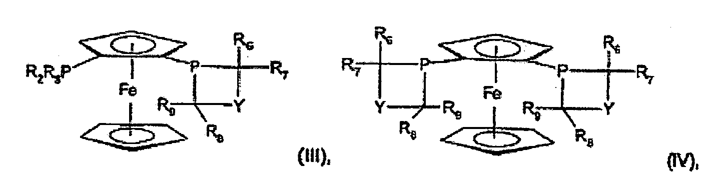 où
R₂ et R₃, indépendamment les uns des autres, représentent un radical hydrocarboné qui comporte de 1 à 20 atomes de carbone et est non substitué ou substitué par un substituant halogène, alkyle en C₁-C₆, halogénoalkyle en C₁-C₆, alcoxy en C₁-C₆, halogénoalcoxy en C₁-C₆, di-(alkyle en C₁-C₄)-amino, (C₆H₅)₃Si, (alkyle en C₁-C₁₂)₃Si, ou -CO₂-(alkyle en C₁-C₆),
Y représente -CH₂-, -CH₂CH₂-, -CH₂CH₂CH₂-, - CH(OH)CH(OH)-, -CH(O-alkyle en C₁-C₄)CH(O-alkyle en C₁-C₄)-, ou bien représente un radical de formule 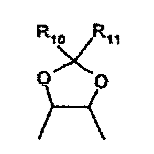
R₆, R₇, R₈ et R₉ représentent, indépendamment les uns des autres, un atome d'hydrogène, un groupe alkyle en C₁-C₄ ou benzyle, et au moins l'un des radicaux R₆, R₇, R₈ et R₉ représente un groupe alkyle en C₁-C₄, benzyle ou -CH₂-O-(alkyle en C₁-C₄) ou bien -CH₂-O-(aryle en C₆-C₁₀),
R₁₀ représente un atome d'hydrogène ou un groupe alkyle en C₁-C₄, et
R₁₁ représente un groupe alkyle en C₁-C₄.

5. Procédé de préparation de composés de formule I sous forme de racémates, de mélanges de diastéréoisomères
ou de distéréoisomères essentiellement purs, 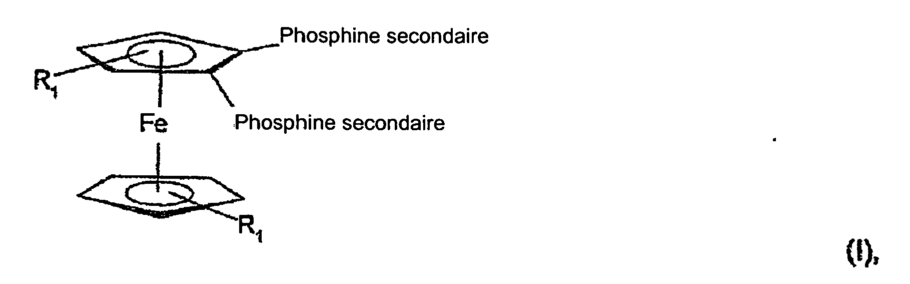 où
R₁ représente un atome d'hydrogène ou un groupe alkyle en C₁-C₄, et au moins une phosphine secondaire représente un groupe phosphine cyclique substitué ou non substitué, comprenant les étapes
a) Réaction d'un composé de formule V 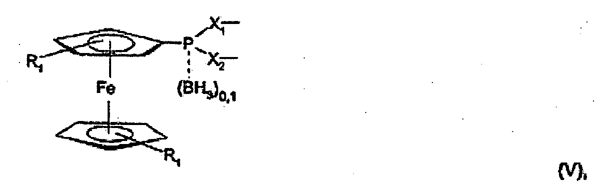 où
X₁ et X₂ représentent, indépendamment les uns des autres, un atome d'oxygène ou d'azote, et des radicaux à liaisons carbone-carbone ou carbone-hétéroatome sont liés aux liaisons libres des atomes d'oxygène et d'azote,
sur au moins des quantités équivalentes d'alkyle de lithium, d'un composé de Grignard-magnésium, ou d'un amide secondaire-Li aliphatique ou amide secondaire X₃Mg pour former un composé de formule VI, 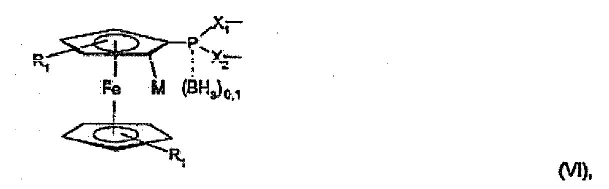 où
M représente -Li ou -MgX₃, et X₃ représente Cl, Br ou I,
b) Réaction du composé de formule VI sur au moins des quantités équivalentes d'un halogénure de diaminophosphine secondaire, d'un halogénure de dialcoxyphosphine, d'un halogénure de diamino-P(O) secondaire, d'un halogénure de dialcoxy-P(O) ou de PCl₃ ou de PBr₃ pour former un composé de formule VII 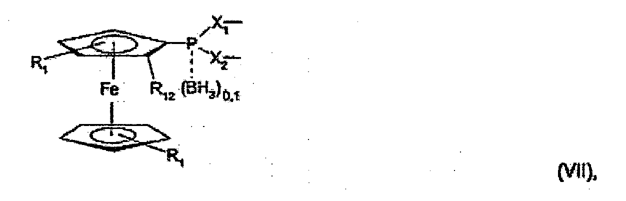 où
R₁₂ représente -PCl₂, -PBr₂, di(amino secondaire) P-, dialcoxyP-, diamino secondaire-P(O)-, dialcoxy-P(O)-, et
b1) on élimine d'un composé de formule VII, lorsqu'il est présent, le groupe borane, puis on sépare les radicaux (hétéro)hydrocarboné-X₁, (hétéro)hydrocarboné-X₂, ou X₁-(hétéro)hydrocarboné-X₂ ainsi que le groupe diamino secondaire ou dialcoxy par de l'HCl ou du HBr avec formation d'un groupe -PCl₂- ou - PBr₂, puis on hydrogène les groupes -(O)PCl₂, -(O)PBr₂,-PCl₂ ou -PBr₂ pour former un composé de formule VIII, ou
b2) on sépare d'un composé de formule VII les radicaux (hétéro)hydrocarboné-X₁, (hétéro)hydrocarboné-X₂, ou X₁-(hétéro)hydrocarboné-X₂ ainsi que diamino secondaire ou dialcoxy par de l'HCl ou du HBr avec formation d'un groupe -PCl₂ ou -PBr₂, puis on hydrogène les groupes - (O) PCl₂, - (O) PBr₂, -PCl₂ ou -PBr₂, et on élimine le groupe borane pour former un composé de formule VIII, 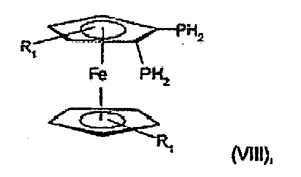 ou
c) Réaction d'un composé de formule VI sur un halogénure de phosphine secondaire pour former un composé de formule IX, 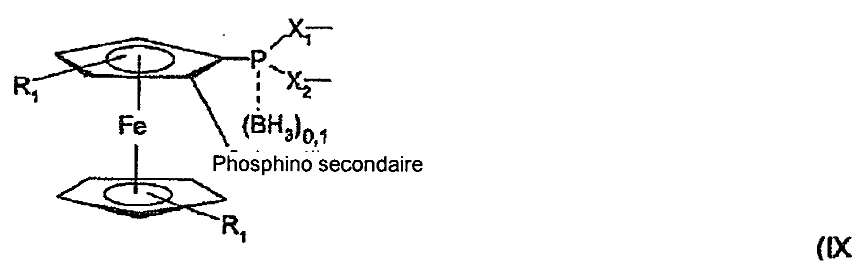
c1) on élimine d'un composé de formule IX, lorsqu'il est présent, le groupe borane, puis on sépare les radicaux (hétéro)hydrocarboné-X₁, (hétéro)hydrocarboné-X₂, ou X₁-(hétéro)hydrocarboné-X₂ par de l'HCl ou du HBr avec formation d'un groupe -PCl₂ ou -PBr₂, puis on hydrogène les groupes -PCl₂ ou -PBr₂ pour former un composé de formule X, ou
c2) on sépare d'un composé de formule IX les radicaux (hétéro)hydrocarboné-X₁, (hétéro)hydrocarboné-X₂, ou X₁-(hétéro)hydrocarboné-X₂ par de l'HCl ou du HBr avec formation d'un groupe -PCl₂ ou -PBr₂, puis on hydrogène les groupes -PCl₂ ou -PBr₂, puis on élimine le groupe borane pour former un composé de formule X 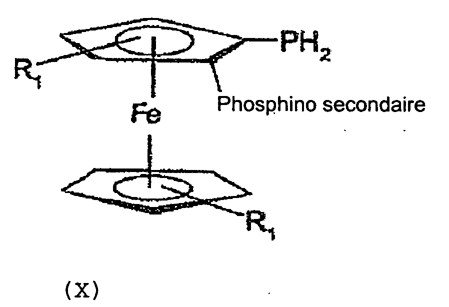 ou
d) Réaction d'un composé de formule VI sur un réactif d'halogénation pour former un composé de formule XI 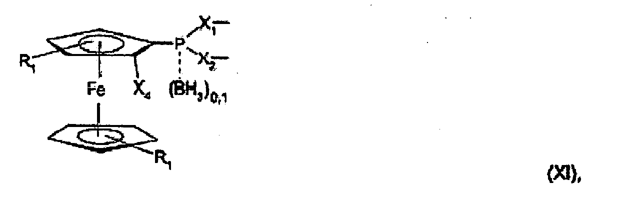 où
X₄ représente Cl, Br ou I,
d1) on élimine d'un composé de formule XI, lorsqu'il est présent, le groupe borane, puis on sépare les radicaux (hétéro) hydrocarboné-X₁, (hétéro)hydrocarboné-X₂, ou X₁-(hétéro)hydrocarboné-X₂ par de l'HCl ou du HBr avec formation d'un groupe -PCl₂ ou -PBr₂, puis on hydrogène le groupe -PCl₂ ou -PBr₂ pour former un composé de formule XII, ou
d2) on sépare d'un composé de formule XI les radicaux (hétéro)hydrocarboné-X₁, (hétéro)hydrocarboné-X₂, ou X₁-(hétéro)hydrocarboné-X₂ par de l'HCl ou du HBr avec formation d'un groupe -PCl₂ ou -PBr₂, puis on hydrogène les groupes -PCl₂ ou -PBr₂, puis on élimine le groupe borane pour former un composé de formule XII 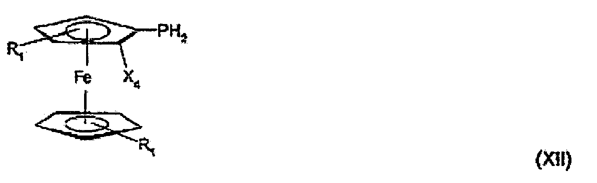 et
d3) on fait réagir le composé de formule XII sur un phosphure secondaire métallique pour former un composé de formule X,
e) Réaction du composé de formule VII sur au moins 2 équivalents et du composé de formule X sur au moins 1 équivalent d'un sulfate cyclique ou d'un disulfonate à chaîne ouverte pour produire des composés de formule I, où un ou deux groupes phosphino secondaire représentent des groupes phosphino secondaire cycliques, ou
f) Réaction d'un composé de formule XII sur au moins 1 équivalent d'un sulfate cyclique ou d'un disulfonate à chaîne ouverte pour produire des composés de formule XIII, 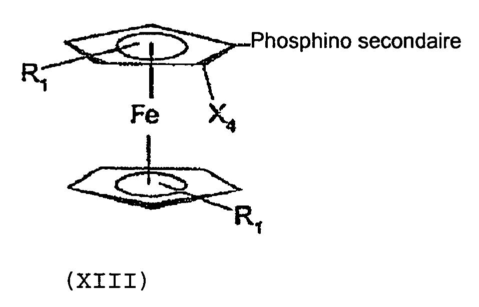 où
le groupe phosphino secondaire représente un groupe phosphino secondaire cyclique, éventuellement protégé par BH₃, puis réaction d'un composé de formule XIII sur au moins 1 équivalent d'alkyle de lithium et puis sur au moins 1 équivalent d'halogénure de phosphine secondaire pour former un composé de formule I.

6. Composés des formules VII, IX et XI, 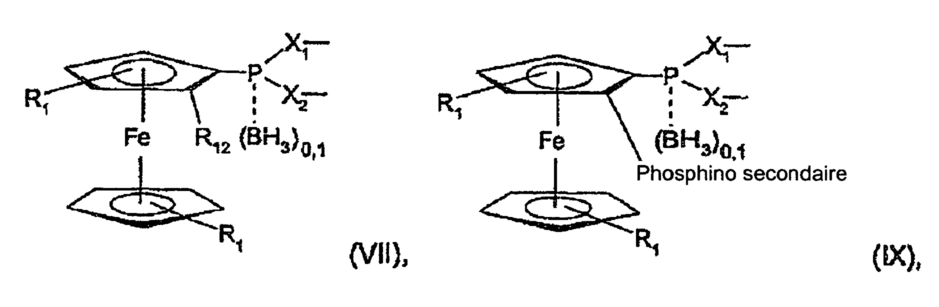 et 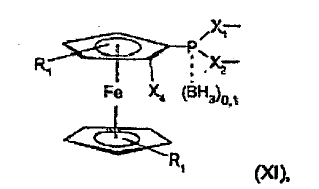 où
X₁ et X₂ représentent, indépendamment les uns des autres, un atome d'oxygène ou d'azote, et des radicaux à liaisons carbone-carbone ou carbone-hétéroatome sont liés aux liaisons libres des atomes d'oxygène et d'azote, et
R₁, R₁₂ et X₄ ont les significations données dans la revendication 5.

7. Composés des formules VIII, X et XII, 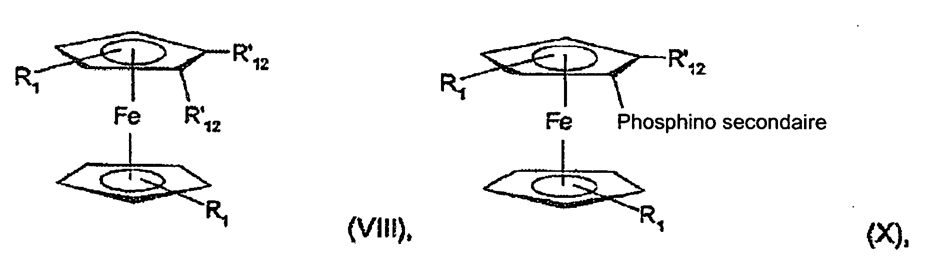 et 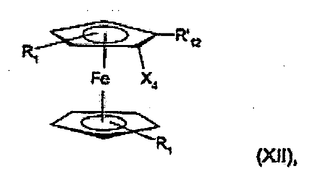 où R'₁₂ représente -PCl₂, -PBr₂ ou -PH₂, et R₁ et X₄ ont les significations données dans la revendication 5.

8. Composés de formule XIII 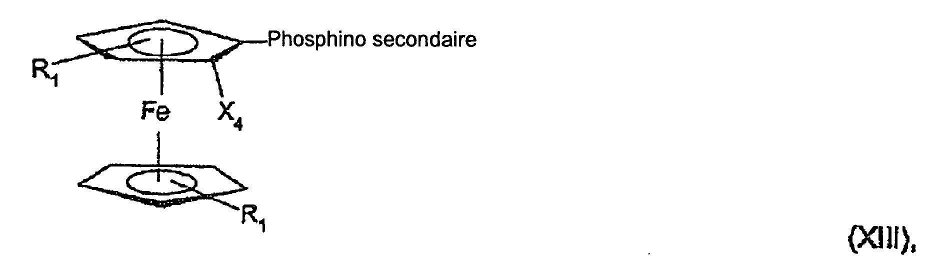 où
R₁ et X₄ ont les significations données dans la revendication 5, et le groupe phosphino secondaire représente un groupe phosphino secondaire cyclique.

9. Complexe d'un métal choisi dans le groupe consituté par les métaux TM8 avec des composés des formules I en tant que ligands.

10. Complexes métalliques selon la revendication 9, où le métal TM-8 est le ruthénium, le rhodium ou l'iridium.

11. Complexes métalliques selon la revendication 9, **caractérisés en ce qu'**ils correspondent aux formules XIV et XV,
A₁MeLₙ (XIV),
(A₁MeLₙ)^{(z+)}(E⁻)_{z} (XV),
où
A₁ représente un composé de formule I,
L représente des ligands identiques ou différents monodentates, anioniques ou non ioniques ou deux L conjointement formant des ligands identiques ou différents bidentates, anioniques ou non ioniques ;
n vaut 2, 3 ou 4 lorsque L est un ligand monodentate, ou n vaut 1 ou 2 lorsque L est un ligand bidentate ;
z vaut 1, 2 ou 3 ;
Me est un métal choisi dans le groupe constitué par Rh, Ir et Ru, avec le métal en état d'oxydation 0, 1, 2, 3 ou 4 ;
E⁻ est un anion acide oxo ou acide complexe ; et
les ligands anioniques équilibrent la charge du métal à l'état d'oxydation 1, 2, 3 ou 4.

12. Complexes métalliques selon la revendication 9, **caractérisés en ce qu'**ils correspondent aux formules XVI et XVII,
[A₁Me₂YZ] (XVI),
[A₁Me₂Y]⁺E₁⁻ (XVII),
où
A1 représente un composé de formule I ;
Me₂ représente le rhodium ou l'iridium;
Y représente deux oléfines ou un diène ;
Z représente Cl, Br ou I; et
E₁⁻ est un anion acide oxo ou acide complexe.

13. Utilisation de complexes métalliques selon la revendication 9 en tant que catalyseurs homogènes pour la production de composés organiques chiraux par addition asymétrique d'hydrogène, d'hydrures de bore ou de silanes aux liaisons multiples carbone-carbone ou carbone-hétéroatome dans des composés organiques pro-chiraux, ou par addition asymétrique de nucléophiles carbonés ou d'amines à des composés allyliques.

14. Procédé de préparation de composés organiques chiraux par addition asymétrique d'hydrogène, d'hydrures de bore ou de silanes aux liaisons multiples carbone-carbone ou carbone-hétéroatome dans des composés organiques pro-chiraux, ou par addition asymétrique de nucléophiles carbonés ou d'amines à des composés allyliques en présence d'un catalyseur, **caractérisé en ce qu'**on met en oeuvre la réaction d'addition en présence de quantités catalytiques d'au moins un complexe métallique selon la revendication 9.
